# EUROPEAN PATENT APPLICATION

(11) **EP 4 534 097 A1**
(43) Date of publication of application: **09.04.2025**
(21) Application number: 23816362.0
(22) Date of filing: 31.05.2023
(51) Int. Cl.: A61K 38/17, A61P 27/02, C07K 14/47

(54) **COMPOSITION FOR TREATING RETINAL OR MACULAR DISEASES**

(30) Priority: 31.05.2022 KR 20220066635; 30.05.2023 KR 20230068976
(71) Applicant: NEURACLE GENETICS INC., Seoul 02841 (KR)
(72) Inventor: KIM, Jee Yong, Gwacheon-si Gyeonggi-do 13836 (KR); BAK, Jeongyun, Seoul 02745 (KR); SHIM, Juwon, Seoul 04702 (KR); LEE, Junwoo, Seoul 03935 (KR); HAN, Joo Seok, Seoul 02725 (KR)
(74) Representative: V.O.
(86) International application number: PCT/KR2023/007442
(87) International publication number: WO 2023/234701

(57) **Abstract**

The present disclosure relates to a pharmaceutical composition for use in preventing or treating retinal or macular diseases, the composition comprising: a polypeptide comprising an amino acid sequence with at least 70% sequence identity with that of TAFA protein (e.g., TAFA1 to TAFA4); a nucleic acid encoding the polypeptide; a vector comprising the nucleic acid, a recombinant virus particle comprising the vector and capsid protein; or a cell comprising the nucleic acid or vector. In addition, the present disclosure relates to an adenoassociated virus (AAV) vector that comprises a nucleic acid encoding a polypeptide having an amino acid sequence with at least 70% sequence identity with that of TAFA protein, and a recombinant virus particle containing the AAV vector and capsid protein. The AAV vector, the recombinant virus, or the composition comprising the same according to the present disclosure can be advantageously used for the prevention or treatment of retinal or macular diseases by recovering damaged retina.

## Description

### [Technical Field]

The present disclosure relates to a pharmaceutical composition for the prevention or treatment of retinal or macular diseases, which comprises a polypeptide including an amino acid sequence with at least 70% sequence identity with that of TAFA protein (e.g., TAFA1 to TAFA4), a nucleic acid encoding the polypeptide, a vector comprising the nucleic acid, a recombinant virus particle comprising the vector and capsid protein, or a cell comprising the nucleic acid or vector.

### [Background Art]

The retina is a transparent and thin membrane located at the innermost part of the eye wall and in contact with the vitreous body. It plays the role of the primary visual information organ that converts optical information of objects into electrical signals and transmits images to the central visual area of the brain through the optic nerve. The retina is composed of more than 100 million light-sensitive photoreceptor cells, more than 1 million ganglion cells, and nerve cells that connect them. The macula is a nerve tissue located in the center of the retina, where most of optic cells are gathered. It is responsible for central vision as the location where images of objects are formed. The macula lutea forms a thinner part of retina that is composed of a photoreceptor cell layer consisting of cone cells and a ganglion cell layer. In the macula lutea, the electrical signal of an image is converted into a chemical signal when it is bright light and transmitted to the brain through the optic nerve, which is the axon of ganglion cells. The retina other than the macula lutea recognizes the periphery and plays a major role when it is dark. Therefore, when an abnormality occurs in the retina or macula due to aging or external factors, it can lead to blindness along with visual impairment that causes problems with vision and sight. Retinal or macular diseases include diabetic retinopathy, choroidal neovascularization, macular degeneration, retinal degeneration, macular edema, retinal edema, macular swelling, etc.

The TAFA protein family is a group of proteins that are abundantly expressed throughout brain regions and consists of five proteins ranging from TAFA1 to 5. These proteins contain structurally conserved cysteine residues, and the C, CC and CXC motifs between the cysteine residues are associated with chemokines. TAFA1 to 4 have a high degree of similarity in the number of cysteine residues and the space of cysteine residues, while TAFA5 has fewer cysteine residues.

These proteins are evolutionarily conserved well in vertebrates and have been shown to be important for the normal functioning of the central nervous system. Weight loss, decreased anxiety behavior, impaired memory of fear, etc. have been reported for TAFA1-knock mice (Lei X, Liu L, Terrillion CE, et al. FASEB J. 2019; 33(12): 14734-14747 and Yong HJ, Ha N, Cho EB, et al. Sci Rep. 2020; 10(1): 3969). Conversely, increase in anxiety behaviors was reported for TAFA2- and TAFA3-knockout mice (Choi JH, Jeong YM, Kim S, et al. Proc Natl Acad Sci USA. 2018; 115(5): E1041-E1050 and Kim S, Lee B, Choi JH, Kim JH, Kim CH, Shin HS. Sci Rep. 2017; 7(1): 16503). In TAFA4-null mice, allodynia and hyperalgesia have been reported (Delfini MC, Mantilleri A, Gaillard S, et al. Cell Rep. 2013; 5(2): 378-388). In TAFA5-knockout mice, behavioral changes such as increased depressive behavior and loss of spatial memory have been reported (Huang S, Zheng C, Xie G, et al. FAM19A5/TAFA5, a novel neurokine, plays a crucial role in depressive-like and spatial memory-related behaviors in mice. Mol Psychiatry. 2021; 26(6): 2363-2379).

Despite the important role of these proteins in the central nervous system, there is very limited understanding of their functions and their pathophysiological or therapeutic functions, as well as their role in other than the central nervous system.

The matters described above as the background art are only for the purpose of improving the understanding of the background of the present disclosure, and should not be taken as an acknowledgment that they correspond to the prior art already known to those skilled in the art.

### [Disclosure]

### [Technical Problem]

The present disclosure provides a pharmaceutical composition for the prevention or treatment of retinal or macular diseases, which comprises a polypeptide comprises an amino acid sequence with at least 70% sequence identity with an amino acid sequence represented by SEQ ID NO 75, a nucleic acid encoding the polypeptide, a vector comprising the nucleic acid, a recombinant viral particle comprising the vector and capsid protein, or a cell comprising the nucleic acid or vector.

The present disclosure also provides the polypeptide, a nucleic acid encoding the polypeptide, a vector comprising the nucleic acid, a recombinant viral particle comprising the vector and capsid protein, or a cell comprising the nucleic acid or vector for use in therapy.

The present disclosure also provides the AAV vector or the recombinant virus particle for use in the prevention or treatment of retinal or macular diseases. The present disclosure also provides a method for preventing or treating retinal or macular diseases in a subject in need thereof, which comprises administering the polypeptide, a nucleic acid encoding the polypeptide, a vector comprising the nucleic acid, a recombinant viral particle comprising the vector and capsid protein, or a cell comprising the nucleic acid or vector to the subject.

In some aspects, an amino acid sequence with at least 70% sequence identity with the amino acid sequence represented by SEQ ID NO 75 includes an amino acid sequence of General Formula 1 (from N-terminal to C-terminal):

<General Formula 1> X1-X2-X3-G-T-C-E-V-X4-A-X5-H-X6-C-C-N-X7-N-X8-I-E-E-X9-S-Q-T-X10-X11-C-S-C-X12-X13-G-X14-V-A-G-T-T-X15-X16-X17-P-S-C-V-X18-A-X19-I-V-X20-X21-X22-W-W-C-X23-M-X24-P-C-X25-X26-G-E-X27-C-K-X28-L-P-D-X29-X30-G-W-X31-C-X32-X33-G-X34-K-X35-K-T-T-X36-X37-X38-X39

In General Formula 1,
X1 is nonexistent, V, I or L,
X2 is K, E, R or Q,
X3 is G, T, Q, P or A,
X4 is V or I,
X5 is A, L, V or I,
X6 is R or L,
X7 is K, R or Q,
X8 is R or K,
X9 is R or L,
X10 is V or G,
X11 is K or N,
X12 is F or L,
X13 is P or S,
X14 is Q or K,
X15 is R, H or Q,
X16 is A, N, S or T,
X17 is A, Q, R, K or T,
X18 is D or E,
X19 is S or A,
X20 is I, E, L, A or V,
X21 is Q, G or E,
X22 is K or R,
X23 is H, Q or E,
X24 is E, Q, N, D, S or H,
X25 is L, V or M,
X26 is E, D, P, L or A,
X27 is E or D,
X28 is V, T, A or I,
X29 is L, N, R, Y, S or Q,
X30 is S, K or T,
X31 is S or M,
X32 is S, A or Y,
X33 is S, T or R,
X34 is N or H,
X35 is V or I,
X36 is R or K,
X37 is nonexistent, V, A, G, M or N,
X38 is nonexistent, T, I, N, F or S, and
X39 is nonexistent, R, H, V, K, I or Q.

In some aspects, the polypeptide further comprises an amino acid sequence of General Formula 2 (from N-terminal to C-terminal):

<General Formula 2> X1-X2-H-H-K-A-X3-H

In General Formula 2,
X1 is A or V,
X2 is N or I, and
X3 is H or Q.

In some aspects, the polypeptide comprises an amino acid sequence of General Formula 2-General Formula 1 (from N-terminal to C-terminal).

In some aspects, the polypeptide further comprises an amino acid sequence of General Formula 3 (from N-terminal to C-terminal):

<General Formula 3> X1-X2-X3-X4-X5-X6-X7-X8-X9-X10

In General Formula 3,
X1 is A or S,
X2 is L, T or S,
X3 is Q, E or H,
X4 is nonexistent, P, L or H,
X5 is P or R,
X6 is T, S or I,
X7 is A, P, T, S or H,
X8 is T, A, S or I,
X9 is V or A, and
X10 is L or H.

In some aspects, the polypeptide comprises an amino acid sequence of General Formula 3- General Formula 1 (from N-terminal to C-terminal).

In some aspects, the polypeptide further comprises an amino acid sequence of General Formula 4 (from N-terminal to C-terminal):

<General Formula 4> X1-X2

In General Formula 4,
X1 is H or Y, and
X2 is Q, V or L.

In some aspects, the polypeptide comprises an amino acid sequence of General Formula 4-General Formula 1 (from N-terminal to C-terminal).

In some aspects, the polypeptide further comprises an amino acid sequence of LHRP (SEQ ID NO 293), LHQSGFTSGHFPHHRKLGE (SEQ ID NO 294) or LAPPGTNIQI (SEQ ID NO 295) (from N-terminal to C-terminal).

In some aspects, the polypeptide further comprises an amino acid sequence of General Formula 5 (from N-terminal to C-terminal):

<General Formula 5> X1-X2-X3

In General Formula 5,
X1 is P or G,
X2 is R or H, and
X3 is T, S or L.

In some aspects, the polypeptide further comprises an amino acid sequence of PYTSL (SEQ ID NO 296) or QEDKLK (SEQ ID NO 297) (from N-terminal to C-terminal).

In some aspects, the polypeptide comprises an amino acid sequence of LHRP-General Formula 1, LHQSGFTSGHFPHHRKLGE-General Formula 1 or LAPPGTNIQI-General Formula 1 (from N-terminal to C-terminal). In some aspects, the polypeptide comprises an amino acid sequence of General Formula 1-General Formula 5, General Formula 1-PYTSL or General Formula 1-QEDKLK (from N-terminal to C-terminal). In some aspects, the polypeptide comprises an amino acid sequence of LHRP-General Formula 1-General Formula 5, LHQSGFTSGHFPHHRKLGE-General Formula 1-General Formula 5, LAPPGTNIQI-General Formula 1-QEDKLK or LAPPGTNIQI-General Formula 1-PYTSL (from N-terminal to C-terminal).

In some aspects, the polypeptide comprises one or more amino acid sequence selected from a group consisting of amino acid sequences represented by SEQ ID NOS 75 to 129. In some aspects, the nucleic acid encoding the polypeptide comprises one or more nucleotide sequence selected from a group consisting of nucleotide sequences represented by SEQ ID NOS 184 to 238.

In some aspects, the polypeptide comprises one or more amino acid sequence selected from a group consisting of amino acid sequences represented by SEQ ID NOS 130 to 183. In some aspects, the nucleic acid encoding the polypeptide includes one or more nucleotide sequence selected from a group consisting of nucleotide sequences represented by SEQ ID NOS 239 to 292.

In some aspects, the polypeptide further comprises a signal sequence.

In some aspects, the vector is a viral vector. In some aspects, the virus is an adeno-associated virus (AAV). In some aspects, the AAV is serotype AAV1, AAV2, AAV3, AAV4, AAV5, AAV6, AAV7, AAV8, AAV9 or AAVrh10.

In some aspects, the retinal or macular disease is caused by the damage to all or part of the retina or macula. In some aspects, the retinal or macular disease is a disease caused by the dysfunction or damage of the retinal or macular cells. In some aspects, the retinal or macular disease may be a disease caused by the dysfunction or damage of photoreceptor cells and/or retinal pigment epithelial (RPE) cells of the retina or macula. In some aspects, the retinal or macular disease is retinopathy, choroidal neovascularization, maculopathy, macular degeneration, retinal degeneration, macular edema, retinal edema, macular swelling, retinal cell degeneration, retinal vascular occlusion, retinal detachment, inherited retinal disease or a combination thereof. In some aspects, the macular degeneration is age-related macular degeneration, Best's macular dystrophy, Sorsby's fundus dystrophy, malattia leventinese, Doyne's honeycomb retinal dystrophy, Stargardt's disease (Stargardt's macular dystrophy), myopic macular degeneration, or pigment epithelial detachment-related macular degeneration. In some aspects, the macular degeneration is age-related macular degeneration. In some aspects, the age-related macular degeneration is wet or dry age-related macular degeneration. In some aspects, the retinopathy is retinal dystrophy. In some aspects, the retinopathy is diabetic retinopathy. In some aspects, the diabetic retinopathy is non-proliferative diabetic retinopathy (NPDR), proliferative diabetic retinopathy (PDR), diabetic maculopathy, diabetic macular edema, or a combination thereof. In some aspects, the retinal or macular disease is an inherited retinal disease. In some aspects, the inherited retinal disease is retinitis pigmentosa (RP), Leber congenital amaurosis, Stargardt's disease, Coats retinopathy, cone dystrophy, choroideremia, Usher syndrome, Best disease, X-linked retinoschisis, or unspecified hereditary retinal dystrophy.

The present disclosure also provides an adeno-associated virus (AAV) vector comprising a nucleic acid encoding a polypeptide comprising an amino acid sequence with at least 70% sequence identity with the amino acid sequence represented by SEQ ID NO 75.

In some aspects, the vector further comprises one or more sequence selected from a group consisting of a promoter sequence, an enhancer sequence, an exon sequence, an intron sequence, a signal sequence-coding sequence, a splicing donor sequence, and one or more adeno-associated viral inverted terminal repeat (ITR) sequence.

In some aspects, the intron consists of 51 to 117 nucleotides and comprises a nucleotide sequence represented by SEQ ID NO 57. In some aspects, the intron has at least 70% sequence identity with (i) the 871st to 924th nucleotides of SEQ ID NO 1 (SEQ ID NO 58), (ii) the 861st to 924th nucleotides of SEQ ID NO 1 (SEQ ID NO 59), (iii) the 852nd to 924th nucleotides of SEQ ID NO 1 (SEQ ID NO 3), (iv) the 851st to 924th nucleotides of SEQ ID NO 1 (SEQ ID NO 61), (v) the 830th to 924th nucleotides of SEQ ID NO 1 (SEQ ID NO 2), (vi) the 821st to 924th nucleotides of SEQ ID NO 1 (SEQ ID NO 63), (vii) the 811th to 924th nucleotides of SEQ ID NO 1 (SEQ ID NO 64), or (viii) the 808th to 924th nucleotides of SEQ ID NO 1 (SEQ ID NO 65). In some aspects, the intron comprises (i) the 871st to 924th nucleotides of SEQ ID NO 1 (SEQ ID NO 58), (ii) the 861st to 924th nucleotides of SEQ ID NO 1 (SEQ ID NO 59), (iii) the 852nd to 924th nucleotides of SEQ ID NO 1 (SEQ ID NO 3), (iv) the 851st to 924th nucleotides of SEQ ID NO 1 (SEQ ID NO 61), (v) the 830th to 924th nucleotides of SEQ ID NO 1 (SEQ ID NO 2), (vi) the 821st to 924th nucleotides of SEQ ID NO 1 (SEQ ID NO 63), (vii) the 811th to 924th nucleotides of SEQ ID NO 1 (SEQ ID NO 64), or (viii) the 808th to 924th nucleotides of SEQ ID NO 1 (SEQ ID NO 65).

In some aspects, the vector comprises the followings: (1) a CMV enhancer sequence represented by SEQ ID NO 4; (2) a promoter sequence selected from a CMV promoter sequence represented by SEQ ID NO 5 or 6, an EF-1α promoter sequence represented by SEQ ID NO 7, or a chicken β-actin promoter sequence represented by SEQ ID NO 8; (3) an exon 1 (E1) sequence selected from a CMV E1 sequence represented by SEQ ID NO 12, an EF-1α E1 sequence represented by SEQ ID NO 13, or a chicken β-actin E1 sequence represented by SEQ ID NO 14 or 15; (4) a splicing donor sequence represented by SEQ ID NO 9 or 10; and/or (5) an EF-1α E2 sequence represented by SEQ ID NO 11.

In some aspects, an amino acid sequence with at least 70% sequence identity with the amino acid sequence represented by the SEQ ID NO 75 (from N-terminal to C-terminal) comprises the amino acid sequence of General Formula 1:

<General Formula 1> X1-X2-X3-G-T-C-E-V-X4-A-X5-H-X6-C-C-N-X7-N-X8-I-E-E-X9-S-Q-T-X10-X11-C-S-C-X12-X13-G-X14-V-A-G-T-T-X15-X16-X17-P-S-C-V-X18-A-X19-I-V-X20-X21-X22-W-W-C-X23-M-X24-P-C-X25-X26-G-E-X27-C-K-X28-L-P-D-X29-X30-G-W-X31-C-X32-X33-G-X34-K-X35-K-T-T-X36-X37-X38-X39

In General Formula 1,
X1 is nonexistent, V, I or L,
X2 is K, E, R or Q,
X3 is G, T, Q, P or A,
X4 is V or I,
X5 is A, L, V or I,
X6 is R or L,
X7 is K, R or Q,
X8 is R or K,
X9 is R or L,
X10 is V or G,
X11 is K or N,
X12 is F or L,
X13 is P or S,
X14 is Q or K,
X15 is R, H or Q,
X16 is A, N, S or T,
X17 is A, Q, R, K or T,
X18 is D or E,
X19 is S or A,
X20 is I, E, L, A or V,
X21 is Q, G or E,
X22 is K or R,
X23 is H, Q or E,
X24 is E, Q, N, D, S or H,
X25 is L, V or M,
X26 is E, D, P, L or A,
X27 is E or D,
X28 is V, T, A or I,
X29 is L, N, R, Y, S or Q,
X30 is S, K or T,
X31 is S or M,
X32 is S, A or Y,
X33 is S, T or R,
X34 is N or H,
X35 is V or I,
X36 is R or K,
X37 is nonexistent, V, A, G, M or N,
X38 is nonexistent, T, I, N, F or S, and
X39 is nonexistent, R, H, V, K, I or Q.

In some aspects, the amino acid sequence with at least 70% sequence identity with the amino acid sequence represented by SEQ ID NO 75 consists of 80 to 130, specifically 90 to 120, most specifically 91 to 119, amino acid sequences.

In some aspects, the polypeptide further comprises an amino acid sequence of General Formula 2 (from N-terminal to C-terminal):

<General Formula 2> X1-X2-H-H-K-A-X3-H

In General Formula 2,
X1 is A or V,
X2 is N or I, and
X3 is H or Q.

In some aspects, the polypeptide further comprises an amino acid sequence of General Formula 3 (from N-terminal to C-terminal):

<General Formula 3> X1-X2-X3-X4-X5-X6-X7-X8-X9-X10

In General Formula 3,
X1 is A or S,
X2 is L, T or S,
X3 is Q, E or H,
X4 is nonexistent, P, L or H,
X5 is P or R,
X6 is T, S or H,
X7 is A, P, T, S or I,
X8 is T, A, S or I,
X9 is V or A, and
X10 is L or H.

In some aspects, the polypeptide further comprises an amino acid sequence of General Formula 4 (from N-terminal to C-terminal):

<General Formula 4> X1-X2

In General Formula 4,
X1 is H or Y, and
X2 is Q, V or L.

In some aspects, the polypeptide further comprises the following amino acid sequences (from N-terminal to C-terminal):
LHRP (SEQ ID NO 293);
LHQSGFTSGHFPHHRKLGE (SEQ ID NO 294); or
LAPPGTNIQI (SEQ ID NO 295).

In some aspects, the polypeptide further comprises an amino acid sequence of General Formula 5 (from N-terminal to C-terminal):

<General Formula 5> X1-X2-X3

In General Formula 5,
X1 is P or G,
X2 is R or H, and
X3 is T, S or L.

In some aspects, the polypeptide further comprises the following amino acid sequences (from N-terminal to C-terminal):
PYTSL (SEQ ID NO 296); or
QEDKLK (SEQ ID NO 297).

In some aspects, the polypeptide comprises one or more amino acid sequence selected from a group consisting of amino acid sequences represented by SEQ ID NOS 75 to 129. In some aspects, the nucleic acid encoding the polypeptide comprises one or more nucleotide sequence selected from a group consisting of nucleotide sequences represented by SEQ ID NOS 184 to 238.

In some aspects, the polypeptide comprises one or more amino acid sequence selected from a group consisting of amino acid sequences represented by SEQ ID NOS 130 to 183. In some aspects, the nucleic acid encoding the polypeptide comprises one or more nucleotide sequence selected from a group consisting of nucleotide sequences represented by SEQ ID NOS 239 to 292.

In some aspects, the AAV vector is for use in gene therapy. In some aspects, the AAV vector is for use in the expression of a polypeptide comprising an amino acid sequence with at least 70% sequence identity with the amino acid sequence represented by SEQ ID NO 75.

The present disclosure also provides an isolated host cell comprising the AAV vector.

The present disclosure also provides an isolated host cell transformed with the AAV vector.

The present disclosure also provides a composition comprising the AAV vector or a host cell comprising the AAV vector or transformed with the AAV vector. In some aspects, the composition further comprising a pharmaceutically acceptable carrier. In some aspects, the composition is for use in preventing or treating retinal or macular diseases.

The present disclosure also provides a kit comprising the AAV vector or a host cell comprising the AAV vector or transformed with the AAV vector. In some aspects, the kit further comprises an instruction manual.

The present disclosure also provides a method for producing a recombinant viral particle, which comprises a step of transfecting a cell with i) the AAV vector and ii) a construct comprising a rep and cap genes. In some aspects, the method further comprises a step of isolating the produced recombinant virus particle.

The present disclosure also provides recombinant virus particle produced by the method.

The present disclosure also provides a recombinant virus particle comprising (a) a capsid protein and (b) the AAV vector.

The present disclosure also provides the AAV vector or the recombinant viral particle for use in therapy.

The present disclosure also provides the AAV vector or the recombinant virus particle for use in a method for preventing or treating a disease or disorder.

The present disclosure also provides a method for preventing or treating disease or disorder in a subject in need thereof, which comprises administering the AAV vector or the recombinant virus particle to the subject.

In some aspects, the disease or disorder comprises a retinal or macular disease. In some aspects, the retinal or macular disease is caused by the damage to all or part of the retina or macula. In some aspects, the retinal or macular disease is a disease caused by the dysfunction or damage of the retinal or macular cells. In some aspects, the retinal or macular disease may be a disease caused by the dysfunction or damage of photoreceptor cells and/or retinal pigment epithelial (RPE) cells of the retina or macula. In some aspects, the retinal or macular disease is retinopathy, choroidal neovascularization, maculopathy, macular degeneration, retinal degeneration, macular edema, retinal edema, macular swelling, retinal swelling, retinal cell degeneration, retinal vascular occlusion, retinal detachment, inherited retinal disease or a combination thereof. In some aspects, the method comprises administering an additional therapeutic agent to the subject.

The present disclosure also provides a polypeptide comprising an amino acid sequence with at least 70% sequence identity with the amino acid sequence represented by SEQ ID NO 75, a nucleic acid encoding the polypeptide, a vector comprising the nucleic acid, a recombinant virus particle comprising the vector and capsid protein, or a cell comprising the nucleic or vector for use in the prevention, alleviation or treatment of the retinal or macular disease described above.

### [Technical Solution]

The present disclosure provides a polypeptide comprising an amino acid sequence with at least 70% sequence identity with the amino acid sequence represented by SEQ ID NO 75 (e.g., TAFA1 to TAFA4 sequences in vertebrates), a nucleic acid encoding the polypeptide, a vector comprising the nucleic acid, a recombinant virus particle comprising the vector and a capsid protein, or a cell comprising the nucleic acid or vector for use in the prevention, alleviation, or treatment of retinal or macular disease. The present disclosure also provides a pharmaceutical composition for the prevention or treatment of retinal or macular diseases, which comprises the polypeptide, a nucleic acid encoding the polypeptide, a vector comprising the nucleic acid, a recombinant virus particle comprising the vector and a capsid protein, or a cell comprising the nucleic acid or vector. In addition, the present disclosure provides an adeno-associated virus (AAV) vector comprising a nucleic acid encoding the polypeptide; a host cell comprising the AAV vector or transformed with the AAV vector; a composition or kit comprising the AAV vector or a host cell comprising the AAV vector or transformed with the AAV vector; a method for producing a recombinant viral particle, which comprising transfecting a cell with the AAV vector and a construct comprising a rep and cap genes, a recombinant virus particle produced by the method; and a method for preventing or treating a disease or disorder in a subject in need thereof, which comprises administering the AAV vector or the recombinant viral particle to the subject.

As described herein, the applicant of the present disclosure has identified that the expression of TAFA genes of various species (human TAFA1, human TAFA2, human TAFA3, human TAFA4, mouse TAFA4, zebrafish TAFA4, gecko TAFA4) has excellent ability to recover or protect the retina from damage, and has derived a highly conserved sequence of human TAFA4 mature protein (SEQ ID NO 75) that plays a key role in the above-described effect. As demonstrated herein, in some aspects, the composition of the present disclosure recovers the damaged retina to a level comparable to normal retina. Additional aspects of the present disclosure are provided throughout the present specification.

For the sake of ease of understanding of the disclosure in this specification, a number of terms and phrases will be defined. Additional definitions are given throughout the detailed description.

### I. Definition

Throughout the present disclosure, the term "a" or "an" entity refers to one or more of that entity; for example, "a polypeptide," is understood to represent one or more polypeptides. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The term "at least" prior to a number or series of numbers is understood to include the number adjacent to the term "at least," and all subsequent numbers or integers that could logically be included, as clear from context. For example, the number of nucleotides in a nucleic acid molecule must be an integer. For example, "at least 18 nucleotides of a 21-nucleotide nucleic acid molecule" means that 18, 19, 20, or 21 nucleotides have the indicated property. When at least is present before a series of numbers or a range, it is understood that "at least" can modify each of the numbers in the series or range. "At least" is also not limited to integers (e.g., "at least 5%" includes 5.0%, 5.1%, 5.18% without consideration of the number of significant figures.

It is understood that wherever aspects are described herein with the language "comprising," otherwise analogous aspects described in terms of "consisting of" and/or "consisting essentially of" are also provided.

Unless defined otherwise, all technical and scientific terms used herein have the same meaning as commonly understood by one of ordinary skill in the art to which the present disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in the present disclosure.

Units, prefixes, and symbols are denoted in their Systeme International de Unites (SI) accepted form. Numeric ranges are inclusive of the numbers defining the range. Unless otherwise indicated, amino acid sequences are written left to right in amino to carboxy orientation. The headings provided herein are not limitations of the various aspects of the disclosure, which can be had by reference to the specification as a whole. Accordingly, the terms defined immediately below are more fully defined by reference to the specification in its entirety.

The term "about" is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, e.g., 10 percent, up or down (higher or lower).

The term "adeno-associated virus" (AAV) used herein refers to a single-chain DNA virus which is a helper-dependent human parvovirus. The genome size is about 4.6 kbp. The N-terminal part of the genome codes for the rep gene, which is involved in viral replication and expression of viral genes, and the C-terminal part codes for the cap gene encoding the capsid protein of the virus. And it consists of repeat regions (ITRs) with about 145 bases inserted at both ends. The ITR (inverted terminal repeat) with a size of 145 bp and having a T-shaped structure functions as the origin of replication in the viral genome and acts as the primary packaging signal. Since the ITR is the only cis-acting sequence required to make recombinant AAV constructs, and has enhancer activity in the presence of the Rep protein but has very weak activity in the absence of the Rep protein, it is necessary to consider this when cloning foreign genes into recombinant AAV constructs, and to construct expression constructs with appropriate enhancers, promoters, pA, etc. (R.J. Samulski and N Muzyczka, Annu. Rev. Virolo. 2014. 1: 427-451). Four proteins are translated from the rep gene, which are classified into rep78, rep68, rep52 and rep40 according to their molecular weights, and perform essential functions during AAV DNA replication. Four proteins are translated from the cap gene, of which the VP1, VP2 and VP3 proteins are structural proteins that make up AAV particles, and the assembly-activating protein (AAP) promotes the formation of the AAV particles by the structural proteins. In order for the adeno-associated virus to be replicated efficiently, it requires some proteins and RNAs derived from helper viruses such as adenovirus or herpes simplex virus (Muzyczka N. Curr Top Microbiol Immunol 158, 97-129, 1992).

AAV includes but is not limited to, AAV type 1, AAV type 2, AAV type 3 (including types 3A and 3B), AAV type 4, AAV type 5, AAV type 6, AAV type 7, AAV type 8, AAV type 9, AAV type 10, AAV type 11, AAV type 12, AAV type 13, AAVrh.74, snake AAV, avian AAV, bovine AAV, canine AAV, equine AAV, ovine AAV, goat AAV, shrimp AAV, those AAV serotypes and clades disclosed by Gao et al. (J. Virol. 78: 6381 (2004)) and Moris et al. (Virol. 33: 375 (2004)), and any other AAV now known or later discovered. See, e.g., FIELDS et al. VIROLOGY, volume 2, chapter 69 (4th ed., Lippincott-Raven Publishers). In some aspects, an "AAV" includes a derivative of a known AAV. In some aspects, an "AAV" includes a modified or an artificial AAV.

The terms "administration," "administering," and grammatical variants thereof refer to introducing a composition (e.g., polynucleotide comprising a transgene and untranslated nucleic acid sequence described herein) into a subject via a pharmaceutically acceptable route. The introduction of a composition into a subject is by any suitable route, including intratumorally, orally, pulmonarily, intranasally, parenterally (intravenously, intra-arterially, intramuscularly, intraperitoneally, or subcutaneously), rectally, intralymphatically, intrathecally, periocularly, intraocularly or topically. Administration includes self-administration and the administration by another. A suitable route of administration allows the composition or the agent to perform its intended function. For example, if a suitable route is intravenous, the composition is administered by introducing the composition or agent into a vein of the subject.

In this specification, the term "intraocular" refers to within or under the ocular tissue. As used herein, the term "intraocular administration" refers to any administration that can deliver a composition to sub-tenon, subconjunctival, suprachoroidal, subretinal, intravitreal and similar locations. In some aspects, the intraocular administration includes suprachoroidal, subretinal and intravitreal administration.

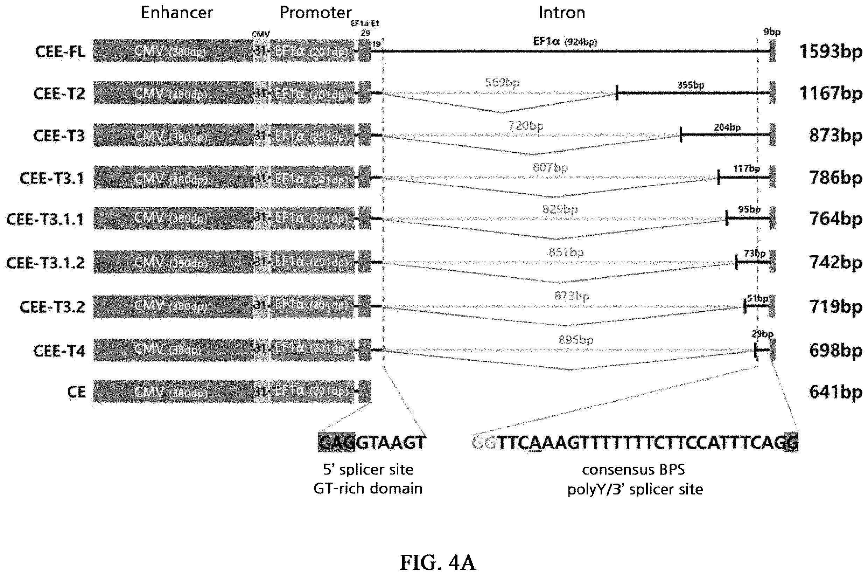

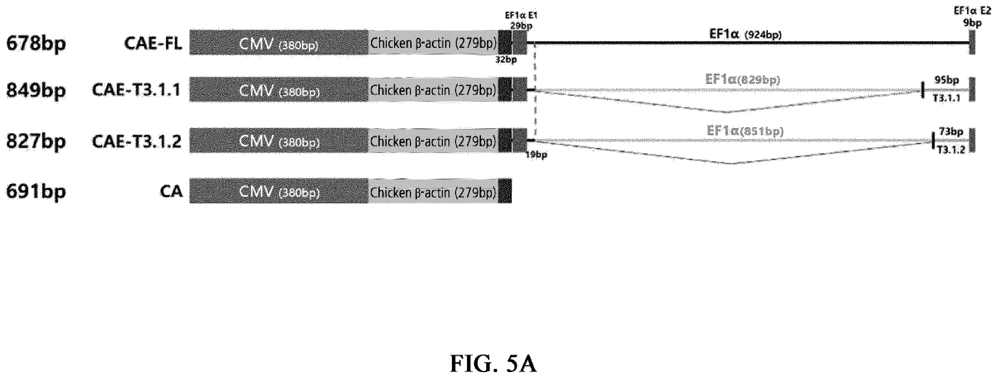

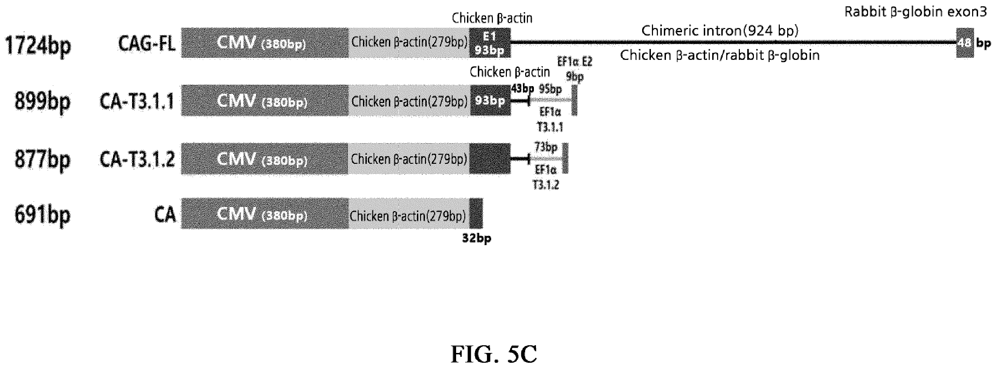

As used herein, a "CEE" construct comprises a CMV enhancer, an EF-1α promoter, and an EF-1α intron fragment. A "CE" construct comprises a CMV enhancer and an EF-1α promoter, but does not contain any intron fragment (e.g., no EF-1α intron fragment). A "CAE" construct comprises a CMV enhancer, a chicken β-actin promoter, and an EF-1α intron fragment. As used herein, a "CAG" construct comprises a CMV enhancer, a chicken β-actin promoter, and a chicken β-actin/rabbit β-globin hybrid intron fragment. A "CA" construct comprises a CMV enhancer and a chicken β-actin promoter, but does not contain any intron fragment (e.g., no EF-1α intron fragment). See FIGS. 4A, 5A and 5C.

As used herein, the term "conserved" refers to nucleotides or amino acid residues of a polynucleotide sequence or polypeptide sequence, respectively, that are those that occur unaltered in the same position of two or more sequences being compared. Nucleotides or amino acids that are relatively conserved are those that are conserved amongst more related sequences than nucleotides or amino acids appearing elsewhere in the sequences.

In some aspects, two or more sequences are said to be "completely conserved" or "identical" if they are 100% identical to one another. In some aspects, two or more sequences are said to be "highly conserved" if they are at least 70% identical, at least 80% identical, at least 90% identical, or at least 95% identical to one another. In some aspects, two or more sequences are said to be "highly conserved" if they are about 70% identical, about 80% identical, about 90% identical, about 95%, about 98%, or about 99% identical to one another. In some aspects, two or more sequences are said to be "conserved" if they are at least 30% identical, at least 40% identical, at least 50% identical, at least 60% identical, at least 70% identical, at least 80% identical, at least 90% identical, or at least 95% identical to one another. In some aspects, two or more sequences are said to be "conserved" if they are about 30% identical, about 40% identical, about 50% identical, about 60% identical, about 70% identical, about 80% identical, about 90% identical, about 95% identical, about 98% identical, or about 99% identical to one another. Conservation of sequence can apply to the entire length of a polynucleotide or polypeptide or can apply to a portion, region or feature thereof.

The terms "complementary" and "complementarity" refer to two or more oligomers *(i.e.,* each comprising a nucleobase sequence), or between an oligomer and a target gene, that are related with one another by Watson-Crick base-pairing rules. For example, the nucleobase sequence "T-G-A (5'→3')" is complementary to the nucleobase sequence "A-C-T (3'→5')". Complementarity can be "partial," in which less than all of the nucleobases of a given sequence are matched to the other nucleobase sequence according to base pairing rules. For example, in some aspects, complementarity between a given sequence and the other nucleobase sequence can be about 70%, about 75%, about 80%, about 85%, about 90%, or about 95%. Accordingly, in certain aspects, the term "complementary" refers to at least about 80%, at least about 85%, at least about 90%, at least about 91%, at least about 92%, at least about 93%, at least about 94%, at least about 95%, at least about 96%, at least about 97%, at least about 98%, or at least about 99% match or complementarity to a target nucleic acid sequence. Or, there can be "complete" or "perfect" (100%) complementarity between a given nucleobase sequence and the other nucleobase sequence. In some aspects, the degree of complementarity between sequences has effects on the efficiency and strength of hybridization between the sequences.

As used herein, the expression "contiguous or non-contiguous nucleotides are truncated" is meant to include contiguous or non-contiguous nucleotide sequences remaining as a result of the truncation compared to the wild type (or original) sequence, but not to include any process of truncation. The term can include "truncation of contiguous nucleotides", "truncation of non-contiguous nucleotides remaining untruncated between truncated nucleotides", and "insertion of different types of nucleotides into positions of truncated contiguous or non-contiguous nucleotides". For example, in the case of the nucleotide sequence "A-T-G-C-C-G-T-C", the truncation of contiguous nucleotides includes truncation of one or more contiguous nucleotides, such as "A-_-_-_-C-G-T-C", the truncation of non-contiguous nucleotides means that one or more nucleotides remain untruncated between truncated nucleotides, such as "A-_-G-_-C-G-T-_", and the insertion of different types of nucleotides into positions of truncated contiguous or non-contiguous nucleotides means that one or more nucleotides different from the original ones are inserted at the positions of truncated nucleotides, such as "A-A-G-_-C-G-T-G".

The term "downstream" refers to a nucleotide sequence that is located 3' to a reference nucleotide sequence. In certain aspects, downstream nucleotide sequences relate to sequences that follow the starting point of transcription. For example, the translation initiation codon of a gene is located downstream of the start site of transcription.

As used herein, the term "enhancer" refers to a segment of DNA which contains sequences capable of providing enhanced transcription and in some instances can function independent of their orientation relative to another control sequence. An enhancer can function cooperatively or additively with promoters and/or other enhancer elements.

The terms "excipient" and "carrier" are used interchangeably and refer to an inert substance added to a pharmaceutical composition to further facilitate administration of a compound, e.g., a polynucleotide comprising a transgene and an untranslated nucleic acid sequence described herein.

The term "exon" refers to a defined section of nucleic acid that encodes for a protein, or a nucleic acid sequence that is represented in the mature form of an RNA molecule after either portions of a pre-processed (or precursor) RNA have been removed by splicing. The mature RNA molecule can be a messenger RNA (mRNA) or a functional form of a non-coding RNA, such as rRNA or tRNA.

The term "expression," as used herein, refers to a process by which a polynucleotide produces a gene product, e.g., RNA or a polypeptide. It includes, without limitation, transcription of the polynucleotide into messenger RNA (mRNA), and the translation of mRNA into a polypeptide. Expression produces a "gene product." As used herein, a gene product can be, e.g., a nucleic acid, such as an RNA produced by transcription of a gene. As used herein, a gene product can be either a nucleic acid or a polypeptide which is translated from a transcript. Gene products described herein further include nucleic acids with post transcriptional modifications, e.g., polyadenylation or splicing, or polypeptides with post translational modifications, e.g., phosphorylation, methylation, glycosylation, the addition of lipids, association with other protein subunits, or proteolytic cleavage.

As used herein, the term "identity" refers to the overall monomer conservation between polymeric molecules, e.g., between polynucleotide molecules. The term "identical" without any additional qualifiers, e.g., polynucleotide A is identical to polynucleotide B, implies the polynucleotide sequences are 100% identical (100% sequence identity). Describing two sequences as, *e.g.,* "70% identical," is equivalent to describing them as having, *e.g.,* "70% sequence identity."

Calculation of the percent identity of two polypeptide or polynucleotide sequences, for example, can be performed by aligning the two sequences for optimal comparison purposes (e.g., gaps can be introduced in one or both of a first and a second polypeptide or polynucleotide sequences for optimal alignment and non-identical sequences can be disregarded for comparison purposes). In certain aspects, the length of a sequence aligned for comparison purposes is at least 30%, at least 40%, at least 50%, at least 60%, at least 70%, at least 80%, at least 90%, at least 95%, or 100% of the length of the reference sequence. The amino acids at corresponding amino acid positions, or bases in the case of polynucleotides, are then compared.

When a position in the first sequence is occupied by the same amino acid or nucleotide as the corresponding position in the second sequence, the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm.

Suitable software programs that can be used to align different sequences (e.g., polynucleotide sequences) are available from various sources. One suitable program to determine percent sequence identity is bl2seq, part of the BLAST suite of program available from the U.S. government's National Center for Biotechnology Information BLAST web site (blast.ncbi.nlm.nih.gov). Bl2seq performs a comparison between two sequences using either the BLASTN or BLASTP algorithm. BLASTN is used to compare nucleic acid sequences, while BLASTP is used to compare amino acid sequences. Other suitable programs are, e.g., Needle, Stretcher, Water, or Matcher, part of the EMBOSS suite of bioinformatics programs and also available from the European Bioinformatics Institute (EBI) at www.ebi.ac.uk/Tools/psa.

Sequence alignments can be conducted using methods known in the art such as MAFFT, Clustal (ClustalW, Clustal X or Clustal Omega), MUSCLE, etc.

Different regions within a single polynucleotide or polypeptide target sequence that aligns with a polynucleotide or polypeptide reference sequence can each have their own percent sequence identity. It is noted that the percent sequence identity value is rounded to the nearest tenth. For example, 80.11, 80.12, 80.13, and 80.14 are rounded down to 80.1, while 80.15, 80.16, 80.17, 80.18, and 80.19 are rounded up to 80.2. It also is noted that the length value will always be an integer.

In certain aspects, the percentage identity (%ID) of a first amino acid sequence (or nucleic acid sequence) to a second amino acid sequence (or nucleic acid sequence) is calculated as %ID = 100 x (Y/Z), where Y is the number of amino acid residues (or nucleobases) scored as identical matches in the alignment of the first and second sequences (as aligned by visual inspection or a particular sequence alignment program) and Z is the total number of residues in the second sequence. If the length of a first sequence is longer than the second sequence, the percent identity of the first sequence to the second sequence will be higher than the percent identity of the second sequence to the first sequence.

One skilled in the art will appreciate that the generation of a sequence alignment for the calculation of a percent sequence identity is not limited to binary sequence-sequence comparisons exclusively driven by primary sequence data. It will also be appreciated that sequence alignments can be generated by integrating sequence data with data from heterogeneous sources such as structural data (e.g., crystallographic protein structures), functional data (e.g., location of mutations), or phylogenetic data. A suitable program that integrates heterogeneous data to generate a multiple sequence alignment is T-Coffee, available at www.tcoffee.org, and alternatively available, e.g., from the EBI. It will also be appreciated that the final alignment used to calculate percent sequence identity can be curated either automatically or manually.

As used herein, the term "intron" refers to section of DNA (intervening sequences) within a gene that does not encode part of the protein that the gene produces, and that is spliced out of the mRNA that is transcribed from the gene before it is exported from the cell nucleus. "Intron sequence" refers to the nucleic acid sequence of the intron. Such sequences are also referred to herein as "untranslated nucleic acid sequence." Thus, introns are those regions of DNA sequences that are transcribed along with the coding sequence (exons) but are removed during the formation of mature mRNA.

As used herein, the term "intron fragment" refers to a fragment derived from a full-length EF-1α intron A sequence *(i.e.,* the first intron of EF-1α, such as that set forth in SEQ ID NO 1). The fragment is meant to exclude full-length EF-1α intron. In some aspects, the "intron fragment" includes a minimum number of nucleotides or elements required to achieve an expression level exceeding that achieved by a corresponding construct in which all nucleotides of EF-1α intron A are lacking. Accordingly, an intron fragment of the present disclosure (also referred to herein as "untranslated nucleic acid sequence") is not particularly limited as long as it comprises a fragment of EF-1α intron and can increase the expression of a transgene. As demonstrated herein, in some aspects, an intron fragment *(i.e.,* untranslated nucleic acid sequence) can increase the transcription of a transgene, resulting in enhanced expression of the transgene. Accordingly, in some aspects, an intron fragment described herein can be an untranslated regulatory element.

As used herein, the terms "isolated," "purified," "extracted," and grammatical variants thereof are used interchangeably and refer to the state of preparation of desired composition of the present disclosure, e.g., a polynucleotide comprising a transgene and an untranslated nucleic acid sequence, that has undergone one or more processes of purification. In some aspects, isolating or purifying as used herein is the process of removing, partially removing (*e.g.,* a fraction) a composition of the present disclosure, e.g., a polynucleotide described herein from a sample containing contaminants.

In some aspects, an isolated composition has no detectable undesired activity or, alternatively, the level or amount of the undesired activity is at or below an acceptable level or amount. In other aspects, an isolated composition has an amount and/or concentration of desired composition of the present disclosure, at or above an acceptable amount and/or concentration and/or activity. In other aspects, the isolated composition is enriched as compared to the starting material from which the composition is obtained. This enrichment can be by at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, at least about 99%, at least about 99.9%, at least about 99.99%, at least about 99.999%, at least about 99.9999%, or greater than 99.9999% as compared to the starting material.

In some aspects, isolated preparations are substantially free of residual biological products. In some aspects, the isolated preparations are 100% free, at least about 99% free, at least about 98% free, at least about 97% free, at least about 96% free, at least about 95% free, at least about 94% free, at least about 93% free, at least about 92% free, at least about 91% free, or at least about 90% free of any contaminating biological matter. Residual biological products can include abiotic materials (including chemicals) or unwanted nucleic acids, proteins, lipids, or metabolites.

The term "linked," as used herein, refers to a first amino acid sequence or polynucleotide sequence covalently or non-covalently joined to a second amino acid sequence or polynucleotide sequence, respectively. The first amino acid or polynucleotide sequence can be directly joined or juxtaposed to the second amino acid or polynucleotide sequence or alternatively an intervening sequence can covalently join the first sequence to the second sequence. The term "linked" means not only a fusion of a first polynucleotide sequence to a second polynucleotide sequence at the 5'-end or the 3'-end, but also includes insertion of the whole first polynucleotide sequence (or the second polynucleotide sequence) into any two nucleotides in the second polynucleotide sequence (or the first polynucleotide sequence, respectively). The first polynucleotide sequence can be linked to a second polynucleotide sequence by a phosphodiester bond or a linker. The linker can be, *e.g.,* a polynucleotide.

As used herein, the term "retinal disease" or "macular disease" refers to a disease, disorder or symptom that affects or is associated with a part or area of the retina or macula. The retinal or macular disease may be a disease, disorder or symptom caused by damage to all or part of the retina or macula. The retinal or macular disease may be caused by the dysfunction or damage of retinal or macular cells. In addition, the retinal or macular disease may be caused by the dysfunction or damage of photoreceptor cells and/or retinal pigment epithelial (RPE) cells of the retina or macula.

NaIO₃ (sodium iodate) has been reported to induce damage and/or degeneration of the retina in part or all of the outer and inner retina and damage to the retinal pigment epithelium (RPE) and photoreceptors (A.E.-H. Koh, et al. Journal of Photochemistry & Photobiology B: Biology 196 (2019) 111514). Therefore, the NaIO₃ model can be used to assess the effectiveness of prevention or treatment of diseases caused by damage to the retina or macula (e.g., dysfunction or damage to photoreceptor cells and/or retinal pigment epithelial cells). Examples of diseases caused by the dysfunction or damage of the photoreceptor cells and/or retinal pigment epithelial cells include retinopathy, choroidal neovascularization, maculopathy, macular degeneration, retinal degeneration, macular edema, retinal edema, macular swelling, retinal swelling, retinal cell degeneration, retinal vascular occlusion, retinal detachment, inherited retinal disease, or a combination thereof.

The term "retinal pigment epithelium" (RPE) used herein refers to a single layer of epithelial cells located at the back of the eye of vertebrates between the choroidal blood supply (choroidal capillary layer) and the neural retina. The RPE acts as one of the components of the blood-retinal barrier, and RPE cells play an important role in maintaining the visual cycle, phagocytosis of the outer segment of the photoreceptor, and transportation of nutrients, metabolic wastes, ions and fluids between the distal retina and the choriocapillaris layer.

As used herein, the term "retinopathy" refers to a disease or damage of the retina (i.e., the tissue lining the inner surface of the back of the eye that captures images passing through the cornea and lens) or retinal cells.

The term "diabetic retinopathy" (DR) used in this specification refers to retinopathy caused by complications related to diabetes. Depending on the severity of the disease, DR can be asymptomatic, cause mild vision problems, or lead to blindness. DR is the result of microvascular retinal changes. Hyperglycemia-induced intramural perivascular cell death and thickening of the basement membrane can lead to vascular wall failure. These damages alter the formation of the blood-retinal barrier and allow higher permeability of the retinal blood vessels. Perivascular cell death may be induced when hyperglycemia persistently activates PKC-δ encoded by protein kinase C-δ and p38 mitogen-activated protein kinase (MAPK) to increase the expression of a previously unknown target of PKC-δ signaling, Src homology 2 domain-containing phosphatase-1 (SHP-1), a protein tyrosine phosphatase. This signaling cascade leads to PDGF receptor-dephosphorylation and a reduction in downstream signaling from this receptor, resulting in "perivascular cell death". Small blood vessels, such as those in the eye, can be especially vulnerable to poor control of blood sugar. Over accumulation of glucose and/or fructose can damage small blood vessels in the retina.

DR can be classified into two separate stages (Wu L., el al., World J Diabetes 4(6): 290-294 (2013)). The first stage, called non-proliferative diabetic retinopathy (NPDR), is associated with early diabetic retinopathy. NPDR usually has no obvious symptoms, or is associated with mild vision distortion caused by blood vessels that leak fluid into surrounding tissues. The only way to detect NPDR is fundus photography, in which microaneurysms (microscopic blood-filled bulges in the artery walls) can be seen. If left untreated, DR can progress to the more advanced second stage called proliferative diabetic retinopathy (PDR). PDR is characterized by abnormal new blood vessel formation (i.e., neovascularization) that can cause rupture and bleeding, which can lead to blurred vision. Other symptoms of PDR include floating spots or dark lines in the field of vision ("floaters"), fluctuating vision, impaired color vision, dark or blank areas in the field of vision, pain, patchy vision and complete loss of vision.

The term "diabetic retinopathy" comprises all types of diabetic retinopathy, including, but not limited to, non-proliferative diabetic retinopathy (NPDR), proliferative diabetic retinopathy (PDR), diabetic maculopathy and diabetic macular edema.

In some aspects, PDR occurs after the onset of NPDR (e.g., subject first diagnosed with NPDR and the disease progresses to PDR). In other aspects, PDR occurs independently of NPDR. The term "diabetic retinopathy" used herein also includes any and all symptoms of diabetic retinopathy, regardless of the cause. Non-limiting examples of the risk factors for diabetic retinopathy include duration of diabetes, heredity, excessive alcohol consumption, smoking, high blood pressure, obesity, dyslipidemia, high cholesterol, kidney disease, pregnancy and kidney damage.

As used herein, the term "maculopathy" refers to any pathological symptom of the macula, which is the central area of the retina that is associated with highly sensitive and accurate vision. In some aspects, the terms "maculopathy" and "retinopathy" can be used interchangeably (i.e., if only the macula is affected). In some aspects, the maculopathy is diabetic maculopathy.

"Diabetic maculopathy" occurs when the macula is affected by retinal changes caused by diabetes. The term includes two distinct eye diseases, diabetic macular edema and diabetic ischemic maculopathy. The two types of maculopathy are often comorbid, i.e., an individual with macular edema also often has ischemic maculopathy. Ischemic maculopathy may occur together with macular edema and can occur even in mild cases of macular edema. In some aspects, retinal changes associated with diabetic maculopathy include decrease in retinal potential, loss of perivascular cells, acellular capillary formation, vascular congestion, vascular dysfunction, vascular leakage, vascular occlusion, tissue swelling (edema), tissue ischemia, or any combination thereof, within the retina of the subject.

As used herein, the term "acellular capillaries" refers to capillary-sized vessel tubes that do not have nuclei anywhere along their length.

The term "vascular congestion" used herein refers to a type of vascular injury which is an important factor in the pathogenesis of various eye diseases (e.g., diabetic macular edema) disclosed herein. Vascular congestion is related to the accumulation of fluid (e.g., blood in the blood vessels) in the vascular tissue. In some aspects, vascular congestion can be caused by hyperglycemia (i.e., high blood sugar).

The term "retinal dystrophy" (RD) used herein refers to retinopathy caused by genetic or other causes (degenerative disorder of the retina).

As used herein, the term "macular degeneration" refers to any number of disorders and conditions in which the central portion of the retina (i.e., the macula) degenerates or loses functional activity. The degeneration or loss of functional activity can arise as a result of, for example, cell death, decreased cell proliferation, loss of normal biological function, or a combination of the foregoing. Macular degeneration can lead to and/or manifest as alterations in the structural integrity of the cells and/or extracellular matrix of the macula, alteration in normal cellular and/or extracellular matrix architecture, and/or the loss of function of macular cells. The cells can be any cell type normally present in or near the macula including RPE cells, photoreceptors, and/or capillary endothelial cells. Age-related macular degeneration is the most common of the macular degeneration, but the term "macular degeneration" does not necessarily exclude macular degeneration in patients who are not the elderly. Non-limiting examples of macular degenerations include age-related macular degeneration (wet or dry), Best's macular dystrophy, Sorsby fundus dystrophy, malattia leventinese, Doyne's honeycomb retinal dystrophy, Stargardt's disease (also called Stargardt macular dystrophy, juvenile macular degeneration, or fundus flavimaculatus), and pigment epithelial detachment associated macular degeneration.

As used herein, the term "age-related macular degeneration" (AMD) refers to a retinopathy which usually affects older individuals and is associated with a loss of central vision, resulting from damage to the central part (i.e., macula) of the retina. AMD is generally characterized by a progressive accumulation or aggregation of yellowish insoluble extracellular deposits, called drusen (buildup of extracellular proteins, such as amyloid beta, and lipids), in the macula (primarily between the retinal pigment epithelium (RPE) and the underlying choroid). The accumulation or aggregation of these deposits within the macula can cause gradual deterioration of the macula, resulting in central vision impairment. As used herein, the term "macula" refers to the central part of the retina that is responsible for central, high-resolution, color vision.

The pathogenesis of age-related macular degeneration is not well known, although some theories have been put forward, including oxidative stress, mitochondrial dysfunction, and inflammatory processes. The imbalance between the production of damaged cellular components and degradation leads to the accumulation of harmful products, for example, intracellular lipofuscin and extracellular drusen. Incipient atrophy is demarcated by areas of retinal pigment epithelium (RPE) thinning or depigmentation that precede geographic atrophy in the early stages of AMD. In advanced stages of AMD, atrophy of the RPE (geographic atrophy) and/or development of new blood vessels (neovascularization) result in the death of photoreceptors and central vision loss. In the dry (nonexudative) AMD, cellular debris called drusen accumulates between the retina and the choroid, causing atrophy and scarring to the retina. In the wet (exudative) AMD, which is more severe, blood vessels grow up from the choroid (neovascularization) behind the retina which can leak exudate and fluid and also cause hemorrhaging.

Depending on the extent of the drusen present, AMD can be categorized into three primary stages: (i) early, (ii) intermediate, and (iii) advanced or late. The early stage AMD is characterized by the presence of either several small (e.g., less than about 63 microns in diameter) drusen or a few medium-sized (e.g., between about 63 to 124 microns in diameter) drusen. During the early stage, patients have no apparent symptoms with no vision loss. The intermediate stage is characterized by the presence of many medium-sized drusen or one or more large (e.g., more than about 125 microns in diameter) drusen. During this stage, some patients can begin to experience blurred spots in the center of their vision. The advanced or late stage AMD is characterized by large areas of damage to the retinal tissue, causing central blind spots and the eventual loss of central vision. Based on the type of damage (e.g., presence or absence of neovascularization), advanced or late stage AMD can be further divided into two subtypes: (i) geographic atrophy (also called atrophic AMD) and (ii) wet AMD (also called neovascular or exudative AMD).

There are two primary forms of AMD: (i) dry AMD and (ii) wet AMD. Unless specified otherwise, the term "age-related macular degeneration" comprises both dry AMD and wet AMD. As used herein, the term "age-related macular degeneration" also comprises all types of age-related macular degeneration regardless of the cause and any and all symptoms of age-related macular degeneration. Non-limiting examples of symptoms associated with macular degeneration (e.g., age-related macular degeneration) include: loss of central vision, distortion, decreased contrast sensitivity, blurred vision, difficulty adapting to low light level, sudden onset and rapid worsening of symptoms, and decreased color vision. In some aspects, macular degeneration (e.g., age-related macular degeneration) can cause macular edema (i.e., swelling of the macula resulting from collection of fluid and protein deposits on or under the macula).

As used herein, the term "dry AMD" (also referred to as atrophic age-related macular degeneration or non-exudative AMD) refers to all forms of AMD that are not wet (neovascular) AMD. This includes early and intermediate forms of AMD, as well as the advanced form of dry AMD known as geographic atrophy. Dry AMD patients tend to have minimal symptoms in the earlier stages; visual function loss occurs more often if the condition advances to geographic atrophy.

As used herein, the term "wet AMD" (also referred to as neovascular age-related macular degeneration or exudative AMD) refers to retinal condition characterized by the presence of retinal neovascularization and is the most advanced form of AMD. In wet AMD, blood vessels grow from the choriocapillaris through defects in Bruch's membrane, and in some cases the underlying retinal pigment epithelium (choroidal neovascularization or angiogenesis). Organization of serous or hemorrhagic exudates escaping from these vessels can result in fibrovascular scarring of the macular region with attendant degeneration of the neuroretina, detachment and tears of the retinal pigment epithelium, vitreous hemorrhage, and permanent loss of central vision.

As used herein, the term "neovascularization" refers to the growth of new abnormal blood vessels in different parts of the eye that can cause bleeding and vision loss. As used herein, the term "choroidal neovascularization" refers to the abnormal growth of new blood vessels in the choroid (i.e., the vascular layer of the eye that contains connective tissues and lies between the retina and the sclera). In wet AMD, new blood vessels can grow into the retina via the retinal pigment epithelium (RPE) and choroid and can impair visual function due to blood and lipid leakage. As used herein, the term "retinal neovascularization" refers to the abnormal development, proliferation and/or growth of blood vessels on or in the retina, for example, on the retinal surface. Retinal neovascularization can occur in many retinopathies associated with retinal ischemia (e.g., diabetic retinopathy, sickle cell retinopathy, Eales disease, ocular ischemic syndrome, carotid-cavernous fistula, familial exudative vitreous retinopathy, hyperviscosity syndrome, radiation retinopathy, retinal vein occlusion, retinal artery occlusion, retinal embolism, birdshot retinochoroidopathy, choroidal melanoma, chronic retinal detachment, anterior ischemic optic neuropathy (AION), non-arteritic anterior ischemic optic neuropathy (NAION), and incontinentia pigmenti). Methods for detecting neovascularization are known in the art and include, but are not limited to, measuement of the expression of CD31 (platelet endothelial cell adhesion molecule, also known as PECAM-1) and vascular endothelial growth factor (VEGF) in tissues. For example, see Schluter A., et al., BMC Cancer 18(1): 272 (2018).

As used herein, the term "inherited retinal disease" refers to a retinal disease in which the structure and function of retinal cells are abnormal due to the defect or abnormality of genes. The timing of onset and symptoms vary depending on the causative genes of the inherited retinal disease. Non-limiting examples of the inherited retinal disease include retinitis pigmentosa (RP), Leber congenital amaurosis, Stargardt's disease, Coats retinopathy, cone dystrophy, choroideremia, Usher syndrome, Best disease, X-linked retinoschisis, and unspecified hereditary retinal dystrophy.

As used herein, the term "retinitis pigmentosa" (RP) refers to a retinal disease in which the optic cells and retinal pigment epithelial cells of the retina are damaged (degenerated). As the optic cells are damaged, night blindness appears in the early stages, and the field of vision narrows gradually, eventually resulting in complete blindness. Defects in genes involved in the conversion of light into electrical signals in the optic cells are the main cause. Some genetic abnormalities are also found in relation to retinal pigment epithelium, and these gene abnormalities can cause extensive retinal damage.

The most conspicuous symptom of retinitis pigmentosa is the gradual destruction of rod cells and cone cells. The rod cells are damaged earlier than the cone cells. When the destruction of optic cells progresses to the cone cells, the central vision is blurred, resulting in complete blindness.

As used herein, the term "Leber congenital amaurosis" refers to an inherited retinal disease that can cause congenital blindness from birth or immediately after birth . Patients with Leber congenital amaurosis do not have rods and cone cells in the retina with normal function, so both cone and rod cell responses are lost in electroretinogram. Leber's congenital melanoma is a disease caused by a genetic abnormality, in which mutations in the gene are found in 40 to 50% of patients. Of the related 12 genes, 11 autosomal genes are inherited recessively (GUCY2D, RPE65, SPATA7, AIPL1, LCA5, RPGRIP1, CRB1, CEP290, IMPDH1, RD3 and RDH12) and, rarely, one autosomal gene is inherited dominantly (CRX).

As used herein, the term "Stargardt's disease" refers to a type of retinal dystrophy inherited autosomal recessively. Stargardt's disease appears between the ages of 8 and 15. Gradual loss of central vision occurs due to macular degeneration of both eyes. At present, it is thought that the mutation of a gene called ABCA4 is the cause of Stargardt's disease. The mutation of ABCA4 causes the accumulation of a substance similar to lipofuscin in the retinal pigment epithelial layer, resulting in RPE apoptosis and loss of photoreceptors. The mutation of ABCA4 is associated with dysplasia of cone and rod cells, as well as severe retinal dystrophy.

As used herein, the term "Coats retinopathy" refers to a retinal vascular disease that causes dilation of retinal capillaries and vascular flow, resulting in the accumulation of exudate in the intraretinal and subretinal spaces and causing exudative retinal detachment. Somatic mutations in the NDP gene on the X chromosome are known to cause the deficiency of the norrin protein, which is necessary for the development of the retina.

As used herein, the term "cone dystrophy" refers to a disease in which cone cells responsible for color vision and central vision among the cells of the retina are denatured due to genetic abnormalities, resulting in loss of central vision. The cone dystrophy is broadly classified into pure cone dystrophy and cone-rod dystrophy. The inheritance pattens is various, such as autosomal dominant, autosomal recessive, sex chromosome-linked, etc. also, it is not inherited and can be caused by mutation.

As used herein, the term "choroideremia" refers to a rare X-chromosome-linked progressive degeneration of the choroid, retinal pigment epithelium, and photoreceptors. In patients, mutation in the CHM gene causes deficiency of the Rab escort protein 1 (REP1) protein. As a result, photoreceptor cells in the retina lose their functions and die gradually. It is known that the symptoms of the disease usually occur in males. It is also known that night blindness is typically the first symptom occurring in childhood, peripheral vision decreases as the disease progresses, and central vision is preserved to some extent in the early stages. In general, male patients in their 40s have good eyesight, but their field of vision is very narrow, and they lose their vision by the age of 50 to 70. Decrease in color perception occurs in some patients.

As used herein, the term "Usher syndrome" refers to a genetic disorder in which visual impairment progresses along with deafness. In the Usher syndrome, auditory impairment is due to abnormalities in the inner ear and visual impairment is associated with retinal pigmentation (RP).

As used herein, ,the term "Best disease" refers to an inherited retinal disease caused by mutations in the BEST1 (VMD2) gene. It progresses slowly and can cause decline in central vision. Mutations in the BEST1 (VMD2) gene can lead to abnormal function of bestrophin, a calcium-chlorine channel protein in the basal plasma membrane of the retinal pigment epithelium, which can lead to impaired water transport through the retinal pigment epithelium, resulting in serous retinal detachment or retinal pigment epithelial detachment.

As used herein, the term "X-linked retinoschisis" refers to a disease in which interlayer separation of the medial retina occurs due to mutation in the retinoschisis gene (RS 1). Retinoschisis causes visual impairment due to abnormal separation of the nerve fiber layer among the 10 layers that make up the retina. Juvenile retinoschisis is a rare X-chromosome recessive genetic disease with a prevalence of 1:120,000 worldwide.

The terms "miRNA", "miR", and "microRNA" are used interchangeably and refer to a microRNA molecule found in eukaryotes that is involved in RNA-based gene regulation. The term will be used to refer to the single-stranded RNA molecule processed from a precursor. In some aspects, the term "antisense oligomers" can also be used to describe the microRNA molecules of the present disclosure. Names of miRNAs and their sequences related to the present disclosure are provided herein. MicroRNAs recognize and bind to target mRNAs through imperfect base pairing leading to destabilization or translational inhibition of the target mRNA and thereby downregulate target gene expression. Conversely, targeting miRNAs via molecules comprising a miRNA binding site (generally a molecule comprising a sequence complementary to the seed region of the miRNA) can reduce or inhibit the miRNA-induced translational inhibition leading to an upregulation of the target gene.

"Nucleic acid", "nucleic acid molecule", "nucleotide sequence", "polynucleotide", and grammatical variants thereof are used interchangeably and refer to a sequence of nucleotides connected by phosphodiester linkages. Polynucleotides are presented herein in the direction from the 5' to the 3' direction. A polynucleotide of the present disclosure can be a deoxyribonucleic acid (DNA) molecule or ribonucleic acid (RNA) molecule. Nucleotide bases are indicated herein by a single letter code: adenine (A), guanine (G), thymine (T), cytosine (C), inosine (I) and uracil (U).

As used herein, the term "operatively linked" or "operably linked" means that DNA sequences to be linked are located adjacent to each other to perform a desired function. For instance, a promoter is operatively linked to a coding region if the promoter helps initiate transcription of the coding sequence (*e.g.,* transgene). As long as this functional relationship is maintained, the promoter needs not be contiguous with the coding region.

The terms "pharmaceutically acceptable carrier," "pharmaceutically acceptable excipient," and grammatical variations thereof, encompass any of the agents approved by a regulatory agency of the U.S. Federal government or listed in the U.S. Pharmacopeia for use in animals, including humans, as well as any carrier or diluent that does not cause the production of undesirable physiological effects to a degree that prohibits administration of the composition to a subject and does not abrogate the biological activity and properties of the administered compound. Included are excipients and carriers that are useful in preparing a pharmaceutical composition and are generally safe, non-toxic, and desirable.

As used herein, the term "pharmaceutical composition" refers to one or more of the compositions described herein (*e.g.,* polynucleotides, vectors, cells, and/or recombinant viruses) mixed or intermingled with, or suspended in one or more other chemical components, such as pharmaceutically acceptable carriers and excipients.

As used herein, the terms "promoter" and "promoter sequence" are interchangeable and refer to a DNA sequence capable of controlling the expression of a coding sequence or functional RNA. In general, a coding sequence is located 3' to a promoter sequence. Promoters can be derived in their entirety from a native gene, or be composed of different elements derived from different promoters found in nature, or even comprise synthetic DNA segments. It is understood by those skilled in the art that different promoters can direct the expression of a gene in different tissues or cell types, or at different stages of development, or in response to different environmental or physiological conditions. Promoters that cause a gene to be expressed in most cell types at most times are commonly referred to as "constitutive promoters." Promoters that cause a gene to be expressed in a specific cell type are commonly referred to as "cell-specific promoters" or "tissue-specific promoters." Promoters that cause a gene to be expressed at a specific stage of development or cell differentiation are commonly referred to as "developmentally-specific promoters" or "cell differentiation-specific promoters." Promoters that are induced and cause a gene to be expressed following exposure or treatment of the cell with an agent, biological molecule, chemical, ligand, light, or the like that induces the promoter are commonly referred to as "inducible promoters" or "regulatable promoters." It is further recognized that since in most cases the exact boundaries of regulatory sequences have not been completely defined, DNA fragments of different lengths can have identical promoter activity.

The promoter sequence is typically bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase. In some aspects, a promoter that can be used with the present disclosure includes a tissue specific promoter.

As used herein, the term "gene regulatory region" or "regulatory region" refers to nucleotide sequences located upstream (5' non-coding sequences), within, or downstream (3' non-coding sequences) of a coding region, and which influence the transcription, RNA processing, stability, or translation of the associated coding region. Regulatory regions can include promoters, translation leader sequences, introns, polyadenylation recognition sequences, RNA processing sites, effector binding sites, or stem-loop structures. If a coding region is intended for expression in a eukaryotic cell, a polyadenylation signal and transcription termination sequence will usually be located 3' to the coding sequence.

In some aspects, a polynucleotide described herein (e.g., comprising a transgene and an untranslated nucleic acid sequence) can include a promoter and/or other expression (e.g., transcription) control elements operably associated with one or more coding regions. In an operable association, a coding region for a gene product is associated with one or more regulatory regions in such a way as to place expression of the gene product under the influence or control of the regulatory region(s). For example, a coding region and a promoter are "operably associated" if induction of promoter function results in the transcription of mRNA encoding the gene product encoded by the coding region, and if the nature of the linkage between the promoter and the coding region does not interfere with the ability of the promoter to direct the expression of the gene product or interfere with the ability of the DNA template to be transcribed. Other expression control elements, besides a promoter, for example enhancers, operators, repressors, and transcription termination signals, can also be operably associated with a coding region to direct gene product expression.

As used herein, the term "subject," "patient," "individual," and "host," and variants thereof, are interchangeable and refer to any mammalian subject to which any of the compositions described herein (e.g., polynucleotides, the recombinant expression constructs, cells, pharmaceutical compositions, or recombinant viruses) are administered. Non-limiting examples include humans, domestic animals *(e.g.,* dogs, cats and the like), farm animals (*e.g.,* cows, sheep, pigs, horses and the like), and laboratory animals (*e.g.,* monkey, rats, mice, rabbits, guinea pigs and the like) for whom diagnosis, treatment, or therapy is desired, particularly humans. The methods described herein are applicable to both human therapy and veterinary applications.

As used herein, the phrase "subject in need thereof" includes subjects, such as mammalian subjects, that would benefit from administration of composition described herein.

As used herein, the term "therapeutically effective amount" is the amount of reagent or pharmaceutical compound comprising a composition of the present disclosure (*e.g.,* polynucleotide comprising a transgene and an untranslated nucleic acid sequence) that is sufficient to a produce a desired therapeutic effect, pharmacologic and/or physiologic effect on a subject in need thereof. A therapeutically effective amount can be a "prophylactically effective amount" as prophylaxis can be considered therapy.

As used herein, the term "transgene" refers to at least one polynucleotide or polynucleotide region encoded in a recombinant expression construct or an expression product of the polynucleotide or polynucleotide region, a polynucleotide encoding a polypeptide or multi-polypeptide or a modulatory or regulatory nucleic acid. In some aspects, the transgene can be heterologous to the cell (i.e., not naturally expressed in the cell) in which it is inserted (or transduced).

The terms "treat," "treatment," or "treating," as used herein refers to, e.g., the reduction in severity of a disease or condition; the reduction in the duration of a disease course; the amelioration or elimination of one or more symptoms associated with a disease or condition; the provision of beneficial effects to a subject with a disease or condition, without necessarily curing the disease or condition. The term also includes prophylaxis or prevention of a disease or condition or its symptoms thereof.

The term "upstream" refers to a nucleotide sequence that is located 5' to a reference nucleotide sequence.

As used herein, the term "vector" or "construct" refers to any vehicle into which a nucleic acid or a gene can be inserted, such as delivery vehicles into which a nucleic acid sequence can be inserted for introduction into a cell where it can be replicated. The nucleic acid sequence which can be inserted into a vector can be exogenous or heterologous. The nucleic acid sequence can be a transgene. Examples of constructs include, but are not limited to, plasmids, cosmids and viruses (*e.g.,* AAVs). Those skilled in the art can construct the vector or construct through standard recombinant techniques (Maniatis, et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994, etc.). As used herein, the term "expression vector" or "expression construct" refers to a vector or construct including a nucleotide sequence coding for at least a portion of a gene product to be transcribed. In some cases, RNA molecules are then translated into a protein, polypeptide or peptide. Expression constructs can include various control elements. In addition to regulatory sequences that govern transcription and translation, vectors and expression vectors can include nucleotide sequence that serve other functions as well. The viruses that can be used in the present disclosure include retroviruses, herpes simplex viruses, lentiviruses, poxviruses, vaccinia viruses, rhabdoviruses, adenoviruses, helper-dependent adenoviruses, adeno-associated viruses (AAV), etc., although not being limited thereto.

Vectors can be engineered to encode selectable markers or reporters that provide for the selection or identification of cells that have incorporated the vector. Expression of selectable markers or reporters allows identification and/or selection of host cells that incorporate and express other coding regions contained on the vector. Examples of selectable marker genes known and used in the art include: genes providing resistance to ampicillin, streptomycin, gentamycin, kanamycin, hygromycin, bialaphos herbicide, sulfonamide, and the like; and genes that are used as phenotypic markers, i.e., anthocyanin regulatory genes, isopentanyl transferase gene, and the like. Examples of reporters known and used in the art include: luciferase (Luc), green fluorescent protein (GFP), chloramphenicol acetyltransferase (CAT), β-galactosidase (LacZ), β-glucuronidase (Gus), and the like. Selectable markers can also be considered to be reporters.

In general, recombinant adeno-associated viruses (AAVs) are produced by triple transfection of host cells (e.g., HEK293 cells) by 1) AAV construct plasmid comprising gene expression cassettes flanked by inverted terminal repeats (ITRs), 2) "Rep-Cap plasmid" that provide Rep proteins necessary for replication of adeno-associated viral genomes and capsid proteins that constitute viral particles, and finally 3) "helper plasmid" that provide adenovirus proteins (E2a, E4) and RNA (VA RNA) that aid the life cycle of AAVs. AAVs are produced when HEK293 cells, etc., which provide the E1 and E3 genes of adenoviruses, are transfected with these three plasmids.

As used herein, the term "dual helper plasmid" refers to a plasmid that can provide two or more of the requirements for producing AAV in cells. As is evident from the present disclosure, in some aspects, the dual helper plasmid described herein provide the requirements of 2) "plasmid" and 3) "helper plasmid" described above. For example, in some aspects, the dual helper plasmid comprises the rep gene, the cap gene, the E2a gene, the E4 gene and the VA RNA gene.

The dual helper plasmid described herein comprises the genes described above, as well as the genes are arranged in a certain order within the plasmid. For example, in some aspects, the E2a gene, the E4 gene and the VA RNA gene are sequentially linked within the dual helper plasmid, while the rep gene and the cap gene (collectively referred to herein as the "rep-cap gene") are linked sequentially in a clockwise direction (from 5' to 3') between the 5'-end of the E2a gene and the 3'-end of the VA RNA gene. More specifically, in some aspects, the 5'-end of the rep-cap gene is linked to the 5'-end of the E2a gene, and wherein the 3'-end of the rep-cap gene is linked to the 3'-end of the VA RNA gene. In some aspects, the E2a gene, the E4 gene and the VA RNA genes are sequentially linked, and the rep-cap gene is linked in a counterclockwise direction (from 3' to 5') between the 5'-end of the E2a gene and the 3'-end of the VA RNA gene. More specifically, in some aspects, the 3'-end of the rep-cap gene is linked to the 5'-end of the E2a gene, and wherein the 5'-end of the rep-cap gene is linked to the 3'-end of the VA RNA gene.

As used herein,, the term "cell" includes eukaryotic and prokaryotic cells, and refers to any transformable cell capable of replicating the vector described above or expressing a gene encoded by the vector. The cells can be transfected, transduced or transformed by the vector, which refers to a process by which exogenous polynucleotides (nucleic acid molecules) are delivered or introduced into the host cell. As used herein,, the term "transformation" is used to include transfection and transduction.

The (host) cells of the present disclosure include, but are not limited to, specifically insect cells or mammalian cells, more specifically insect cells such as Sf9 cells, or mammalian cells such as HEK293 cells, HeLa cells, ARPE-19 cells, RPE-1 cells, HepG2 cells, Hep3B cells, Huh-7 cells, C8D1a cells, Neuro2A cells, CHO cells, MES13 cells, BHK-21 cells, COS7 cells, COP5 cells, A549 cells, MCF-7 cells, HC70 cells, HCC1428 cells, BT-549 cells, PC3 cells, LNCaP cells, Capan-1 cells, Panc-1 cells, MIA PaCa-2 cells, SW480 cells, HCT166 cells, LoVo cells, A172 cells, MKN-45 cells, MKN-74 cells , Kato-III cells, NCI-N87 cells, HT-144 cells, SK-MEL-2 cells, SH-SY5Y cells, C6 cells, HT-22 cells, PC-12 cells, NIH3T3 cells, etc. In some aspects, the host cell may be an isolated host cell.

As used herein, the term "family with sequence similarity 19" or "FAM19" or "TAFA" refers to a protein that belongs to the TAFA family of five proteins (also known as the FAM19 family) and is primarily expressed in the brain and the spinal cord. FAM19A1 is also known as TAFA1, FAM19A2 as TAFA2, FAM19A3 as TAFA3, FAM19A4 as TAFA4, and FAM19A5 as TAFA5.

The human TAFA1 gene encodes a sequence of 133 amino acids, and although there is disagreement over the length of the signal peptide and mature protein, the human TAFA1 protein (SEQ ID NO 302) is expected to consist of a signal sequence of 35 amino acids and a mature protein of 98 amino acids. TAFA1 is highly expressed in the frontal, temporal, occipital and parietal cortices , and expressed at lower levels in the basal ganglia, lateral tubes and cerebellum. Through cell experiments, TAFA1 has been shown to influence the determination of the differentiation fate of neural stem cells, inhibit the differentiation of neural stem cells into astrocytes, and promote their differentiation into nerve cells. Experiments using TAFA1 knock-out (KO) mice have shown that TAFA1 can regulate locomotor activity, anxiety-like behaviors, learning and memory, and somatosensory functions.

The human TAFA2 gene encodes a sequence of 131 amino acids, and although there is disagreement over the length of the signal peptide and mature protein, the human TAFA2 protein (SEQ ID NO 303) is expected to consist of a signal sequence of 30 amino acids and a mature protein of 101 amino acids. TAFA2 is expressed most abundantly in the occipital, frontal cortices, and medulla oblongata within the central nervous system. It is known that, when recombinant TAFA2 protein is injected into the third ventricle of mice, the food intake and meal frequency, energy expenditure, and respiratory exchange rate are increased. This suggests that TAFA2 may play a role in regulating food intake and energy metabolism. It is also known that inhibition of TAFA2 in zebrafish and mice increases anxiety-like behaviors.

The human TAFA3 gene encodes a sequence of 133 amino acids, and although there is disagreement over the length of the signal peptide and mature protein, the human TAFA3 protein (SEQ ID NO 304) is expected to consist of a signal sequence of 30 amino acids and a mature protein of 103 amino acids. It is known that, in a mouse model of transient focal cerebral ischemia, the expression of TAFA3 is increased in microglia and the microglia treated with TAFA3 are polarized into anti-inflammatory microglia. In addition, when TAFA3 is knocked out in a mouse model, three major behavioral deficits are observed on the autism spectrum disorders, such as decreased response to social novelty, impaired social communication, and increased repetitive behavior. This suggests that TAFA3 is involved in normal functioning of social relationships formation.

The human TAFA4 gene encodes a sequence of 140 amino acids, and although there is disagreement over the length of the signal peptide and mature protein, the human TAFA4 protein (SEQ ID NO 299) is expected to consist of a signal sequence of 45 amino acids and a mature protein of 95 amino acids. TAFA4 is expressed mainly in sensory neurons of the peripheral nervous system. The TAFA4 protein is expressed specifically in C low-threshold mechanoreceptors and appears to reduce pain by regulating the activity of interneurons, especially GABAergic neurons.

The human TAFA5 gene encodes a sequence of 132 amino acids, and although there is disagreement over the length of the signal peptide and mature protein, the human TAFA5 protein is expected to consist of a signal peptide of 43 amino acids and a mature protein of 89 amino acids. TAFA5 is highly expressed in the basal ganglia region and cerebellum. It is known that the expression of TAFA5 in the hypothalamus of mice is increased by inflammatory stimuli such as TNF-α and that, when TAFA5 is knocked out, suppression of food intake, body weight loss and increased inflammatory cytokines induced by TNF-α are partially reversed.

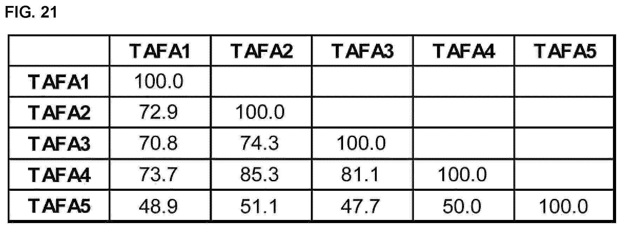

Each of the five TAFA families is expected to have different functions in vivo, however, in the case of TAFA1 to TAFA4 mature proteins, they exhibit high sequence identity (e.g., human TAFA4 has 73.7% sequence identity with TAFA1, 85.3% sequence identity with TAFA2, and 81.1% sequence identity with TAFA3). On the other hand, TAFA5 is expected to have a completely different function since it exhibits low sequence identity with TAFA1 to 4 (e.g., human TAFA5 has 48.9% sequence identity with TAFA1, 51.1% sequence identity with TAFA2, 47.7% sequence identity with TAFA3, and 50.0% sequence identity with TAFA4) (see FIG. 21).

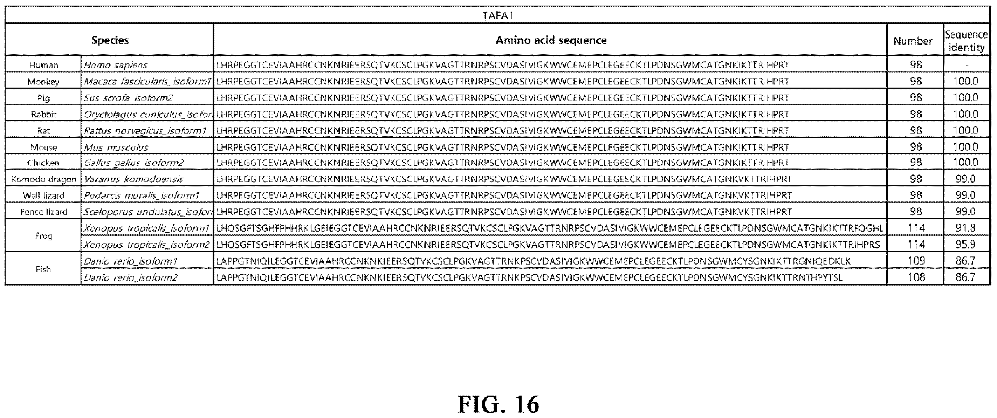

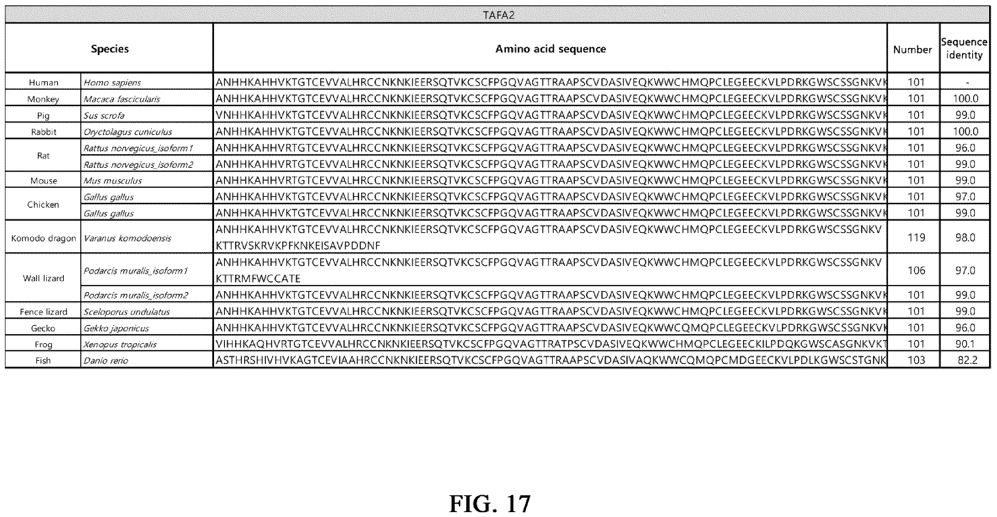

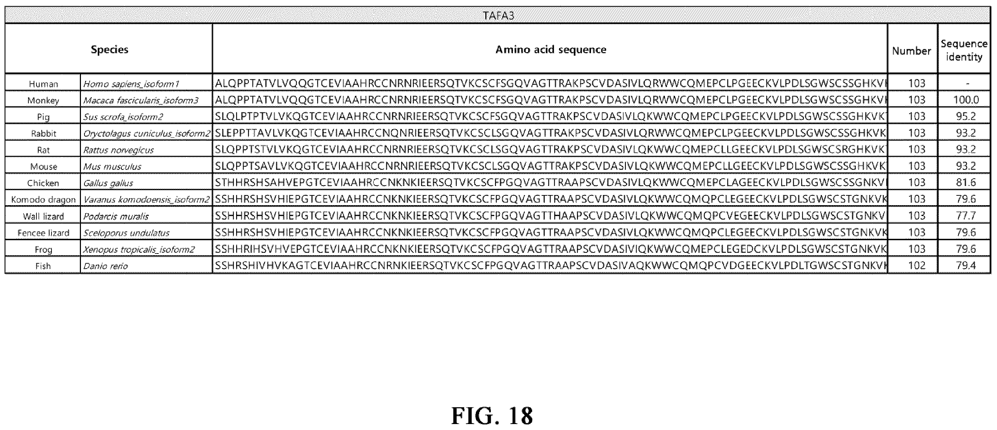

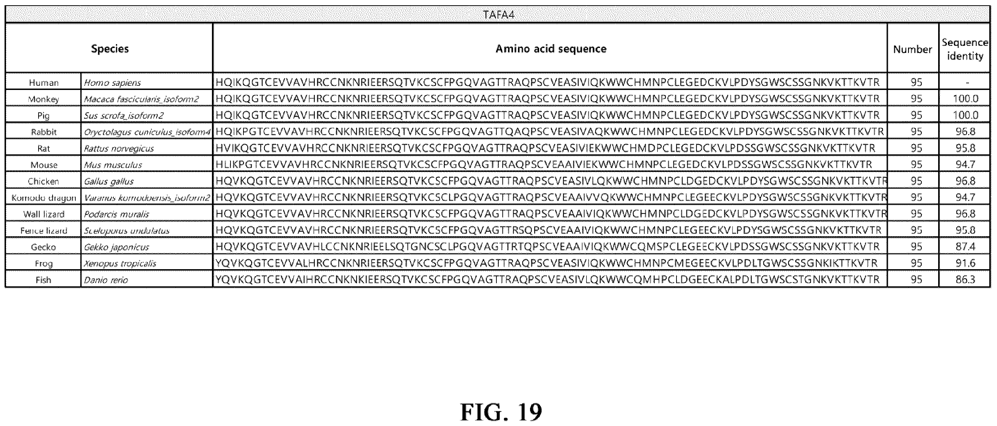

Human TAFA4 (TAFA chemokine-like family member 4) protein (SEQ ID NO 299) is a member of the TAFA protein family (TAFA1-5) that is abundantly expressed throughout brain regions. TAFA4, consisting of 140 amino acids, has 95 amino acids (SEQ ID NO 171), except for the 45 amino acids in the preceding part, which are evolutionarily very well conserved among vertebrates. In addition, among the TAFA protein groups, TAFA1 to 4 are found to have very well conserved interspecies sequence identity in vertebrates. i) The human mature TAFA4 amino acid sequence shows sequence identity 90% or higher compared to TAFA4 in mammals and amphibians, 95% or higher compared to TAFA4 in birds, 85% or higher compared to TAFA4 in reptiles and fish (see FIG. 19). ii) The human TAFA1 amino acid sequence shows sequence identity 90% or higher compared to TAFA1 in mammals and amphibians, 95% or higher compared to TAFA1 in birds, and 85% or higher compared to TAFA1 in reptiles and fish (see FIG. 16). iii) The human TAFA2 amino acid sequence shows sequence identity 90% or higher compared to TAFA2 in mammals and amphibians, 95% or higher compared to TAFA2 in birds, and 80% or higher compared to TAFA2 in reptiles and fish (see FIG. 17). And, iv) the human TAFA3 amino acid sequence shows sequence identity 75% or higher compared to TAFA3 in mammals and amphibians, 80% or higher compared to TAFA3 in birds, and 75% or higher compared to TAFA3 in reptiles and fish (see FIG. 18). It can be seen that they exhibit very high sequence identity regardless of the species.

### II. Polynucleotides

### II.A. Untranslated Nucleic Acid Sequences

The present disclosure is directed to polynucleotides comprising an untranslated nucleic acid sequence, wherein the untranslated nucleic acid sequence is capable of increasing the expression of a transgene when translated. Specifically, provided herein are elongation factor-1 alpha (EF-1α) intron sequences that are shorter than the full-length EF-1α intron.

Elongation factor 1 alpha (EF-1α) is a gene located on chromosome 6 (nucleotides 73,489,308-73,525,587 of GenBank Accession Number NC_000006.12; minus strand orientation). The EF-1α gene includes 8 exons and 7 introns, and encodes the eukaryotic elongation factor 1A (also known as eEF1A1 and eEF1A) protein which plays an important role in mRNA translation (*e.g.,* carries aminoacyl-tRNA to the A site of the ribosome as a ternary complex eEF1A1-GTP-aa-tRNA). See Scaggiante et al., Atlas Genet Cytogenet Oncol Haematol 19(4): 256-265 (Mar. 2015). The nucleotide sequence of the full-length EF-1α intron is set forth in SEQ ID NO: 1 (924 nucleotides long). As described herein, the untranslated nucleic acid sequences of the present disclosure *(i.e.,* EF-1α intron fragment sequences) provide distinct advantages over the full-length EF-1α intron (or any other intron known in the art). For instance, in some aspects, the untranslated nucleic acid sequences described herein can increase the expression of a transgene to a greater extent than the full-length EF-1α intron. Also, because the untranslated nucleic acid sequences of the present disclosure are shorter than the full-length counterpart, in some aspects, they can be used in combination with larger transgenes. For instance, an AAV capsid (such as those described herein) can accommodate a maximum of about 4.7 kb of nucleic acid. Accordingly, in some aspects, with the EF-1α intron fragments described herein *(i.e.,* untranslated nucleic acid sequences), it is possible to incorporate larger transgenes and/or additional cis-elements for much enhanced gene expression.

In some aspects, an untranslated nucleic acid sequence described herein *(i.e.,* the EF-1α intron fragment) comprises nucleotides at positions 874 to 924 of the sequence set forth in SEQ ID NO: 1, but does not comprise SEQ ID NO: 1. In some aspects, an untranslated nucleic acid sequence consists essentially of nucleotides 874-924 of SEQ ID NO: 1. In some aspects, an untranslated nucleic acid sequence consists of nucleotides 874-924 of SEQ ID NO: 1. Such an EF-1α intron fragment is also referred to herein as "T3.2 fragment" and set forth in SEQ ID NO 57. In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 874 to 924 of SEQ ID NO: 1, wherein the untranslated nucleic acid sequence does not comprise SEQ ID NO: 1.

In some aspects, an untranslated nucleic acid sequence described herein *(i.e.,* the EF-1α intron fragment) comprises nucleotides 852-924 of SEQ ID NO: 1, but does not comprise SEQ ID NO: 1. In some aspects, an untranslated nucleic acid sequence of the present disclosure consists essentially of nucleotides 852-924 of SEQ ID NO: 1. In some aspects, an untranslated nucleic acid sequence consists of nucleotides 852-924 of SEQ ID NO: 1. Such an EF-1α intron fragment is also referred to herein as "T3.1.2 fragment" and set forth in SEQ ID NO: 3. In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 852-924 of SEQ ID NO: 1, wherein the untranslated nucleic acid sequence does not comprise SEQ ID NO: 1.

In some aspects, an untranslated nucleic acid sequence described herein *(i.e.,* the EF-1α intron fragment) comprises nucleotides 830-924 of SEQ ID NO: 1, but does not comprise SEQ ID NO: 1. In some aspects, an untranslated nucleic acid sequence of the present disclosure consists essentially of nucleotides 830-924 of SEQ ID NO: 1. In some aspects, an untranslated nucleic acid sequence consists of nucleotides 830-924 of SEQ ID NO: 1. Such an EF-1α intron fragment is also referred to herein as "T3.1.1 fragment" and set forth in SEQ ID NO: 2. In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 830-924 of SEQ ID NO: 1, wherein the untranslated nucleic acid sequence does not comprise SEQ ID NO: 1.

In some aspects, the non-translated nucleic acid sequence (i.e., EF-1α intron fragment) described herein consists of 29 to 174 nucleotides, and the non-translated nucleic acid sequence comprises a nucleotide sequence having at least about 70% sequence identity with the nucleotide sequence represented by SEQ ID NO 57. In some aspects, the non-translated nucleic acid sequence consists of 51 to 117 nucleotides, and the non-translated nucleic acid sequence comprises a nucleotide sequence having sequence identity of at least about 70% with the nucleotide sequence represented by SEQ ID NO 57. In some aspects, the non-translated nucleic acid sequence consists of 51 to 117 nucleotides, and the non-translated nucleic acid sequence comprises the nucleotide sequence represented by SEQ ID NO 57.

In some aspects, the untranslated nucleic acid sequence of a polynucleotide described herein has at least about 70%, at least about 80%, at least about 85%, at least about 90%, at least about 95%, at least about 96%, at least about 97%, at least about 98%, at least about 99%, or about 100% sequence identity to (i) nucleotides 871 to 924 of SEQ ID NO: 1 *(i.e.,* SEQ ID NO: 58), (ii) nucleotides 861 to 924 of SEQ ID NO: 1 *(i.e.,* SEQ ID NO: 59), (iii) nucleotides 852 to 924 of SEQ ID NO: 1 *(i.e.,* SEQ ID NO: 60), (iv) nucleotides 851 to 924 of SEQ ID NO: 1 *(i.e.,* SEQ ID NO: 61), (v) nucleotides 830 to 924 of SEQ ID NO: 1 *(i.e.,* SEQ ID NO: 2), (vi) nucleotides 821 to 924 of SEQ ID NO: 1 *(i.e.,* SEQ ID NO: 63), (vii) nucleotides 811 to 924 of SEQ ID NO: 1 *(i.e.,* SEQ ID NO: 64), (viii) nucleotides 808 to 924 of SEQ ID NO: 1 *(i.e.,* SEQ ID NO: 65), (ix) nucleotides 801 to 924 of SEQ ID NO: 1 *(i.e.,* SEQ ID NO: 66), (x) nucleotides 751 to 924 of SEQ ID NO: 1 *(i.e.,* SEQ ID NO: 67), (xi) nucleotides 721 to 924 of SEQ ID NO: 1 *(i.e.,* SEQ ID NO: 68), (xii) nucleotides 701 to 924 of SEQ ID NO: *1 (i.e.,* SEQ ID NO: 69), (xiii) nucleotides 651 to 924 of SEQ ID NO: 1 *(i.e.,* SEQ ID NO: 70), (xiv) nucleotides 601 to 924 of SEQ ID NO: 1 *(i.e.,* SEQ ID NO: 71), (xv) nucleotides 570 to 924 of SEQ ID NO: 1 *(i.e.,* SEQ ID NO: 72), (xvi) nucleotides 551 to 924 of SEQ ID NO: 1 *(i.e.,* SEQ ID NO; 73), or (xvii) nucleotides 501 to 924 of SEQ ID NO: 1 *(i.e.,* SEQ ID NO: 74).

In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 871 to 924 of SEQ ID NO: 1 *(i.e.,* SEQ ID NO: 58). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 861 to 924 of SEQ ID NO: 1 *(i.e.,* SEQ ID NO: 59). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 852 to 924 of SEQ ID NO: 1 *(i.e.,* SEQ ID NO: 60). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 851 to 924 of SEQ ID NO: 1 *(i.e.,* SEQ ID NO: 61). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 830 to 924 of SEQ ID NO: 1 *(i.e.,* SEQ ID NO: 2). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 821 to 924 of SEQ ID NO: 1 *(i.e.,* SEQ ID NO: 63). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 811 to 924 of SEQ ID NO: 1 *(i.e.,* SEQ ID NO: 64). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 808 to 924 of SEQ ID NO: 1 *(i.e.,* SEQ ID NO: 65). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 801 to 924 of SEQ ID NO: 1 *(i.e.,* SEQ ID NO: 66). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 751 to 924 of SEQ ID NO: 1 *(i.e.,* SEQ ID NO: 67). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 721 to 924 of SEQ ID NO: 1 *(i.e.,* SEQ ID NO: 68). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 701 to 924 of SEQ ID NO: 1 *(i.e.,* SEQ ID NO: 69). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 651 to 924 of SEQ ID NO: 1 *(i.e.,* SEQ ID NO: 70). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 601 to 924 of SEQ ID NO: 1 *(i.e.,* SEQ ID NO: 71). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 570 to 924 of SEQ ID NO: 1 *(i.e.,* SEQ ID NO: 72). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to, nucleotides 551 to 924 of SEQ ID NO: 1 *(i.e.,* SEQ ID NO: 73). In some aspects, the untranslated nucleic acid sequence has at least about 70%, at least about 80%, at least about 85%, at least about 90%, at least about 95%, at least about 96%, at least about 97%, at least about 98%, at least about 99%, or about 100% sequence identity to nucleotides 501 to 924 of SEQ ID NO: 1 *(i.e.,* SEQ ID NO: 74).

As is apparent from the above disclosure, in some aspects, a polynucleotide comprising an untranslated nucleic acid sequence described herein comprises at least about one, at least about two, at least three, at least about four, at least about five, at least about 10, at least about 15, at least about 20, at least about 25, at least about 30, at least about 40, at least about 50, at least about 60, at least about 70, at least about 80, at least about 90, or at least about 100 additional nucleotides at the 5' terminus ("5' region") of the untranslated nucleic acid sequence. In some aspects, a polynucleotide described herein comprises at least about one, at least about two, at least three, at least about four, at least about five, at least about 10, at least about 15, at least about 20, at least about 25, at least about 30, at least about 40, at least about 50, at least about 60, at least about 70, at least about 80, at least about 90, or at least about 100 additional nucleotides at the 3' terminus ("3' region") of the untranslated nucleic acid sequence. In some aspects, a polynucleotide comprising an untranslated nucleic acid sequence comprises at least about one, at least about two, at least three, at least about four, at least about five, at least about 10, at least about 15, at least about 20, at least about 25, at least about 30, at least about 40, at least about 50, at least about 60, at least about 70, at least about 80, at least about 90, or at least about 100 additional nucleotides at both the 5' region and the 3' region of the untranslated nucleic acid sequence.

In some aspects, a polynucleotide comprises one or more contiguous or non - contiguous nucleotides corresponding to positions 1 to 873 in SEQ ID NO: 1 at the 5' region of the untranslated nucleic acid sequence.

### II.B. Transgene

In some aspects, a polynucleotide comprising an untranslated nucleic acid sequence described herein further comprises a transgene. In some aspects, the transgene is a nucleic acid encoding a polypeptide comprising an amino acid sequence with at least 70% sequence identity with the amino acid sequence represented by SEQ ID NO 75. In some aspects, the nucleic acid is a nucleic acid with sequence identity of at least 70%, at least 80%, at least 85%, at least 90%, at least 95%, at least 96%, at least 97%, at least 98%, at least 99% or 100% with SEQ ID NO 184.

### II.C. Control Element

In some aspects, a polynucleotide described herein further comprises a control element. Accordingly, in some aspects, a polynucleotide comprises (1) a control element, (2) an untranslated nucleic acid sequence described herein, and (3) a transgene (e.g., a nucleic acid sequence with at least 70% sequence identity to SEQ ID NO: 184).

As used herein, the term "control element" refers to a nucleic acid sequence that regulate (e.g., increase or decrease) the expression of an operably linked nucleic acid. Control elements useful for the present disclosure comprises an enhancer (e.g., a CMV enhancer), a promoter (e.g., a CMV promoter, an EF-1α promoter, or a β-actin promoter), an exon (e.g., exon 1 or exon 2), splicing donor sequence, receptor sequences, or combinations thereof. In some aspects, the control element can include a sequence for transcription termination *(e.g.,* poly A), a sequence for stable transgene expression *(e.g.,* a WPRE sequence), a sequence for the reduction of transgene-specific immunity (e.g., a miRNA target sequence), or combinations thereof.

### II.C.1. Enhancer

In some aspects, the control element is an enhancer. Accordingly, in some aspects, a polynucleotide described herein comprises (in no particular order) (1) an enhancer, (2) an untranslated nucleic acid sequence, and (3) a transgene (e.g., a nucleic acid sequence with at least 70% sequence identity to SEQ ID NO: 184). In some aspects, a polynucleotide described herein comprises (from 5' to 3'): (1) an enhancer, (2) an untranslated nucleic acid sequence, and (3) a transgene.

Any suitable enhancers known in the art can be used with the present disclosure. Non-limiting examples of suitable enhancers include: a cytomegalovirus (CMV) enhancer, a SV40 early enhancer, an adenovirus 5 E1A enhancer, a HBV enhancer-1 regulatory region (Eh-1), a HPV-16 or -18 E6/7 long control region (LCR), a HIV-1 long terminal repeat (LTR), or any combination thereof. In some aspects, the enhancer is a cytomegalovirus (CMV) enhancer. In some aspects, the CMV enhancer comprises a sequence having at least about 70%, at least about 80%, at least about 85%, at least about 90%, at least about 95%, at least about 96%, at least about 97%, at least about 98%, at least about 99%, or about 100% sequence identity to SEQ ID NO: 4. In some aspects, the cytomegalovirus (CMV) enhancer includes the nucleotide sequence set forth in SEQ ID NO: 4.

### II.C.2. Promoter

In some aspects, the control element is a promoter. Accordingly, in some aspects, a polynucleotide described herein comprises (in no particular order) (1) a promoter, (2) an untranslated nucleic acid sequence, and (3) a transgene (e.g., a nucleic acid sequence with at least 70% sequence identity to SEQ ID NO: 184). In some aspects, a polynucleotide comprises (from 5' to 3'): (1) a promoter, (2) an untranslated nucleic acid sequence, and (3) a transgene. In some aspects, the control element comprises both an enhancer and a promoter. In such aspects, a polynucleotide can comprise (in no particular order) (1) an enhancer, (2) a promoter, (3) an untranslated nucleic acid sequence, and (4) a transgene. In some aspects, a polynucleotide comprises (from 5' to 3'): (1) an enhancer, (2) a promoter, (3) an untranslated nucleic acid sequence, and (4) a transgene. Any suitable promoters known in the art can be used with the present disclosure.

In some aspects, the promoter comprises a cytomegalovirus (CMV) promoter, an EF-1α promoter, a β-actin promoter, a glyceraldehyde 3-phosphate dehydrogenase (GAPDH) promoter, a 70-kDa heat shock protein (HSP70) promoter, a 78-kDa glucose-regulated protein (GRP78) promoter, an eukaryotic initiation factor-4A (elF4a) promoter, an alpha-1-antitrypsin (AAT) promoter, a transthyretin (TTR) promoter, a glial fibrillary acidic protein (GFAP) promoter, an early promoter of simian vacuolating virus 40 (SV40) promoter, a synapsin I (SYN1) promoter, a G protein-coupled receptor kinases (GRK) promoter, a rodopsin (Rho) promoter, or combinations thereof.

In some aspects, a promoter useful for the present disclosure is a CMV promoter. Accordingly, in some aspects, a polynucleotide comprises (1) an enhancer (e.g., CMV enhancer), (2) a CMV promoter, (3) an untranslated nucleic acid sequence, and (4) a transgene. In some aspects, the CMV promoter comprises a sequence having at least about 70%, at least about 80%, at least about 85%, at least about 90%, at least about 95%, at least about 96%, at least about 97%, at least about 98%, at least about 99%, or about 100% sequence identity to SEQ ID NO: 5 or 6. In some aspects, the CMV promoter includes the nucleotide sequence set forth in SEQ ID NO: 5 or 6.

In some aspects, a promoter that can be used with the present disclosure is an EF-1α promoter. In some aspects, a polynucleotide comprises (1) an enhancer (e.g., CMV enhancer), (2) an EF-1α promoter, (3) an untranslated nucleic acid sequence, and (4) a transgene. In some aspects, the EF-1α promoter comprises a sequence having at least about 70%, at least about 80%, at least about 85%, at least about 90%, at least about 95%, at least about 96%, at least about 97%, at least about 98%, at least about 99%, or about 100% sequence identity to SEQ ID NO: 7. In some aspects, the EF-1α promoter includes the nucleotide sequence set forth in SEQ ID NO: 7.

In some aspects, the promoter is a β-actin promoter. Therefore, in some aspects, a polynucleotide described herein comprises (1) an enhancer (e.g., CMV enhancer), (2) a β-actin promoter, (3) an untranslated nucleic acid sequence, and (4) a transgene. In some aspects, the β-actin promoter comprises a sequence having at least about 70%, at least about 80%, at least about 85%, at least about 90%, at least about 95%, at least about 96%, at least about 97%, at least about 98%, at least about 99%, or about 100% sequence identity to SEQ ID NO: 8. In some aspects, the β-actin promoter is a chicken β-actin promoter, such as that set forth in SEQ ID NO: 8.

As demonstrated herein (see FIG. 4A), in some aspects, a polynucleotide described herein (e.g., comprising a transgene and an untranslated nucleic acid sequence) can comprise multiple promoters. For instance, in some aspects, a polynucleotide comprises a combination of a CMV promoter, EF-1α promoter, and/or β-actin promoter. In some aspects, a polynucleotide comprises both a CMV promoter and an EF-1α promoter. In such aspects, the CMV promoter can be a portion of the full-length CMV promoter, such as the sequence set forth in SEQ ID NO: 5 *(i.e.,* first 31 nucleotides from the 5'-end of SEQ ID NO: 6).

### II.C.3. Splicing Donor Sequence

In some aspects, a polynucleotide described herein *(i.e.,* comprising an untranslated nucleic acid sequence) comprises a splicing donor sequence. As used herein, the term "splicing donor sequence" or "splicing donor site" refers to a guanine-thymine (GT) rich domain present at the 5'-end of an intron (*e.g.,* EF-1α intron) (signals the border between introns and exons). As demonstrated herein, such sequences can be targeted to produce the untranslated nucleic acid sequences of the present disclosure. In some aspects, a splicing donor sequence is linked upstream of the EF-1α intron fragment *(i.e.,* untranslated nucleic acid sequence). For instance, in some aspects, a polynucleotide described herein comprises (in no particular order) (1) an enhancer *(e.g.,* CMV enhancer), (2) a promoter *(e.g.,* CMV promoter, EF-1α promoter, and/or β-actin promoter), (3) a splicing donor sequence, (4) an untranslated nucleic acid sequence, and (5) a transgene (e.g., a nucleic acid sequence with at least 70% sequence identity to SEQ ID NO: 184). In some aspects, a polynucleotide comprises (from 5' to 3'): (1) an enhancer *(e.g.,* CMV enhancer), (2) a promoter *(e.g.,* CMV promoter, EF-1α promoter, and/or β-actin promoter), (3) a splicing donor sequence, (4) an untranslated nucleic acid sequence, and (5) a transgene.

In some aspects, a splicing donor sequence useful for the present disclosure has at least about 70%, at least about 80%, at least about 85%, at least about 90%, at least about 95%, at least about 96%, at least about 97%, at least about 98%, at least about 99%, or about 100% sequence identity to the nucleotide sequence set forth in SEQ ID NO: 9 or SEQ ID NO: 10. In some aspects, the splicing donor sequence includes the nucleotide sequence set forth in SEQ ID NO: 9 or 10.

### II.C.4. Exon Sequences

As demonstrated herein, in some aspects, a polynucleotide of the present disclosure further comprises one or more exon sequences. For instance, in some aspects, a polynucleotide described herein includes an EF-1α exon 2 (E2) sequence. Accordingly, in some aspects, a polynucleotide described herein comprises the following features (in no particular order): (1) an enhancer *(e.g.,* CMV enhancer), (2) a promoter *(e.g.,* CMV promoter, EF-1α promoter, and/or β-actin promoter), (3) a splicing donor sequence, (4) an untranslated nucleic acid sequence, (5) an EF-1α E2 sequence, and (6) a transgene (e.g., a nucleic acid sequence with at least 70% sequence identity to SEQ ID NO: 184). In some aspects, such a polynucleotide comprises (from 5' to 3'): (1) an enhancer (e.g., CMV enhancer), (2) a promoter (*e.g.,* CMV promoter, EF-1α promoter, and/or β-actin promoter), (3) a splicing donor sequence, (4) an untranslated nucleic acid sequence, (5) an EF-1α E2 sequence, and (6) a transgene. In some aspects, the EF-1α E2 sequence has at least about 70%, at least about 80%, at least about 85%, at least about 90%, at least about 95%, at least about 96%, at least about 97%, at least about 98%, at least about 99%, or about 100% sequence identity to SEQ ID NO: 11. In some aspects, the EF-1α E2 sequence includes the nucleotide sequence set forth in SEQ ID NO: 11.

In some aspects, the one or more exon sequences that can be included in a polynucleotide described herein comprises a cytomegalovirus (CMV), EF-1α or β-actin exon 1 (E1) sequence. In some aspects, a polynucleotide described herein can comprise both the E1 and E2 sequences. For instance, in some aspects, a polynucleotide comprises (in no particular order): (1) an enhancer *(e.g.,* CMV enhancer), (2) a promoter *(e.g.,* CMV promoter, EF-1α promoter, and/or β-actin promoter), (3) an E1 sequence (*e.g.,* CMV E1 sequence, EF-1α E1 sequence, and/or β-actin E1 sequence), (4) a splicing donor sequence, (5) an untranslated nucleic acid sequence, (6) an EF-1α E2 sequence, and (7) a transgene. In some aspects, a polynucleotide comprises (from 5' to 3'): (1) an enhancer (*e.g.,* CMV enhancer), (2) a promoter (*e.g.,* CMV promoter, EF-1α promoter, and/or β-actin promoter), (3) an E1 sequence (*e.g.,* CMV E1 sequence, EF-1α E1 sequence, and/or β-actin E1 sequence), (4) a splicing donor sequence, (5) an untranslated nucleic acid sequence, (6) an EF-1α E2 sequence, and (7) a transgene.

In some aspects, the E1 sequence that can be used with the present disclosure is a CMV E1 sequence. In some aspects, the CMV E1 sequence has at least about 70%, at least about 80%, at least about 85%, at least about 90%, at least about 95%, at least about 96%, at least about 97%, at least about 98%, at least about 99%, or about 100% sequence identity to SEQ ID NO: 12. In some aspects, the CMV E1 sequence includes the nucleotide sequence set forth in SEQ ID NO: 12.

In some aspects, the E1 sequence is an EF-1α E1 sequence. In some aspects, the EF-1α E1 sequence has at least about 70%, at least about 80%, at least about 85%, at least about 90%, at least about 95%, at least about 96%, at least about 97%, at least about 98%, at least about 99%, or about 100% sequence identity to SEQ ID NO: 13. In some aspects, the EF-1α E1 sequence includes the nucleotide sequence set forth in SEQ ID NO: 13.

In some aspects, the E1 sequence is a β-actin E1 sequence. In some aspects, the β-actin E1 sequence has at least about 70%, at least about 80%, at least about 85%, at least about 90%, at least about 95%, at least about 96%, at least about 97%, at least about 98%, at least about 99%, or about 100% sequence identity to SEQ ID NO: 14 or 15. In some aspects, the β-actin E1 sequence includes the nucleotide sequence set forth in SEQ ID NO: 14 or 15.

### II.C.5. miRNA Target Sequences

As described herein, in some aspects, the control element of a polynucleotide described herein comprises one or more target sequences for microRNA (miRNA) specific to immune cells ("miRNA target sequences"). Accordingly, in some aspects, a polynucleotide described herein comprises the following features (in no particular order): (1) an enhancer *(e.g.,* CMV enhancer), (2) a promoter *(e.g.,* CMV promoter, EF-1α promoter, and/or β-actin promoter), (3) an E1 sequence (e.g., CMV E1 sequence, EF-1α E1 sequence, and/or β-actin E1 sequence), (4) a splicing donor sequence, (5) an untranslated nucleic acid sequence, (6) an EF-1α E2 sequence, (7) a transgene (e.g., a nucleic acid sequence with at least 70% sequence identity to SEQ ID NO: 184), and (8) one or more miRNA target sequences. In some aspects, a polynucleotide comprises (from 5' to 3'): (1) an enhancer (e.g., CMV enhancer), (2) a promoter (e.g., CMV promoter, EF-1α promoter, and/or β-actin promoter), (3) an E1 sequence (e.g., CMV E1 sequence, EF-1α E1 sequence, and/or β-actin E1 sequence), (4) a splicing donor sequence, (5) an untranslated nucleic acid sequence, (6) an EF-1α E2 sequence, (7) a transgene, and (8) one or more miRNA target sequences.

In some aspects, a polynucleotide described herein comprises one, two, three, four, five, six, seven, eight, nine, or ten or more miRNA target sequences. In some aspects, the number of the target sequences for miRNA that can be included in a polynucleotide described herein is about two to about six (e.g., 2 to 6). In some aspects, the multiple miRNA target sequences are the same. In some aspects, one or more of the multiple miRNA target sequences are different.

As is apparent from the present disclosure, the inclusion of one or more miRNA target sequences can enhance the specificity of the polynucleotides described herein. For instance, when inhibition of the transgene is desirable in certain cell types (*e.g.,* immune cells), the target sequences for miRNA specific to immune cells can be used to inhibit the expression of transgenes in immune cells, with the result that the generation of transgene-specific immunity by immune cells can be blocked. Accordingly, by using different miRNA target sequences, the expression of the transgene in different cells/tissues can be regulated.

Any suitable miRNA target sequences known in the art can be used with the present disclosure. In some aspects, the miRNA target sequences are specific for miR142-3p or miR142-5p. In some aspects, the target sequences for miRNA are selected from antisense oligonucleotides, antagomirs, small hairpin RNA (shRNA) molecules, small interfering RNA (siRNA) molecules, ribozymes, peptide nucleic acids (PNA) oligonucleotides, locked nucleic acid (LNA) oligonucleotides, or combinations thereof, that have sequences complementary to the full-length or partial sequence of miR142-3p or miR142-5p.

In some aspects, the miRNA target sequence has at least about 70%, at least about 80%, at least about 85%, at least about 90%, at least about 95%, at least about 96%, at least about 97%, at least about 98%, at least about 99%, or about 100% sequence identity to SEQ ID NO: 16. In some aspects, the miRNA target sequence has at least about 70%, at least about 80%, at least about 85%, at least about 90%, at least about 95%, at least about 96%, at least about 97%, at least about 98%, at least about 99%, or about 100% sequence identity to SEQ ID NO: 17. In some aspects, the miRNA target sequence includes the nucleotide sequence set forth in SEQ ID NO: 16 or SEQ ID NO: 17.

### II.C.6. WPRE Sequences

In some aspects, a polynucleotide described herein *(i.e.,* comprising an untranslated nucleic acid sequence) further comprises a woodchuck hepatitis virus posttranscriptional regulatory element (WPRE) sequence. Accordingly, in some aspects, a polynucleotide described herein comprises (in no particular order) (1) an enhancer (*e.g.,* CMV enhancer), (2) a promoter (*e.g.,* CMV promoter, EF-1α promoter, and/or β-actin promoter), (3) an E1 sequence (*e.g.,* CMV E1 sequence, EF-1α E1 sequence, and/or β-actin E1 sequence), (4) a splicing donor sequence, (5) an untranslated nucleic acid sequence, (6) an EF-1α E2 sequence, (7) a transgene, (8) one or more miRNA target sequences, and (9) a WPRE sequence. In some aspects, a polynucleotide described herein comprises (from 5' to 3'): (1) an enhancer *(e.g.,* CMV enhancer), (2) a promoter *(e.g.,* CMV promoter, EF-1α promoter, and/or β-actin promoter), (3) an E1 sequence (*e.g.,* CMV E1 sequence, EF-1α E1 sequence, and/or β-actin E1 sequence), (4) a splicing donor sequence, (5) an untranslated nucleic acid sequence, (6) an EF-1α E2 sequence, (7) a transgene (e.g., a nucleic acid sequence with at least 70% sequence identity to SEQ ID NO: 184), (8) one or more miRNA target sequences, and (9) a WPRE sequence.

In some aspects, the WPRE sequence has at least about 70%, at least about 80%, at least about 85%, at least about 90%, at least about 95%, at least about 96%, at least about 97%, at least about 98%, at least about 99%, or about 100% sequence identity to SEQ ID NO: 18. In some aspects, the WPRE sequence includes the nucleotide sequence set forth in SEQ ID NO: 18.

### II.C.7. Polyadenylation Sequences

In some aspects, a polynucleotide described herein *(i.e.,* comprising an untranslated nucleic acid sequence) further includes one or more polyadenylation (pA) sequences. Accordingly, in some aspects, a polynucleotide comprises (in no particular order): (1) an enhancer *(e.g.,* CMV enhancer), (2) a promoter *(e.g.,* CMV promoter, EF-1α promoter, and/or β-actin promoter), (3) an E1 sequence (*e.g.,* CMV E1 sequence, EF-1α E1 sequence, and/or β-actin E1 sequence), (4) a splicing donor sequence, (5) an untranslated nucleic acid sequence, (6) an EF-1α E2 sequence, (7) a transgene (e.g., a nucleic acid sequence with at least 70% sequence identity to SEQ ID NO: 184), (8) one or more miRNA target sequences, (9) a WPRE sequence, and (10) one or more pA sequences. In some aspects, a polynucleotide comprises (from 5' to 3'): (1) an enhancer (*e.g.,* CMV enhancer), (2) a promoter *(e.g.,* CMV promoter, EF-1α promoter, and/or β-actin promoter), (3) an E1 sequence (*e.g.,* CMV E1 sequence, EF-1α E1 sequence, and/or β-actin E1 sequence), (4) a splicing donor sequence, (5) an untranslated nucleic acid sequence, (6) an EF-1α E2 sequence, (7) a transgene, (8) one or more miRNA target sequences, (9) a WPRE sequence, and (10) one or more pA sequences.

Any suitable pA sequences known in the art can be used with the present disclosure. In some aspects, examples of polyadenylation sequences include human growth hormone (hGH) pA sequences, bovine growth hormone (bGH) pA sequences, simian vacuolating virus 40 (SV40) early pA sequences, and SV40 late pA sequences, but are not limited thereto.

In some aspects, the pA sequence has at least about 70%, at least about 80%, at least about 85%, at least about 90%, at least about 95%, at least about 96%, at least about 97%, at least about 98%, at least about 99%, or about 100% sequence identity to SEQ ID NO: 19. In some aspects, the pA sequence has at least about 70%, at least about 80%, at least about 85%, at least about 90%, at least about 95%, at least about 96%, at least about 97%, at least about 98%, at least about 99%, or about 100% sequence identity to SEQ ID NO: 20. In some aspects, the pA sequence has at least about 70%, at least about 80%, at least about 85%, at least about 90%, at least about 95%, at least about 96%, at least about 97%, at least about 98%, at least about 99%, or about 100% sequence identity to SEQ ID NO: 21. In some aspects, the pA sequence has at least about 70%, at least about 80%, at least about 85%, at least about 90%, at least about 95%, at least about 96%, at least about 97%, at least about 98%, at least about 99%, or about 100% sequence identity to SEQ ID NO: 22. In some aspects, the polyadenylation sequences are selected from the group consisting of the nucleotide sequences set forth in SEQ ID NOS: 19 to 22.

As described above, in some aspects, a polynucleotide described herein comprises (i) a transgene (e.g., a nucleic acid sequence with at least 70% sequence identity to SEQ ID NO: 184) and (ii) a control element operably linked to the transgene, wherein the control element comprises (from 5' to 3'): (1) the CMV enhancer sequence set forth in SEQ ID NO: 4, (2) the CMV promoter sequence set forth in SEQ ID NO: 5 or 6, the EF-1α promoter sequence set forth in SEQ ID NO: 7, or the chicken β-actin promoter sequence set forth in SEQ ID NO: 8, (3) the CMV E1 sequence set forth in SEQ ID NO: 12, the EF-1α E1 sequence set forth in SEQ ID NO: 13, or the chicken β-actin E1 sequence set forth in SEQ ID NO: 14 or 15, (4) the splicing donor sequence set forth in SEQ ID NO: 9 or 10, (5) the EF-1α intron fragment sequence *(i.e.,* untranslated nucleic acid sequence) comprising, consisting essentially of, or consisting of the nucleotide sequence set forth in SEQ ID NO: 2, SEQ ID NO: 3, or SEQ ID NO: 57, and (6) the EF-1α E2 sequence set forth in SEQ ID NO: 11.

### III. TAFA

The present disclosure provides a polypeptide comprising an amino acid sequence with at least 70% sequence identity with the amino acid sequence of TAFA protein (e.g., TAFA1 to TAFA4), a nucleic acid encoding the polypeptide, a vector comprising the nucleic acid, a recombinant virus particle comprising the vector and capsid protein, or a cell comprising the nucleic acid or vector.

### III.A. Polypeptides

The present disclosure provides a polypeptide comprising an amino acid sequence that is at least 70% sequence identical to an amino acid sequence represented by SEQ ID NO 75. In some aspects, an amino acid sequence with at least 70% sequence identity with the amino acid sequence represented by SEQ ID NO 75 comprises an amino acid sequence of General Formula 1 (from N-terminal to C-terminal):

<General Formula 1> X1-X2-X3-G-T-C-E-V-X4-A-X5-H-X6-C-C-N-X7-N-X8-I-E-E-X9-S-Q-T-X10-X11-C-S-C-X12-X13-G-X14-V-A-G-T-T-X15-X16-X17-P-S-C-V-X18-A-X19-I-V-X20-X21-X22-W-W-C-X23-M-X24-P-C-X25-X26-G-E-X27-C-K-X28-L-P-D-X29-X30-G-W-X31-C-X32-X33-G-X34-K-X35-K-T-T-X36-X37-X38-X39

In General Formula 1,
X1 is nonexistent, V, I or L,
X2 is K, E, R or Q,
X3 is G, T, Q, P or A,
X4 is V or I,
X5 is A, L, V or I,
X6 is R or L,
X7 is K, R or Q,
X8 is R or K,
X9 is R or L,
X10 is V or G,
X11 is K or N,
X12 is F or L,
X13 is P or S,
X14 is Q or K,
X15 is R, H or Q,
X16 is A, N, S or T,
X17 is A, Q, R, K or T,
X18 is D or E,
X19 is S or A,
X20 is I, E, L, A or V,
X21 is Q, G or E,
X22 is K or R,
X23 is H, Q or E,
X24 is E, Q, N, D, S or H,
X25 is L, V or M,
X26 is E, D, P, L or A,
X27 is E or D,
X28 is V, T, A or I,
X29 is L, N, R, Y, S or Q,
X30 is S, K or T,
X31 is S or M,
X32 is S, A or Y,
X33 is S, T or R,
X34 is N or H,
X35 is V or I,
X36 is R or K,
X37 is nonexistent, V, A, G, M or N,
X38 is nonexistent, T, I, N, F or S, and
X39 is nonexistent, R, H, V, K, I or Q.

In some aspects, the amino acid sequence with at least 70% sequence identity with the amino acid sequence represented by SEQ ID NO 75 consists of 80 to 130, specifically 90 to 120, most specifically 91 to 119, amino acid sequences.

In some aspects, the polypeptide has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94% or at least 95%, sequence identity with the amino acid sequence represented by SEQ ID NO 75.

In some aspects, the polypeptide further comprises an amino acid sequence of General Formula 2 (from N-terminal to C-terminal):

<General Formula 2> X1-X2-H-H-K-A-X3-H

In General Formula 2,
X1 is A or V,
X2 is N or I, and
X3 is H or Q.

In some aspects, the polypeptide comprises an amino acid sequence of General Formula 2-General Formula 1 (from N-terminal to C-terminal).

In some aspects, the polypeptide further comprises an amino acid sequence of General Formula 3 (from N-terminal to C-terminal):

<General Formula 3> X1-X2-X3-X4-X5-X6-X7-X8-X9-X10

In General Formula 3,
X1 is A or S,
X2 is L, T or S,
X3 is Q, E or H,
X4 is nonexistent, P, L or H,
X5 is P or R,
X6 is T, S or I,
X7 is A, P, T, S or H,
X8 is T, A, S or I,
X9 is V or A, and
X10 is L or H.

In some aspects, the polypeptide comprises an amino acid sequence of General Formula 3- General Formula 1 (from N-terminal to C-terminal).

In some aspects, the polypeptide further comprises an amino acid sequence of General Formula 4 (from N-terminal to C-terminal):

<General Formula 4> X1-X2

In General Formula 4,
X1 is H or Y, and
X2 is Q, V or L.

In some aspects, the polypeptide includes an amino acid sequence of General Formula 4-General Formula 1 (from N-terminal to C-terminal).

In some aspects, the polypeptide further comprises the following amino acid sequences (from N-terminal to C-terminal):
LHRP (SEQ ID NO 293);
LHQSGFTSGHFPHHRKLGE (SEQ ID NO 294); or
LAPPGTNIQI (SEQ ID NO 295).

In some aspects, the polypeptide further comprises an amino acid sequence of General Formula 5 (from N-terminal to C-terminal):

<General Formula 5> X1-X2-X3

In General Formula 5,
X1 is P or G,
X2 is R or H, and
X3 is T, S or L.

In some aspects, the polypeptide further comprises the following amino acid sequences (from N-terminal to C-terminal):
PYTSL (SEQ ID NO 296); or
QEDKLK (SEQ ID NO 297).

In some aspects, the polypeptide comprises an amino acid sequence of LHRP-General Formula 1, LHQSGFTSGHFPHHRKLGE-General Formula 1 or LAPPGTNIQI-General Formula 1 (from N-terminal to C-terminal). In some aspects, the polypeptide comprises an amino acid sequence of General Formula 1-General Formula 5, General Formula 1-PYTSL or General Formula 1-QEDKLK (from N-terminal to C-terminal). In some aspects, the polypeptide includes an amino acid sequence of LHRP-General Formula 1-General Formula 5, LHQSGFTSGHFPHHRKLGE-General Formula 1-General Formula 5, LAPPGTNIQI-General Formula 1-QEDKLK, or LAPPGTNIQI-General Formula 1- PYTSL (from N-terminal to C-terminal).

In some aspects, the polypeptide comprises one or more amino acid sequence selected from a group consisting of amino acid sequences represented by SEQ ID NOS 75 to 129. In some aspects, the polypeptide comprises one or more amino acid sequence selected from a group consisting of amino acid sequences represented by SEQ ID NOS 130 to 183. In some aspects, the polypeptide further comprises a signal sequence. In some aspects, the polypeptide comprises one or more amino acid sequence selected from a group consisting of amino acid sequences represented by SEQ ID NOS 298 to 304.

### III.B. Nucleic acids encoding polypeptides

The present disclosure provides a nucleic acid encoding a polypeptide comprising an amino acid sequence with at least 70% sequence identity with the amino acid sequence represented by SEQ ID NO 75.

The nucleic acid useful in the present disclosure is not particularly limited as long as the nucleic acid can be translated into a polypeptide upon transduction into a cell. In some aspects, the nucleic acid encodes the polypeptide (or any variant thereof) or a fusion protein.

In some aspects, the nucleic acid encodes a protein useful for the prevention or treatment of the disease or disorder such as those described herein. In some aspects, the nucleic acid encodes a peptide for the prevention or treatment of a particular disease, which is intended for continuous expression within the body of a subject or patient. In some aspects, the nucleic acid has sequence identity of at least 70%, at least 80%, at least 85%, at least 90%, at least 95%, at least 96%, at least 97%, at least 98%, at least 99% or 100% with SEQ ID NO 184.

In some aspects, the nucleic acid encodes a polypeptide comprising the amino acid sequence of General Formula 1 (from N-terminal to C-terminal):

<General Formula 1> X1-X2-X3-G-T-C-E-V-X4-A-X5-H-X6-C-C-N-X7-N-X8-I-E-E-X9-S-Q-T-X10-X11-C-S-C-X12-X13-G-X14-V-A-G-T-T-X15-X16-X17-P-S-C-V-X18-A-X19-I-V-X20-X21-X22-W-W-C-X23-M-X24-P-C-X25-X26-G-E-X27-C-K-X28-L-P-D-X29-X30-G-W-X31-C-X32-X33-G-X34-K-X35-K-T-T-X36-X37-X38-X39

In General Formula 1,
X1 is nonexistent, V, I or L,
X2 is K, E, R or Q,
X3 is G, T, Q, P or A,
X4 is V or I,
X5 is A, L, V or I,
X6 is R or L,
X7 is K, R or Q,
X8 is R or K,
X9 is R or L,
X10 is V or G,
X11 is K or N,
X12 is F or L,
X13 is P or S,
X14 is Q or K,
X15 is R, H or Q,
X16 is A, N, S or T,
X17 is A, Q, R, K or T,
X18 is D or E,
X19 is S or A,
X20 is I, E, L, A or V,
X21 is Q, G or E,
X22 is K or R,
X23 is H, Q or E,
X24 is E, Q, N, D, S or H,
X25 is L, V or M,
X26 is E, D, P, L or A,
X27 is E or D,
X28 is V, T, A or I,
X29 is L, N, R, Y, S or Q,
X30 is S, K or T,
X31 is S or M,
X32 is S, A or Y,
X33 is S, T or R,
X34 is N or H,
X35 is V or I,
X36 is R or K,
X37 is nonexistent, V, A, G, M or N,
X38 is nonexistent, T, I, N, F or S, and
X39 is nonexistent, R, H, V, K, I or Q.

In some aspects, the nucleic acid encodes a polypeptide comprising the amino acid sequence of General Formula 2 and the amino acid sequence of General Formula 1 (from N-terminal to C-terminal):

<General Formula 2> X1-X2-H-H-K-A-X3-H

In General Formula 2,
X1 is A or V,
X2 is N or I, and
X3 is H or Q.

In some aspects, the nucleic acid encodes a polypeptide comprising the amino acid sequence of General Formula 2- General Formula 1 (from N-terminal to C-terminal).

In some aspects, the nucleic acid encodes a polypeptide comprising the amino acid sequence of General Formula 3 and the amino acid sequence of General Formula 1 (from N-terminal to C-terminal):

<General Formula 3> X1-X2-X3-X4-X5-X6-X7-X8-X9-X10

In General Formula 3,
X1 is A or S,
X2 is L, T or S,
X3 is Q, E or H,
X4 is nonexistent, P, L or H,
X5 is P or R,
X6 is T, S or I,
X7 is A, P, T, S or H,
X8 is T, A, S or I,
X9 is V or A, and
X10 is L or H.

In some aspects, the nucleic acid encodes a polypeptide comprising the amino acid sequence of General Formula 3-General Formula 1 (from N-terminal to C-terminal).

In some aspects, the nucleic acid encodes a polypeptide comprising the amino acid sequence of General Formula 4 and the amino acid sequence of General Formula 1 of the following (from the N-terminal to the C-terminal):

<General Formula 4> X1-X2

In General Formula 4,
X1 is H or Y, and
X2 is Q, V or L.

In some aspects, the nucleic acid encodes a polypeptide comprising the amino acid sequence of General Formula 4-General Formula 1 (from N-terminal to C-terminal).

In some aspects, the nucleic acid encodes a polypeptide comprising the following amino acid sequence in addition to the amino acid sequence of General Formula 1 (from N-terminal to C-terminal):
LHRP (SEQ ID NO 293);
LHQSGFTSGHFPHHRKLGE (SEQ ID NO 294); or
LAPPGTNIQI (SEQ ID NO 295).

In some aspects, the nucleic acid encodes a polypeptide comprising an amino acid sequence of General Formula 5 in addition to the amino acid sequence of General Formula 1 (from N-terminal to C-terminal):

<General Formula 5> X1-X2-X3

In General Formula 5,
X1 is P or G,
X2 is R or H, and
X3 is T, S or L.

In some aspects, the polypeptide encodes a polypeptide comprising the following amino acid sequence (from N-terminal to C-terminal):
PYTSL (SEQ ID NO 296); or
QEDKLK (SEQ ID NO 297).

In some aspects, the nucleic acid encodes a polypeptide comprising the amino acid sequence of LHRP-General Formula 1, LHQSGFTSGHFPHHRKLGE-General Formula 1, or LAPPGTNIQl-General Formula 1 (from N-terminal to C-terminal). In some aspects, the nucleic acid encodes a polypeptide comprising the amino acid sequence of General Formula 1-General Formula 5, General Formula 1-PYTSL, or General Formula 1-QEDKLK (from N-terminal to C-terminal). In some aspects, the nucleic acid encodes a polypeptide comprising the amino acid sequence of LHRP-General Formula 1-General Formula 5, LHQSGFTSGHFPHHRKLGE-General Formula 1-General Formula 5, LAPPGTNIQI-General Formula 1-QEDKLK, or LAPPGTNIQI-General Formula 1- PYTSL (from N-terminal to C-terminal).

In some aspects, the nucleic acid encoding the polypeptide is one or more nucleotide sequence selected from a group consisting of nucleotide sequences represented by SEQ ID NOS 184 to 238. In some aspects, the nucleic acid encoding the polypeptide is one or more nucleotide sequence selected from a group consisting of nucleotide sequences represented by SEQ ID NOS 239 to 292. In some aspects, the nucleic acid encoding the polypeptide further comprises a sequence encoding a signal peptide. In some aspects, the nucleic acid encoding the polypeptide is one or more nucleotide sequence selected from a group consisting of the nucleotide sequences represented by SEQ ID NOS 305 to 311.

### IV. Vectors comprising nucleic acids encoding polypeptides

The present disclosure provides a vector comprising a nucleic acid encoding a polypeptide comprising an amino acid sequence with at least 70% sequence identity with the amino acid sequence represented by SEQ ID NO 75.

The nucleic acid is the same as described in III.B.

As described herein, such vectors are useful for recombinant expression in host cells and cells targeted for therapeutic intervention. In some aspects, a vector useful for the delivery of a polynucleotide described herein (*e*.*g*., comprising a transgene (e.g., a nucleic acid encoding a polypeptide including an amino acid sequence with at least 70% sequence identity to SEQ ID NO: 75 or a nucleic acid sequence with at least 70% sequence identity to SEQ ID NO: 184) and an untranslated nucleic acid sequence) comprises a viral vector. Examples of viruses that can be used as vectors in the present disclosure include, but are not limited to, retroviruses, herpes simplex viruses, lentiviruses, poxviruses, vaccinia viruses, rhabdoviruses, adenoviruses, helper-dependent adenoviruses, adeno-associated viruses (AAVs), baculovirus, and combinations thereof. In some aspects, a vector that can be used with the present disclosure comprises a non-viral vector. Non-limiting examples of such vectors include a plasmid, a cosmid, a yeast artificial chromosome (YAC), a bacteriophage, and combinations thereof.

In some aspects, the vector comprising the nucleic acid encoding a polypeptide comprising an amino acid sequence with at least 70% sequence identity with the amino acid sequence represented by SEQ ID NO 75 is an adeno-associated virus (AAV) vector.

In some aspects, the vector further comprises one or more sequence selected from a group consisting of a promoter sequence, an enhancer sequence, an exon sequence, an intron sequence, a signal sequence-coding sequence, a splicing donor sequence, and one or more adeno-associated viral inverted terminal repeat (ITR) sequence.

In some aspects, the intron does not include the nucleotide sequence represented by SEQ ID NO 1.

In some aspects, the intron consists of 29 to 174 nucleotides. In some aspects, the intron consists of 51 to 117 nucleotides.

In some aspects, the intron comprises a nucleotide sequence represented by SEQ ID NO 57. In some aspects, the intron comprises a nucleotide sequence represented by SEQ ID NO 58. In some aspects, the intron comprises a nucleotide sequence represented by SEQ ID NO 59. In some aspects, the intron comprises a nucleotide sequence represented by SEQ ID NO 3.

In some aspects, the intron has at least 70% sequence identity with (i) the 871st to 924th nucleotides of SEQ ID NO 1 (SEQ ID NO 58), (ii) the 861st to 924th nucleotides of SEQ ID NO 1 (SEQ ID NO 59), (iii) the 852nd to 924th nucleotides of SEQ ID NO 1 (SEQ ID NO 3), (iv) the 851st to 924th nucleotides of SEQ ID NO 1 (SEQ ID NO 61), (v) the 830th to 924th nucleotides of SEQ ID NO 1 (SEQ ID NO 2), (vi) the 821st to 924th nucleotides of SEQ ID NO 1 (SEQ ID NO 63), (vii) the 811th to 924th nucleotides of SEQ ID NO 1 (SEQ ID NO 64), or (viii) the 808th to 924th nucleotides of SEQ ID NO 1 (SEQ ID NO 65).

In some aspects, the intron comprises (i) the 871st to 924th nucleotides of SEQ ID NO 1 (SEQ ID NO 58), (ii) the 861st to 924th nucleotides of SEQ ID NO 1 (SEQ ID NO 59), (iii) the 852nd to 924th nucleotides of SEQ ID NO 1 (SEQ ID NO 3), (iv) the 851st to 924th nucleotides of SEQ ID NO 1 (SEQ ID NO 61), (v) the 830th to 924th nucleotides of SEQ ID NO 1 (SEQ ID NO 2), (vi) the 821st to 924th nucleotides of SEQ ID NO 1 (SEQ ID NO 63), (vii) the 811th to 924th nucleotides of SEQ ID NO 1 (SEQ ID NO 64), or (viii) the 808th to 924th nucleotides of SEQ ID NO 1 (SEQ ID NO 65).

In some aspects, the vector comprises the followings:
(1) a CMV enhancer sequence represented by SEQ ID NO 4;
(2) a promoter sequence selected from a CMV promoter sequence represented by SEQ ID NO 5 or 6, an EF-1α promoter sequence represented by SEQ ID NO 7, or a chicken β actin promoter represented by SEQ ID NO SEQ ID NO 8;
(3) an exon 1 (E1) sequence selected from a CMV E1 sequence represented by SEQ ID NO 12, an EF-1α E1 sequence represented by SEQ ID NO 13, or a chicken β actin E1 sequence represented by SEQ ID NO 14 or 15;
(4) a splicing donor sequence represented by SEQ ID NO 9 or 10; and/or
(5) an EF-1α E2 sequence represented by SEQ ID NO 11.

In some aspects, the AAV vector is for use in gene therapy. In some aspects, the AAV vector is for use in the expression of a polypeptide comprising an amino acid sequence with at least 70% sequence identity with the amino acid sequence represented by SEQ ID NO 75.

### V. AAV

In some aspects, polynucleotides described herein (e.g., comprising a transgene and an untranslated nucleic acid sequence) are delivered, e.g., to a cell, using an AAV. Adeno-associated viruses (AAVs) as single-stranded DNA viruses are helper-dependent human parvoviruses. The AAV genome has a size of about 4.7 kbp and consists of an N-terminal encoding the rep gene involved in viral replication and the expression of viral genes, a C-terminal coding for the cap gene encoding the capsid protein of the virus, and an inverted terminal repeat (ITR) with about 145 base insertions at each terminus. The 145 bp inverted terminal repeat (ITR) has a T-shaped structure, serves as an origin of replication during viral genome replication, and functions as a primary packaging signal. The ITR is only a cis-acting nucleotide sequence required to prepare a recombinant AAV (rAAV) construct. The ITR has an enhancer activity in the presence of the Rep protein but its activity is very weak in the absence of the Rep protein. In view of these features, when a transgene is cloned into a recombinant AAV construct, an enhancer, a promoter, pA, etc. are properly assembled to prepare an expression construct (RJ Samulski and N Muzyczka, Annu. Rev. Virolo. 2014. 1: 427-451). Four proteins are translated from the rep gene. These proteins are classified into rep78, rep68, rep52, and rep40 by their molecular weight and perform important functions in AAV DNA replication. Four proteins are translated from the cap gene. Of these, VP1, VP2, and VP3 proteins are structural proteins constituting AAV particles and the assembly-activating protein (AAP) promotes the assembly of AAV particles by the structural proteins. Some proteins and RNAs derived from helper viruses such as adenoviruses or herpes simplex viruses are required for efficient replication of adeno-associated viruses (Muzyczka N. Curr Top Microbiol Immunol 158, 97-129, 1992).

AAV possesses unique features that make it attractive as a vector system for delivering foreign DNA into cells. AAV infection of cells in culture has generally been noncytopathic, and natural infection of humans and other animals is silent and asymptomatic. Moreover, AAV infects many different types of mammalian cells allowing the possibility of targeting many different tissues *in vivo.* AAV also possesses additional advantages that make it a particularly attractive viral system for gene delivery, including the promotion of an immune response that is relatively mild compared to other forms of gene delivery, and persistent expression in both dividing and quiescent cells based on non-integrating, episomal vector DNA. Also, AAV withstands the conditions used to inactivate adenovirus (56 to 65 °C for several hours), making cold preservation of rAAV-based vaccines less critical.

The types or serotypes of adeno-associated viruses that can be used with the present disclosure includes AAVrh.10 (AAVrh10), AAV-DJ (AAVDJ), AAV-DJ8 (AAVDJ8), AAV1, AAV2, AAV2G9, AAV3, AAV3a, AAV3b , AAV3-3, AAV4, AAV4-4, AAV5, AAV6, AAV6.1, AAV6.2, AAV6.1.2, AAV7, AAV7.2, AAV8, AAV9, AAV9.11, AAV9.13, AAV9.16, AAV9 .24, AAV9.45, AAV9.47, AAV9.61, AAV9.68, AAV9.84, AAV9.9, AAV10, AAV11, AAV12, AAV16.3, AAV24.1, AAV27.3, AAV42.12, AAV42-1b, AAV42-2, AAV42-3a, AAV42-3b, AAV42-4, AAV42-5a, AAV42-5b, AAV42-6b, AAV42-8, AAV42-10, AAV42-11, AAV42-12, AAV42-13, AAV42-15, AAV42-aa, AAV43-1, AAV43-12, AAV43-20, AAV43-21, AAV43-23, AAV43-25, AAV43-5, AAV44.1, AAV44.2, AAV44.5, AAV223.1, AAV223.2, AAV223.4, AAV223.5, AAV223.6, AAV223.7, AAV1-7/rh.48, AAV1-8/rh.49, AAV2-15/rh.62, AAV2-3/rh.61, AAV2-4/rh.50, AAV2-5/rh.51, AAV3.1/hu.6, AAV3.1/hu.9, AAV3-9/rh.52, AAV3-11/rh.53, AAV4-8/r11.64, AAV4-9/rh.54, AAV4-19/rh.55, AAV5-3/rh.57, AAV5-22/rh.58, AAV7.3/hu.7, AAV16.8/hu.10, AAV16.12/hu.11, AAV29.3/bb.1, AAV29.5/bb.2, AAV106.1/hu.37, AAV114.3/hu.40, AAV127.2/hu.41, AAV127.5/hu.42, AAV128.3/hu.44, AAV130.4/hu.48, AAV145.1/hu.53, AAV145.5/hu.54, AAV145.6/hu.55, AAV161.10/hu.60, AAV161.6/hu.61, AAV33.12/hu.17, AAV33.4/hu.15, AAV33.8/hu.16, AAV52/hu.19, AAV52.1/hu.20, AAV58.2/hu.25, AAVA3.3, AAVA3.4, AAVA3.5, AAVA3.7, AAVC1, AAVC2, AAVC5, AAVF3, AAVF5, AAVH2, AAVrh.72, AAVhu.8, AAVrh.68, AAVrh.70, AAVpl.1, AAVpl.3, AAVpl.2, AAVrh.60, AAVrh.44, AAVrh.65, AAVrh.55, AAVrh.47, AAVrh.69, AAVrh.45, AAVrh.59, AAVhu.12, AAVH6, AAVLK03, AAVH-1/hu.1, AAVH-5/hu.3, AAVLG-10/rh.40, AAVLG-4/rh.38, AAVLG-9/hu.39, AAVN721-8/rh.43, AAVCh.5, AAVCh.5R1, AAVcy.2, AAVcy.3, AAVcy.4, AAVcy.5, AAVCy.5R1, AAVCy.5R2, AAVCy.5R3, AAVCy.5R4, AAVcy.6, AAVhu.1, AAVhu.2, AAVhu.3, AAVhu.4, AAVhu.5, AAVhu.6, AAVhu.7, AAVhu.9, AAVhu.10, AAVhu.11, AAVhu.13, AAVhu.15, AAVhu.16, AAVhu.17, AAVhu.18, AAVhu.20, AAVhu.21, AAVhu.22, AAVhu.23.2, AAVhu.24, AAVhu.25, AAVhu.27, AAVhu.28, AAVhu.29, AAVhu.29R, AAVhu.31, AAVhu.32, AAVhu.34, AAVhu.35, AAVhu.37, AAVhu.39, AAVhu.40, AAVhu.41, AAVhu.42, AAVhu.43, AAVhu.44, AAVhu.44R1, AAVhu.44R2, AAVhu.44R3, AAVhu.45, AAVhu.46, AAVhu.47, AAVhu.48, AAVhu.48R1, AAVhu.48R2, AAVhu.48R3, AAVhu.49, AAVhu.51, AAVhu.52, AAVhu.54, AAVhu.55, AAVhu.56, AAVhu.57, AAVhu.58, AAVhu.60, AAVhu.61, AAVhu.63, AAVhu.64, AAVhu.66, AAVhu. 67, AAVhu.14/9, AAVhu.t19, AAVrh.2, AAVrh.2R, AAVrh.8, AAVrh.8R, AAVrh.12, AAVrh.13, AAVrh.13R, AAVrh.14, AAVrh.17, AAVrh.18, AAVrh.19, AAVrh.20, AAVrh.21, AAVrh.22, AAVrh.23, AAVrh.24, AAVrh.25, AAVrh.31, AAVrh.32, AAVrh.33, AAVrh.34, AAVrh.35, AAVrh.36, AAVrh.37, AAVrh.37R2, AAVrh.38, AAVrh.39, AAVrh.40, AAVrh.46, AAVrh.48, AAVrh.48.1, AA Vrh.48.1.2, AAVrh.48.2, AAVrh.49, AAVrh.51, AAVrh.52, AAVrh.53, AAVrh.54, AAVrh.56, AAVrh.57, AAVrh.58, AAVrh.61, AAVrh.64, AAVrh.64R1, AAVrh.64R2, AAVrh.67, AAVrh.73, AAVrh.74, AAVrh8R, AAVrh8R A586R variant, AAVrh8R R533A variant, AAAV, BAAV, caprine AAV, bovine AAV, AAVhE1.1, AAVhEr1.5, AAVhER1.14, AAVhEr1.8, AAVhEr1.16, AAVhEr1.18, AAVhEr1.35, AAVhEr1.7, AAVhEr1.36, AAVhEr2.29, AAVhEr2.4, AAVhEr2.16, AAVhEr2.16, AAVhEr2.30, AAVhEr2.31, AAVhEr2.36, AAVhER1.23, AAVhEr3.1, AAV2.5T, AAV-PAEC, AAV-LK01, AAV-LK02, AAV-LK03, AAV-LK04, AAV-LK05, AAV-LK06, AAV-LK07, AAV-LK08, AAV-LK09, AAV-LK10, AAV-LK11, AAV-LK12, AAV-LK13, AAV-LK14, AAV-LK15, AAV-LK16, AAV-LK17, AAV-LK18, AAV-LK19, AAV-PAEC2, AAV-PAEC4, AAV-PAEC6, AAV-PAEC7, AAV-PAEC8, AAV-PAEC11, AAV-PAEC12, AAV-2-pre-miRNA-101, AAV-8h, AAV-8b, AAV-h, AAV-b, AAV SM 10-2, AAV Shuffle 100-1, AAV Shuffle 100-3, AAV Shuffle 100-7, AAV Shuffle 10-2, AAV Shuffle 10-6, AAV Shuffle 10-8, AAV Shuffle 100-2, AAV SM 10-1, AAV SM 10-8, AAV SM 100-3, AAV SM 100-10, B P61 AAV, B P62 AAV, B P63 AAV, AAVrh.50, AAVrh.43, AAVrh.62, AAVrh.48, AAVhu.19, AAVhu.11, AAVhu.53, AAV4-8/rh.64, AAVLG-9/hu.39, AAV54.5/hu.23, AAV54.2/hu.22, AAV54.7/hu.24, AAV54.1/hu.21, AAV54.4R/hu.27, AAV46.2/hu.28, AAV46.6/hu.29, AAV128.1/hu.43, true type AAV (ttAAV), UPENN AAV 10, and Japanese AAV 10 serotypes but are not limited thereto.

In some aspects, the serotype of the adeno-associated virus is AAV1, AAV2, AAV3, AAV4, AAV5, AAV6, AAV7, AAV8, AAV9 or AAVrh10. In some aspects, the serotype of the AAV is AAV2. In some aspects, the serotype of the AAV is AAV5. In some aspects, the serotype of the AAV is AAV8. In some aspects, the serotype of the AAV is AAV9.

### VI. Cells

In some aspects, provided herein are cells comprising any of the polynucleotides described herein (e.g., comprising an untranslated nucleic acid sequence). For instance, in some aspects, cells described herein are transduced, transfected, or transformed with a recombinant expression construct comprising a transgene and an untranslated nucleic acid sequence for transgene expression.

Not to be bound by any one theory, in some aspects, the cells described herein (e.g., transduced with a polynucleotide comprising an untranslated nucleic acid sequence) are useful for producing a protein, such as that encoded by a transgene described herein (e.g., a VEGF inhibitor). As described herein, in some aspects, an untranslated nucleic acid sequence described herein *(i.e.,* EF-1α intron fragment) can enhance the expression of the protein encoded by the transgene ("encoded protein") in a cell. Accordingly, in some aspects, a cell described herein (e.g., transduced with a polynucleotide comprising a transgene and an untranslated nucleic acid sequence of the present disclosure) produces greater expression of the encoded protein compared to a reference cell. In some aspects, the reference cell is transduced with a corresponding polynucleotide but lacking the untranslated nucleic acid sequence.

In some aspects, the cells described herein can produce the protein encoded by the transgene *in vitro.* In certain aspects, the cells described herein can produce the encoded protein *in vivo* (*e.g.,* in a subject that received an administration of a polynucleotide described herein). In some aspects, the cells described herein can produce the encoded protein both *in vitro* and *in vivo.*

In some aspects, a cell that can be used to produce a protein encoded by a transgene (*e.g., in vitro*) comprises a host cell. As used herein, the term "host cell" is intended to include cells of any organism that can be transduced with the expression construct (e.g., vector) to replicate the expression construct or express the gene encoded by the expression construct. Such cells include eukaryotic cells and prokaryotic cells. As used herein, the term "transduction" is intended to include "transfection" and "transformation". The host cell can be transduced, transfected or transformed with the expression construct. This process means the delivery or introduction of the exogenous nucleic acid molecule into the host cell. In some aspects, the host cell is an isolated host cell comprising the AAV vector. In some aspects, the host cell is an isolated host cell transformed with the AAV vector.

In some aspects, the host cell is a eukaryotic cell. In some aspects, the host cell is selected from the group consisting of a mammalian cell, an insect cell, a yeast cell, a transgenic mammalian cell, and a plant cell. In some aspects, the host cell is a prokaryotic cell. In some aspects, the prokaryotic cell is a bacterial cell.

In some aspects, the host cell is an insect cell. In some aspects, the insect cell is Sf9. In some aspects, the host cell is a mammalian cell. Non-limiting examples of the mammalian cell that can be used in the present disclosure include HEK293, HeLa, ARPE-19, RPE-1, HepG2, Hep3B, Huh-7, C8D1a, Neuro2A, CHO, MES13, BHK-21, COS7, COP5, A549, MCF-7, HC70, HCC1428, BT-549, PC3, LNCaP, Capan-1, Panc-1, MIA PaCa-2, SW480, HCT166, LoVo, A172, MKN-45, MKN-74, Kato-III, NCI-N87, HT-144, SK-MEL-2, SH-SY5Y, C6, HT-22, PC-12 and NIH3T3 cells, and combinations thereof.

In some aspects, a cell that can be used to produce a protein encoded by a transgene described herein (*e.g., in vivo*) comprises a human cell. In some aspects, the human cell is a cell of a subject that is to receive an administration of a nucleic acid molecule described herein. In certain aspects, the human cell is from a donor (*e.g*., healthy human subject).

In some aspects, provided herein is a composition comprising the AAV vector or a host cell comprising the AAV vector or transformed with the AAV vector.

### VII. Compositions

The present disclosure provides a composition comprising the polypeptide, a nucleic acid encoding the polypeptide, a vector comprising the nucleic acid, a recombinant virus particle comprising the vector and capsid protein, or a cell comprising the nucleic acid or vector.

In some aspects, the composition is a pharmaceutical composition.

In some aspects, disclosed herein is a pharmaceutical composition comprising (a) any of the polynucleotides described herein (*e.g*., comprising a transgene and an untranslated nucleic acid sequence) and (b) one or more pharmaceutically acceptable carriers. In some aspects, disclosed herein is a pharmaceutical composition comprising (a) a vector (e.g., rAAV) as described herein and (b) one or more pharmaceutically acceptable carriers. In some aspects, disclosed herein is a pharmaceutical composition comprising (a) a cell as described herein and (b) one or more pharmaceutically acceptable carriers.

In some aspects, the pharmaceutical composition described herein compriss a polypeptide including an amino acid sequence having at least 70% sequence identity with the amino acid sequence represented by SEQ ID NO 75 (e.g., the TAFA1 to TAFA4 sequence in vertebrates), a nucleic acid encoding the polypeptide, a vector comprising the nucleic acid, a recombinant virus particle comprising the vector and capsid protein, or a cell comprising the nucleic acid or vector.

In some aspects, an amino acid sequence with at least 70% sequence identity with the amino acid sequence represented by SEQ ID NO 75 includes an amino acid sequence of General Formula 1 (from N-terminal to C-terminal):

<General Formula 1> X1-X2-X3-G-T-C-E-V-X4-A-X5-H-X6-C-C-N-X7-N-X8-I-E-E-X9-S-Q-T-X10-X11-C-S-C-X12-X13-G-X14-V-A-G-T-T-X15-X16-X17-P-S-C-V-X18-A-X19-I-V-X20-X21-X22-W-W-C-X23-M-X24-P-C-X25-X26-G-E-X27-C-K-X28-L-P-D-X29-X30-G-W-X31-C-X32-X33-G-X34-K-X35-K-T-T-X36-X37-X38-X39

In General Formula 1,
X1 is nonexistent, V, I or L,
X2 is K, E, R or Q,
X3 is G, T, Q, P or A,
X4 is V or I,
X5 is A, L, V or I,
X6 is R or L,
X7 is K, R or Q,
X8 is R or K,
X9 is R or L,
X10 is V or G,
X11 is K or N,
X12 is F or L,
X13 is P or S,
X14 is Q or K,
X15 is R, H or Q,
X16 is A, N, S or T,
X17 is A, Q, R, K or T,
X18 is D or E,
X19 is S or A,
X20 is I, E, L, A or V,
X21 is Q, G or E,
X22 is K or R,
X23 is H, Q or E,
X24 is E, Q, N, D, S or H,
X25 is L, V or M,
X26 is E, D, P, L or A,
X27 is E or D,
X28 is V, T, A or I,
X29 is L, N, R, Y, S or Q,
X30 is S, K or T,
X31 is S or M,
X32 is S, A or Y,
X33 is S, T or R,
X34 is N or H,
X35 is V or I,
X36 is R or K,
X37 is nonexistent, V, A, G, M or N,
X38 is nonexistent, T, I, N, F or S, and
X39 is nonexistent, R, H, V, K, I or Q.

In some aspects, the amino acid sequence with at least 70% sequence identity with the amino acid sequence represented by SEQ ID NO 75 consists of 80 to 130, specifically 90 to 120, most specifically 91 to 119, amino acid sequences.

In some aspects, the polypeptide has at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, or at least 95% sequence identity with the amino acid sequence represented by SEQ ID NO 75.

In some aspects, the polypeptide further comprises an amino acid sequence of General Formula 2 (from N-terminal to C-terminal):

<General Formula 2> X1-X2-H-H-K-A-X3-H

In General Formula 2,
X1 is A or V,
X2 is N or I, and
X3 is H or Q.

In some aspects, the polypeptide comprises an amino acid sequence of General Formula 2-General Formula 1 (from N-terminal to C-terminal).

In some aspects, the polypeptide further comprises an amino acid sequence of General Formula 3 (from N-terminal to C-terminal):

<General Formula 3> X1-X2-X3-X4-X5-X6-X7-X8-X9-X10

In General Formula 3,
X1 is A or S,
X2 is L, T or S,
X3 is Q, E or H,
X4 is nonexistent, P, L or H,
X5 is P or R,
X6 is T, S or I,
X7 is A, P, T, S or H,
X8 is T, A, S or I,
X9 is V or A, and
X10 is L or H.

In some aspects, the polypeptide includes an amino acid sequence of General Formula 3- General Formula 1 (from N-terminal to C-terminal).

In some aspects, the polypeptide further comprises an amino acid sequence of General Formula 4 (from N-terminal to C-terminal):

<General Formula 4> X1-X2

In General Formula 4,
X1 is H or Y, and
X2 is Q, V or L.

In some aspects, the polypeptide includes an amino acid sequence of General Formula 4-General Formula 1 (from N-terminal to C-terminal).

In some aspects, the polypeptide further comprises the following amino acid sequences (from N-terminal to C-terminal):
LHRP (SEQ ID NO 293);
LHQSGFTSGHFPHHRKLGE (SEQ ID NO 294); or
LAPPGTNIQI (SEQ ID NO 295).

In some aspects, the polypeptide further comprises an amino acid sequence of General Formula 5 (from N-terminal to C-terminal):

<General Formula 5> X1-X2-X3

In General Formula 5,
X1 is P or G,
X2 is R or H, and
X3 is T, S or L.

In some aspects, the polypeptide further comprises the following amino acid sequences (from N-terminal to C-terminal):
PYTSL (SEQ ID NO 296); or
QEDKLK (SEQ ID NO 297).

In some aspects, the polypeptide comprises an amino acid sequence of LHRP-General Formula 1, LHQSGFTSGHFPHHRKLGE-General Formula 1 or LAPPGTNIQI-General Formula 1 (from N-terminal to C-terminal). In some aspects, the polypeptide comprises an amino acid sequence of General Formula 1-General Formula 5, General Formula 1-PYTSL or General Formula 1-QEDKLK (from N-terminal to C-terminal). In some aspects, the polypeptide compriss an amino acid sequence of LHRP-General Formula 1-General Formula 5, LHQSGFTSGHFPHHRKLGE-General Formula 1-General Formula 5, LAPPGTNIQI-General Formula 1-QEDKLK, or LAPPGTNIQl-General Formula 1-PYTSL (from N-terminal to C-terminal).

In some aspects, the polypeptide comprises one or more amino acid sequence selected from a group consisting of amino acid sequences represented by SEQ ID NOS 75 to 129. In some aspects, the polypeptide comprises one or more amino acid sequence selected from a group consisting of amino acid sequences represented by SEQ ID NOS 130 to 183. In some aspects, the polypeptide further comprises a signal sequence. The polypeptide comprises one or more amino acid sequence selected from a group consisting of amino acid sequences represented by SEQ ID NOS 298 to 304.

In some aspects, the pharmaceutical composition described herein comprises a recombinant adeno-associated virus and a pharmaceutically acceptable carrier, wherein the recombinant adeno-associated virus comprises (a) a AAV 8-type capsid protein and (b) a polynucleotide comprising (i) a transgene comprising a nucleotide sequence with at least 70% sequence identity with the nucleotide sequence represented by SEQ ID NO 184, and (ii) a regulatory element operably linked to the transgene, wherein the regulatory element comprises: (1) a CMV enhancer sequence represented by SEQ ID NO 4; (2) a chicken β actin promoter sequence represented by SEQ ID NO 8; (3) a chicken β actin exon 1 (E1) sequence represented by SEQ ID NO 15; (4) a splicing donor sequence of chicken β actin intron represented by SEQ ID NO 10; (5) a non-translated nucleic acid sequence comprising, consisting essentially of, or consisting of a nucleotide sequence represented by SEQ ID NO 2, SEQ ID NO 3 or SEQ ID NO 57; and (6) an EF-1α exon 2 (E2) sequence represented by SEQ ID NO 11 (in the 5' to 3' direction).

In some aspects, the pharmaceutical composition is for use in preventing, alleviating or treating a retinal or macular disease.

In some aspects, the retinal or macular disease is caused by the damage to all or part of the retina or macula. In some aspects, the retinal or macular disease is a disease caused by the dysfunction or damage of the retinal or macular cells. In some aspects, the retinal or macular disease may be a disease caused by the dysfunction or damage of photoreceptor cells and/or retinal pigment epithelial (RPE) cells of the retina or macula. In some aspects, the retinal or macular disease is retinopathy, choroidal neovascularization, maculopathy, macular degeneration, retinal degeneration, macular edema, retinal edema, macular swelling, retinal swelling, retinal cell degeneration, retinal vascular occlusion, retinal detachment, inherited retinal disease or a combination thereof. In some aspects, the macular degeneration is age-related macular degeneration, Best's macular dystrophy, Sorsby's fundus dystrophy, malattia leventinese, Doyne's honeycomb retinal dystrophy, Stargardt's disease (Stargardt's macular dystrophy), myopic macular degeneration, or pigment epithelial detachment-related macular degeneration. In some aspects, the macular degeneration is age-related macular degeneration. In some aspects, the age-related macular degeneration is wet or dry age-related macular degeneration. In some aspects, the retinopathy is retinal dystrophy. In some aspects, the retinopathy is diabetic retinopathy. In some aspects, the diabetic retinopathy is non-proliferative diabetic retinopathy (NPDR), proliferative diabetic retinopathy (PDR), diabetic maculopathy, diabetic macular edema, or a combination thereof. In some aspects, the retinal or macular disease is an inherited retinal disease. In some aspects, the inherited retinal disease is retinitis pigmentosa (RP), Leber congenital amaurosis, Stargardt's disease, Coats retinopathy, cone dystrophy, choroideremia, Usher syndrome, Best disease, X-linked retinoschisis, or unspecified hereditary retinal dystrophy.

The pharmaceutically acceptable carriers that can be used with the present disclosure are those that are commonly used for formulation. Examples of the pharmaceutically acceptable carriers include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present disclosure can further include one or more additives selected from the group consisting of lubricating agents, wetting agents, sweetening agents, flavoring agents, emulsifying agents, suspending agents, and preservatives. Details of suitable pharmaceutically acceptable carriers and formulations can be found in Remington's Pharmaceutical Sciences (19th ed., 1995).

A pharmaceutical composition of the disclosure is formulated to be compatible with its intended route of administration. Examples of suitable parenteral routes of administration include intravenous injection, transdermal administration, subcutaneous injection, intramuscular injection, intraocular injection (e.g., any administration that can deliver to sub-tenon, subconjunctival, suprachoroidal, suprachoroidal space, subretinal, intravitreal, or similar sites), eye drop administration, intracerebroventricular injection, intrathecal injection, intraamniotic injection, intraarterial injection, intraarticular injection, intracardiac injection, intracavernous injection, intracerebral injection, intraocisternal injection, intracoronary injection, intracranial injection, intradural injection, epidural injection, intrahippocampal injection, intranasal injection, intraosseous injection, intraperitoneal injection, intrapleural injection, intraspinal injection, intrathoracic injection, intrathymic injection, intrauterine injection, intravaginal injection, intraventricular injection, intravesical injection, subconjunctival injection, intratumoral injection, local injection, intraperitoneal injection, and combinations thereof. In some aspects, the intraocular administration includes suprachoroidal, subretinal and intravitreal administration.

In some aspects, the pharmaceutical composition is administered in a daily dose of 0.0001 to 100 mg/kg.

The pharmaceutical composition of the present disclosure can be formulated with one or more pharmaceutically acceptable carriers and/or excipients. The pharmaceutical composition can be provided in unit dosage forms or dispensed in multi-dose containers. The formulation can be in the form of a solution, suspension or emulsion in an oil or aqueous medium or can be in the form of an extract, powder, granule, tablet or capsule. The formulation can further include a dispersant or a stabilizer.

### VIII. Kits

Also disclosed herein are kits comprising one or more polynucleotides as disclosed herein (e.g., comprising a transgene and an untranslated nucleic acid sequence), one or more vectors (*e.g.,* AAV vector) as disclosed herein, one or more cells (*e.g.,* the host cell comprising the AAV vector or transformed with the AAV vector) as disclosed herein, any pharmaceutical composition as disclosed herein, or any combination thereof. In some aspects, the kit also comprises instructions for administering any of the aforesaid, or a combination thereof, to a subject in need thereof.

The terms "kit" and "system," as used herein, are intended to refer to at least one or more polynucleotides as disclosed herein, one or more vectors (e.g., AAV vector) as disclosed herein, one or more host cells as disclosed herein, any pharmaceutical composition as disclosed herein, or any combination thereof, which, in some aspects, are in combination with one or more other types of elements or components (e.g., other types of biochemical reagents, containers, packages, such as packaging intended for commercial sale, instructions of use, and the like).

### IX. Uses and Methods

### IX.A. Methods of Producing

Also disclosed herein are methods of producing a polypeptide encoded by a transgene (e.g., a nucleic acid encoding a polypeptide including an amino acid sequence with at least 70% sequence identity to SEQ ID NO: 75 or a nucleic acid sequence with at least 70% sequence identity to SEQ ID NO: 184). In some aspects, such a method comprises culturing a cell described herein (e.g., transduced with a polynucleotide comprising a transgene and an untranslated nucleic acid molecule) under suitable conditions and recovering the encoded protein. In certain aspects, a method of producing a polypeptide encoding by a transgene comprises administering a polynucleotide of the present disclosure (e.g., comprising a transgene and an untranslated nucleic acid molecule) to a subject in need thereof, such that the encoded polypeptide is produced in the subject. Additional disclosure relating to such *in vivo* method of producing a polypeptide is provided elsewhere in the present disclosure (see, e.g., therapeutic uses).

In some aspects, the present disclosure provides methods of producing a recombinant adeno-associated virus (rAAV), comprising a polynucleotide described herein (e.g., comprising a transgene and an untranslated nucleic acid sequence). In some aspects, a method of producing such a recombinant AAV, comprises culturing a cell that has been transfected with an AAV vector described herein under conditions whereby the recombinant AAV is produced. In some aspects, the method further comprises a step of isolating the produced recombinant virus particle.

In some aspects, the present disclosure provides a recombinant virus particle produced by the method.

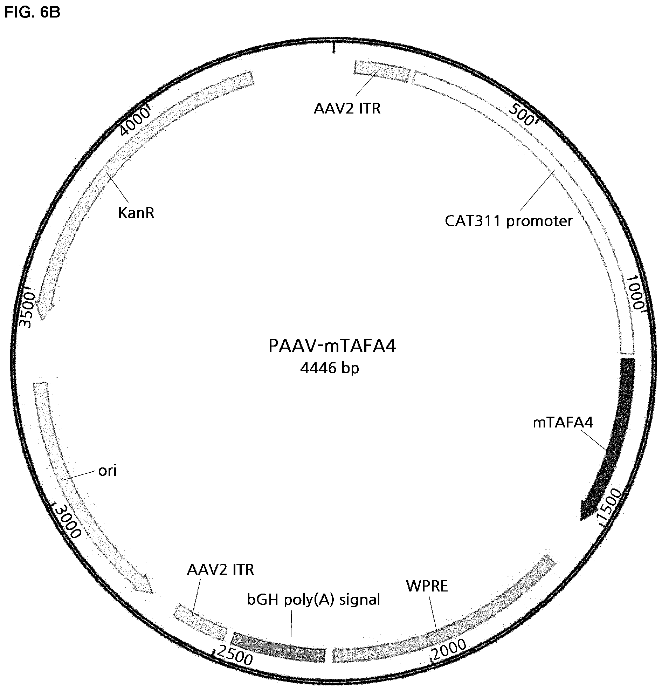

In some aspects, the recombinant virus particle may be produced by a method including a step of transfecting (i) an AAV vector containing the transgene (see, e.g., FIG. 6B) and (ii) a construct containing the rep and cap genes into a cell. Additionally, it may be produced using (iii) a helper construct for transducing a transgene into a host cell. In this aspect, the helper construct may contain an E2A gene that promotes AAV genome replication and gene transcription, an E4 gene that allows AAV mRNA to travel from the nucleus to the cytoplasm, and a VA region producing two VA RNAs regulating translation.

In some aspects, the above-described three constructs can be replaced by two constructs for transfection into the host cell. In such aspects, an AAV construct comprises a transgene and an untranslated nucleic acid sequence, and a separate construct comprises the rep and cap genes, the E2A gene, the E4 gene, and the VA region. Additional methods for producing AAV particles described herein are generally known in the art. *See, e.g.,* Clement et al., Mol Ther Methods Clin Dev 3: 16002 (Mar. 2016); Clark, Kidney Int. 61: S9-15 (Jan. 2002); and Xiao et al., J Virol 72(3): 2224-32 (Mar. 1998); each of which is incorporated herein by reference in its entirety.

The present disclosure also discloses a recombinant viral particle comprising (a) a capsid protein and (b) the AAV vector.

### IX.B. Therapeutic Uses

The nucleic acid molecules described herein (*e.g*., comprising a transgene and an untranslated nucleic acid sequence), vectors and recombinant viruses (*e.g*., rAAV) harboring such nucleic acid molecules, and methods described herein have numerous *in vitro* and *in vivo* utilities. For example, the polynucleotides described herein, *e.g.,* a vector, *e.g.,* an AAV vector, can be administered to cells in culture, *in vitro* or *ex vivo,* or to human subjects, *e.g., in vivo,* to treat diseases. Accordingly, in some aspects, the present disclosure provides use of any of the polynucleotides as described herein (*e.g*., comprising a transgene and an untranslated nucleic acid sequence), the recombinant expression construct as described herein, cells as described herein, pharmaceutical compositions as disclosed herein, or the recombinant virus as described herein for therapeutic applications.

In some aspects, disclosed herein is a method of expressing a transgene in a subject in need thereof, comprising administering to the subject a polynucleotide as disclosed herein (*e*.*g*., comprising a transgene and an untranslated nucleic acid sequence), a vector as disclosed herein, a recombinant virus (*e*.*g*., rAAV) as disclosed herein, a cell as disclosed herein, or a pharmaceutical composition as disclosed herein, wherein after the administration, the expression of the transgene is increased in the subject.

As described herein, the untranslated nucleic acid sequences of the present disclosure can increase the expression of the transgene when the transgene is translated. Accordingly, in some aspects, the present disclosure is directed to a method of increasing the expression of a transgene in a cell, comprising contacting the cell with any of the polynucleotides, vectors, or recombinant viruses (*e*.*g*., rAAV) as disclosed herein. The contacting can occur *ex vivo* or *in vivo.* When the contacting occurs *in vivo,* the method can further comprise administering any of the polynucleotides, vectors, or recombinant viruses to the subject prior to the contacting.

In some aspects, after the contacting, the expression of the transgene is increased by at least about 1 fold, at least about 1.1 fold, at least about 1.2 fold, at least about 1.3 fold, at least about 1.4 fold, at least about 1.5 fold, at least about 1.6 fold, at least about 1.7 fold, at least about 1.8 fold, at least about 1.9 fold, at least about 2 fold, at least about 2.5 fold, at least about 3 fold, at least about 3.5 fold, at least about 4 fold, at least about 5 fold, at least about 6 fold, at least about 7 fold, at least about 8 fold, at least about 9 fold, or at least about 10 fold or more, compared to a reference expression. In some aspects, the reference expression is the expression of the transgene in the cell prior to the contacting. In some aspects, the reference expression is the expression of the transgene in a corresponding cell that was not contacted with the polynucleotide, vector, or recombinant virus described herein (*e*.*g*., either lack the untranslated nucleic acid sequence or comprises the nucleotide sequence set forth in SEQ ID NO: 1).

Another aspect of the present disclosure provides a method for treating a disease in a subject in need thereof, comprising administering an effective amount of any of the polynucleotides, vectors, cells, recombinant viruses, or pharmaceutical compositions to the subject. As is apparent from the present disclosure, the compositions described herein (*e*.*g*., polynucleotides, the recombinant expression constructs, cells, pharmaceutical compositions, or recombinant viruses) can be used to treat any disease of interest, *e.g*., by modifying the transgene.

In some aspects, the method may further comprising administering an additional therapeutic agent (e.g., an inhibitor of vascular endothelial growth factor ("VEGF")) to the subject. In some aspects, the additional therapeutic agent may be administered to the subject before, concurrently with, or after administration of the polypeptide, polynucleotide, vector, cell, recombinant virus, or pharmaceutical composition.

The diseases that can be prevented, alleviated or treated by the present disclosure are is limited, but includes all diseases that require reduction in the number of drug administration. Non-limiting examples of the disease include retinal or macular diseases. In some aspects, the retinal or macular disease is selected from retinopathy, choroidal neovascularization, maculopathy, macular degeneration, retinal degeneration, macular edema, retinal edema, macular swelling, retinal cell degeneration, retinal vascular occlusion, retinal detachment, inherited retinal disease, and a combination thereof.

In some aspects, a retinal or macular disease that can be treated with the present disclosure comprises a macular degeneration. In some aspects, the macular degeneration comprises age-related macular degeneration (AMD). Age-related macular degeneration can be divided into dry (atrophic) macular degeneration and wet (neovascular or exudative) macular degeneration. Age-related macular degeneration can also be divided into early AMD, intermediate AMD, and late or advanced AMD (geographic atrophy). In some aspects, an ophthalmic disease that can be treated with the present disclosure comprises a diabetic retinopathy. In some aspects, diabetic retinopathy is non-proliferative diabetic retinopathy (NPDR). In some aspects, diabetic retinopathy is proliferative diabetic retinopathy (PDR). In some aspects, diabetic retinopathy is diabetic maculopathy. In some aspects, diabetic retinopathy is diabetic macular edema. In some aspects, diabetic retinopathy is any retinopathy associated with an ischemic damage within the retina. Unless indicated otherwise, the present disclosure can be used to treat all forms of AMD and/or diabetic retinopathy.

Another aspect of the present disclosure provides a gene therapeutic agent or a method for treating a disease that can achieve sustained transgene expression.

The use of the viral delivery system described herein enables administration of the composition described herein (*e*.*g*., polynucleotides, the recombinant expression constructs, cells, pharmaceutical compositions, or recombinant viruses) at intervals of about 1 week, about 2 weeks, about 3 weeks, about 1 month, about 2 months, about 3 months, about 4 months, about 5 months, about 6 months, about 7 months, about 8 months, about 9 months, about 10 months, about 11 months, or about 1 year or longer. In some aspects, the interval is about 2 to about 3 months. In some aspects, the interval is about 6 months. In some aspects, the interval is about 1 year. In some aspects, the interval is at least about 1 year. That is, the use of the viral delivery system described herein leads to a drastic reduction in the frequency of administration of the composition, allowing a doctor, patient, or subject to avoid an uncomfortable feeling caused by repeated administration of the composition. Depending on patient's symptoms or needs, the composition can be initially administered at least 2 or 3 times, at intervals of 1 to 2 weeks and then once every 2 to 3 months, every 6 months or every year or more.

### [Advantageous Effects]

The features and advantages of the present disclosure may be summarized as follows:
(i) The present disclosure provides a pharmaceutical composition for the prevention or treatment of retinal or macular diseases, which comprises a polypeptide comprising an amino acid sequence with at least 70% sequence identity with that of TAFA protein (e.g., TAFA1 to TAFA4), a nucleic acid encoding the polypeptide, a vector comprising the nucleic acid, a recombinant virus particle comprising the vector and capsid protein, or a cell comprising the nucleic acid or vector.
(ii) The present disclosure also provides an adeno-associated virus (AAV) vector comprising a nucleic acid encoding a polypeptide comprising an amino acid sequence with at least 70% sequence identity with the amino acid sequence of the TAFA protein, and a recombinant viral particle comprising the AAV vector and capsid protein.
(iii) The AAV vector, recombinant virus, or composition comprising thereof of the present disclosure may be useful in the prevention or treatment of retinal or macular diseases by recovering the damaged retina.

### [Brief Description of Drawings]

These and/or other aspects and advantages of the present disclosure will become apparent and more readily appreciated from the following description of the aspects, taken in conjunction with the accompanying drawings of which:
FIG. 1 shows a cleavage map of a pAAV-eGFP expression construct as described herein.
FIGS. 2A and 2B show increases in the expression of eGFP in cells transduced with expression constructs comprising the full-length EF-1α intron in combination with an EF-1α promoter ("CEE-FL"). Control cells were transduced with the corresponding expression constructs that lacked an EF-1α intron ("CE"). In FIG. 2A, ITR-lacking animal cell expression constructs were used. In FIG. 2B, ITR-containing pAAV expression constructs were used. In both FIGS. 2A and 2B, the eGFP expression is shown in the following transduced cell lines (HEK293, HeLa, ARPE-19, RPE-1, Huh-7, and Hep3B).

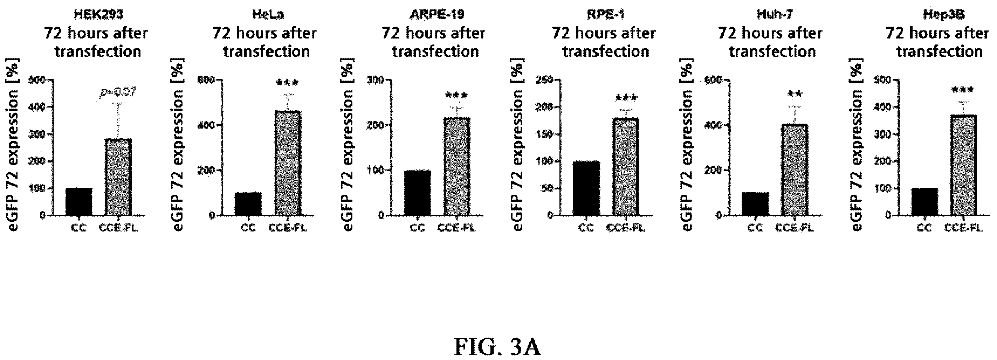

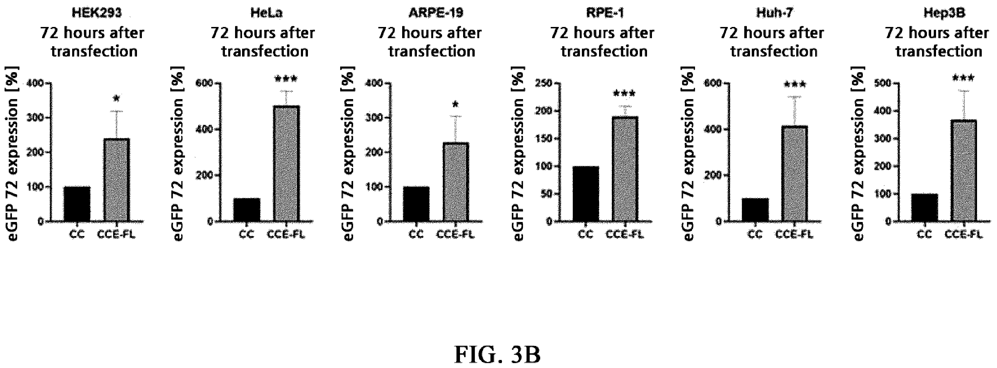

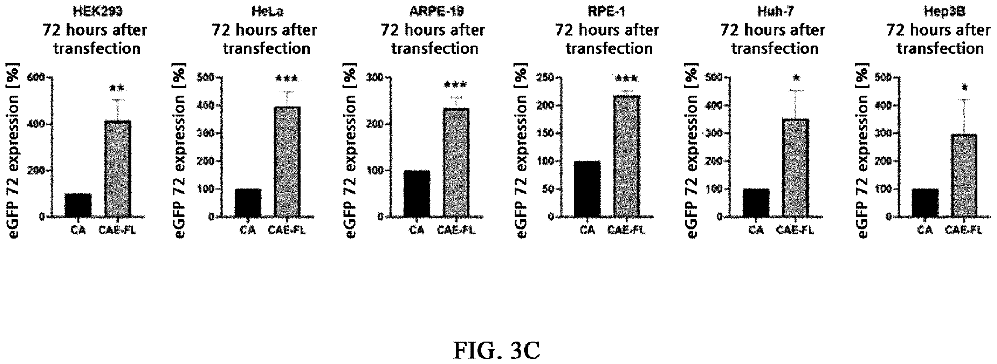

FIGS. 3A, 3B and 3C show the expression of eGFP in cells transduced with expression constructs comprising the full-length EF-1α intron in combination with various promoters ("CCE-FL"). Control cells were transduced with the corresponding expression constructs but lacking the EF-1α intron. In FIG. 3A, a combination of a CMV enhancer and CMV promoter were inserted into animal cell expression constructs *(i.e.,* lacking ITR) and used to transduce the cells. In FIG. 3B, the combination of CMV enhancer and CMV promoter were inserted into pAAV expression constructs and used to transduce the cells. In FIG. 3C, the cells were transduced with animal cell expression constructs comprising a combination of a CMV enhancer and a chicken-β actin promoter. In each of FIGS. 3A, 3B, and 3C, the eGFP expression is shown in the following transduced cell lines (HEK293, HeLa, ARPE-19, RPE-1, Huh-7, and Hep3B).

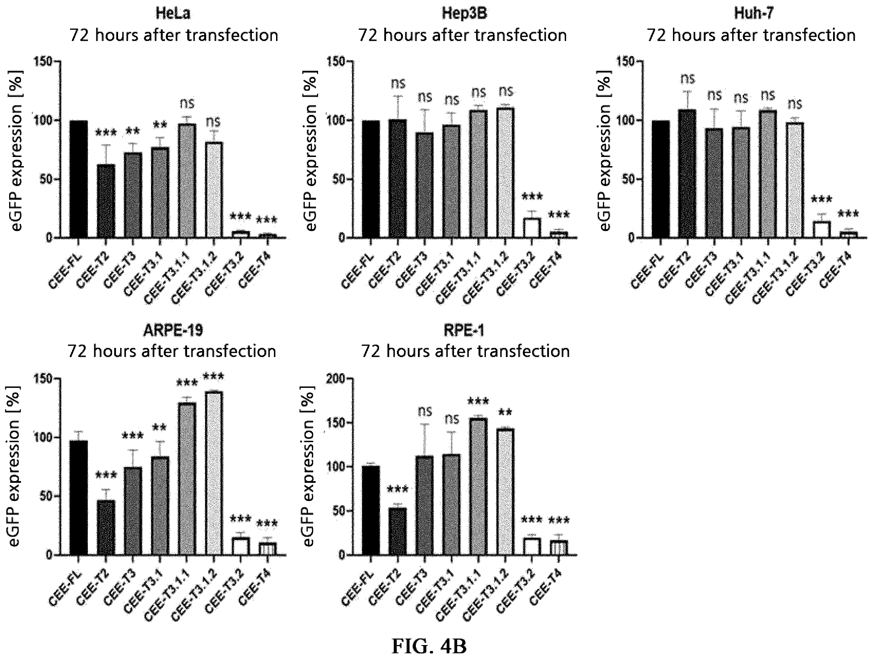

FIGS. 4A and 4B show the increasing or decreasing effect of various EF-1α intron fragment sequences (*i.e.,* untranslated nucleic acid sequences described herein) on gene expression. FIG. 4A schematically shows a series of CEE constructs prepared by sequentially truncating an EF-1α intron sequence. Each of the constructs shown included the following: (1) a CMV enhancer (380 base pairs); (2) a promoter comprising a CMV promoter portion (first 31 base pairs from the 5'-end) and EF-1α promoter (201 base pairs); and (3) EF-1α exon 1 (E1) sequence (29 base pairs). The control "CE" construct did not include any additional components, including the EF-1 alpha intron sequence. The other constructs further comprises the following additional components: (4) splice donor sequence, which consisted of the first 19 base pairs from the 5'-end of the full-length EF-1 alpha intron sequence (*i.e.,* SEQ ID NO: 1); (5) EF-1α intron sequence; and (6) EF-1α exon 2 (E2) sequence (nine base pairs). The EF-1α intron sequences included the full-length sequence (924 base pairs) ("CEE-FL") or ; (ii) nucleotides 570 to 924 of SEQ ID NO: 1 (355 base pairs) ("CEE-T2"); (iii) nucleotides 721 to 924 of SEQ ID NO: 1 (204 base pairs) ("CEE-T3"); (iv) nucleotides 808 to 924 of SEQ ID NO: 1 (117 base pairs) ("CEE-T3.1"); (v) nucleotides 830 to 924 of SEQ ID NO: 1 (95 base pairs) ("CEE-T3.1.1"); (vi) nucleotides 852 to 924 of SEQ ID NO: 1 (73 base pairs) ("CEE-T3.1.2"); (vii) nucleotides 874 to 924 of SEQ ID NO: 1 (51 base pairs) ("CEE-T3.2"); and (viii) nucleotides 896 to 924 of SEQ ID NO: 1 (29 base pairs) ("CEE-T4"). The total length of each of the constructs are provided to the right. FIG. 4B shows the effect of the different EF-1α intron sequences (full-length or truncated) on eGFP (*i.e.,* transgene) expression in five different cell lines (HeLa, Hep3B, Huh-7, ARPE-19, and RPE-1). ARPE-19 and RPE-1 cells originated from retina. Huh-7 and Hep3B cells originated from the liver. HeLa cells originated from the cervix. eGFP expression is shown as a percentage of expression observed in cells transduced with the transgene using the CEE-FL construct. "ns" = no significance. "**" *= p* < 0.01 and "***" *= p <* 0.001.

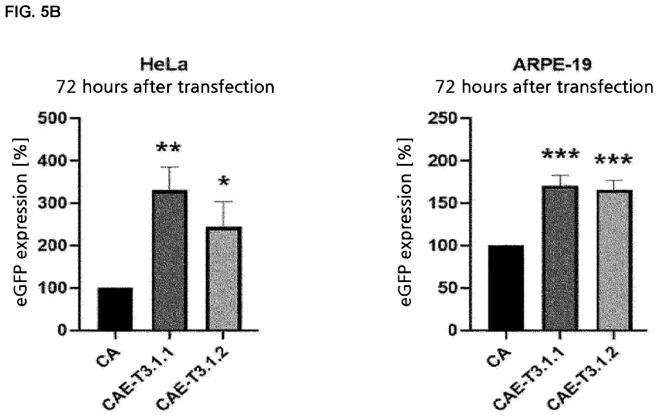

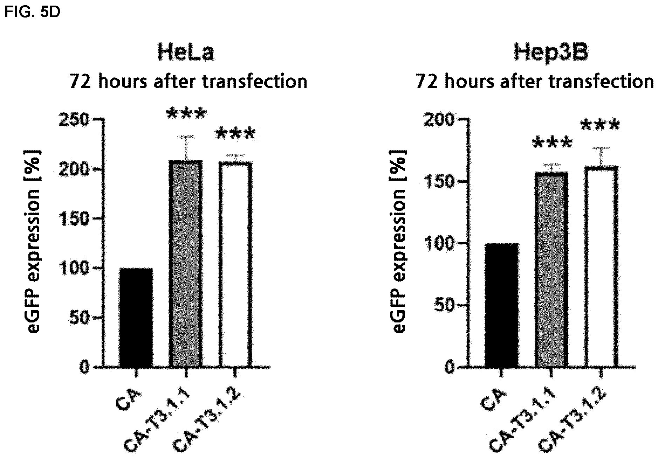

FIGS. 5A, 5B, 5C, and 5D show the abilities of EF-1α intron fragments T3.1.1 (*i.e.,* nucleotides 830 to 924 of SEQ ID NO: 1 (95 base pairs)) and T3.1.2 (*i.e.,* nucleotides 852 to 924 of SEQ ID NO: 1 (73 base pairs)) to increase transgene expression. FIG. 5A schematically shows a series of CAE constructs including EF-1α intron fragments T3.1.1 and T3.1.2 in combination with the following additional components: (1) CMV enhancer (380 base pairs); (2) a chicken β-actin promoter (279 base pairs); (3) chicken β-actin exon 1 (E1) sequence (32 base pairs); (4) EF-1α exon 1 (E1) sequence (29 base pairs); (5) splice donor sequence, which consisted of the first 19 base pairs from the 5'-end of the full-length EF-1α intron sequence (*i.e.,* SEQ ID NO: 1); and (6) EF-1α exon 2 (E2) sequence (nine base pairs). The control "CA" construct included only the CMV enhancer, chicken β-actin promoter, and the chicken β-actin E1 sequence. The control "CAE-FL" construct included the full-length EF-1α intron sequence. The total length of the constructs are provided to the left. FIG. 5B shows eGFP (*i.e.,* transgene) expression in HeLa (left graph) and ARPE-19 (right graph) cells transduced using the CAE-T3.1.1 and CAE-T3.1.2 constructs. Gene expression is shown as a percentage of expression in corresponding cells transduced using the CA construct (*i.e.,* lacked any EF-1α intron sequence). FIG. 5C schematically shows a series of hybrid intron CA constructs (*i.e.,* CA-T3.1.1 and CA-T3.1.2 constructs) including both EF-1α intron fragment T3.1.1 or T3.1.2 and a chicken β-actin intron fragment. The two constructs further comprises: (1) CMV enhancer; (2) chicken β-actin promoter; (3) chicken β-actin E1 sequence; and (4) EF-1α E2 sequence. The control "CAG-FL" construct included the following: (1) CMV enhancer (380 base pairs); (2) chicken β-actin promoter (279 base pairs); (3) chicken β-actin E1 sequence (93 base pairs); (4) a chimeric intron (comprising intron from chicken β-actin and rabbit β-globin) (924 base pairs); and (5) rabbit β-globin exon 3 (E3) sequence (48 base pairs). The "CA" construct is the same as that described in FIG. 5A. FIG. 5D shows eGFP expression in HeLa (left graph) and Hep3B (right graph) cells transduced using the constructs CA-T3.1.1 and CA-T3.1.2. Gene expression is shown as a percentage of expression in corresponding cells transduced using the CA construct (*i.e.,* lacked any EF-1α intron sequence).
FIGS. 6A and 6B show the cleavage maps of AAV constructs for producing AAV8. FIG. 6A shows a pAAV construct which does not contain a transgene, and FIG. 6B shows a pAAV-mTAFA4 construct which contains mouse TAFA4 gene as a transgene.
FIG. 7 shows a result of analyzing the effect of recovering retinal damage in a NaIO₃-induced retinal damage model with subretinal administration of a control group (control AAV8) or a test group (AAV8.mouse TAFA4) using fluorescence angiography (FA). OD (oculus dexter) refers to right eye, and OS (oculus sinister) refers to the left eye.
FIG. 8 shows a result of measuring the extent of change in A-wave and B-wave amplitudes in an electroretinogram (ERG) for the control group (control AAV8) or the test group (AAV8.mouse TAFA4).
FIG. 9 shows a result of relative comparing TAFA4 expression before and after the administration of NaIO₃ and the TAFA4 mRNA expression after administration of a test group.
FIG. 10 shows a result of comparing the interspecies sequence identity of full-length TAFA4 proteins.
FIG. 11 shows a result of analyzing the effect of recovering retinal damage in a NaIO₃-induced retinal damage model withsubretinal administration of a control group (control AAV8) or test groups (AAV8.human TAFA4, AAV8.gecko TAFA4, AAV8.fish TAFA4) using fluorescence angiography (FA). OD (oculus dexter) refers to right eye, and OS (oculus sinister) refers to the left eye.
FIG. 12 shows a result of measuring the extent of change in A-wave and B-wave amplitudes in an electroretinogram (ERG) for a control group (control AAV8) and test groups (AAV8.human TAFA4, AAV8.gecko TAFA4, AAV8.fish TAFA4).
FIG. 13 shows a result of comparing the mRNA and protein expression levels of TAFA4 with different signal sequences.
FIG. 14 shows a result of analyzing the effect of recovering retinal damage in a NaIO₃-induced retinal damage model with subretinal administration of a (control AAV8) or experimental groups (AAV8.TAFA1, AAV8.TAFA2, AAV8.TAFA3, AAV8.TAFA4) by fluorescence angiography (FA). OD (oculus dexter) refers to right eye, and OS (oculus sinister) refers to the left eye.
FIG. 15 shows a result of measuring the extent of change in A-wave and B-wave amplitudes in an electroretinogram (ERG) for a control group (control AAV8) and test groups (AAV8.TAFA1, AAV8.TAFA2, AAV8.TAFA3, AAV8.TAFA4).
FIG. 16 shows a result of comparing the interspecies sequence identity of mature TAFA1 proteins.
FIG. 17 shows a result of comparing the interspecies sequence identity of mature TAFA2 proteins.
FIG. 18 shows a result of comparing the interspecies sequence identity of mature TAFA3 proteins.
FIG. 19 shows a result of comparing the interspecies sequence identity of mature TAFA4 proteins.
FIGS. 20A to 20D show a result of comparing the interspecies amino acid sequence identity of full-length TAFA1, TAFA2, TAFA3 and TAFA4. FIG. 20A compares amino acid sequences 1-70, FIG. 20B compares amino acid sequence 71-140, FIG. 20C compares amino acid sequences 141-210, and FIG. 20D compares amino acid sequences 211-244.
FIG. 21 shows a result of comparing the sequence identity of mature proteins of TAFA1, TAFA2, TAFA3 and TAFA4 in human.

### [Best Mode]

Hereafter, the present disclosure will be described in more detail through examples. These examples are intended solely to explain the present disclosure in more detail, and it will be obvious to those having ordinary knowledge in the art that the scope of the present disclosure is not limited by the examples.

### Examples

### Materials and methods

### Example 1. Preparation of pAAV-eGFP construct including no enhancer-promoter-intron sequences

### Example 1-1. Insertion of bGH polyadenylation signal sequence

Polymerase chain reaction (PCR) was performed using a pcDNA5/FRT/TO construct (Invitrogen, USA, Cat. No. V6520-20) as a template and oligo #001 and #002 to obtain a bovine growth hormone (bGH) polyadenylation signal A sequence (poly A) fragment. Next, human growth hormone (hGH) poly A was removed and bGH poly A was inserted using the Bglll/BstEll sites of a pAAV-MCS-promoterless plasmid (Cellbiolabs, USA, Cat. No. VPK-411).

### Example 1-2. Insertion of eGFP

Human codon optimized eGFP was derived from a pUCIDT-KAN-eGFP construct (GeneArt, Germany) and cloned into the BamHI/Hindlll sites of the *pAAV-bGH* construct prepared in Example 1-1.

### Example 1-3. Insertion of WPRE sequence

A woodchuck hepatitis virus posttranscriptional regulatory element sequence was obtained from a pUC57-WPRE construct (GenScript, USA) and cloned into the Hindlll/Bglll sites of the *pAAV-eGFP-bGH* construct prepared in Example 1-2.

### Example 1-4. Insertion of 4 copies of miRNA142-3p target sequences

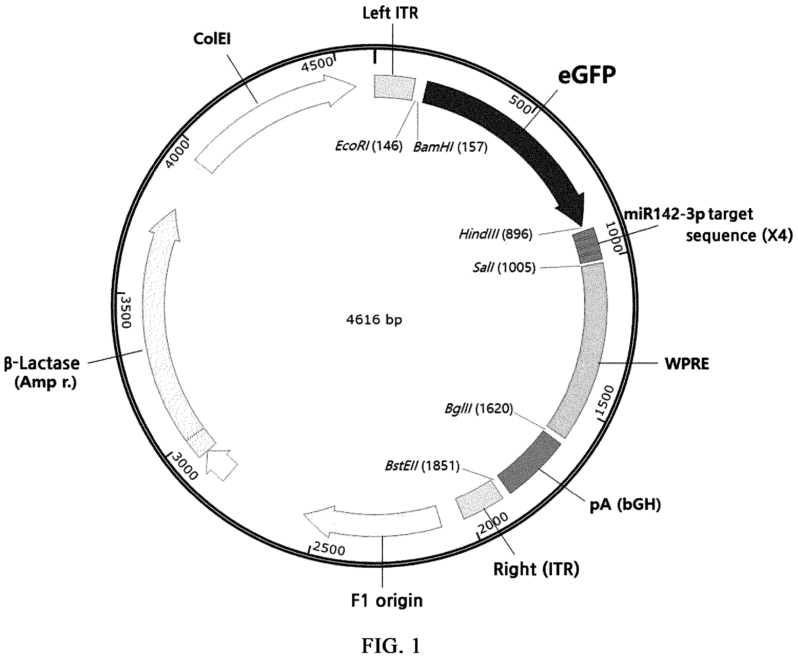

Oligo #003 and #004 were annealed and Oligo #005 and #006 were annealed to prepare two DNA fragments, each of which consisted of 2 copies of miRNA142-3p target sequences. The two short DNA fragments were cloned into the Hindlll/Sall sites of the *pAAV-eGFP-WPRE-bGH* construct prepared in Example 1-3 such that a total of 4 copies of the miRNA142-3p target sequences were inserted. As a result, a pAAV-eGFP construct including no enhancer-promoter-intron sequences was prepared (FIG. 1).

### Example 2. Preparation of constructs including various types of enhancer-promoter-intron sequences

### Example 2-1. Preparation of constructs including CEE series (CEE-FL, CEE-T2, -T3, -T3, -T3.1, -T3.1.1, -T3.1.2, -T3.2, -T4) and CE sequences

### Example 2-1-1. Preparation of construct including CEE-FL (full length) nucleotide sequence

A CEE-FL (CMV enhancer (SEQ ID NO: 4)-31 bp CMV promoter (SEQ ID NO: 5)-EF-1α promoter (SEQ ID NO: 7)-29 bp EF-1α exon 1 (SEQ ID NO: 13)-EF-1α intron (SEQ ID NO: 1)-9 bp EF-1α exon 2 (SEQ ID NO: 11)) DNA fragment was obtained from a pMK-RQ3_PEM construct (GeneArt, Germany) and cloned into the EcoRl/BamHl sites of the construct prepared in Example 1.

### Example 2-1-2. Preparation of construct including CEE-T2 nucleotide sequence

PCR was performed using the construct prepared in Example 2-1-1 as a template and oligo #007/008 and oligo #009/010 combinations. The resulting two DNA fragments were ligated with Gibson Assembly^{®} (NEB, USA, Cat. No. E2611) to prepare a construct having the CEE-T2 sequence.

### Example 2-1-3. Preparation of construct including CEE-T3 nucleotide sequence

PCR was performed using the construct prepared in Example 2-1-1 as a template and oligo #011/008 and oligo #012/010 combinations. The resulting two DNA fragments were ligated with Gibson assembly to prepare a construct having the CEE-T3 sequence.

### Example 2-1-4. Preparation of construct including CEE-T3.1 nucleotide sequence

PCR was performed using the construct prepared in Example 2-1-1 as a template and oligo #013/008 and oligo #014/010 combinations. The resulting two DNA fragments were ligated with Gibson assembly to prepare a construct having the CEE-T3.1 sequence.

### Example 2-1-5. Preparation of construct including CEE-T3.2 nucleotide sequence

PCR was performed using the construct prepared in Example 2-1-1 as a template and oligo #015/008 and oligo #016/010 combinations. The resulting two DNA fragments were ligated with Gibson assembly to prepare a construct having the CEE-T3.2 sequence.

### Example 2-1-6. Preparation of construct including CEE-T4 nucleotide sequence

PCR was performed using the construct prepared in Example 2-1-1 as a template and oligo #017/008 and oligo #018/010 combinations. The resulting two DNA fragments were ligated with Gibson assembly to prepare a construct having the CEE-T4 sequence.

### Example 2-1-7. Preparation of construct including CEE-T3.1.1 nucleotide sequence

The CEE-T3.1.1 nucleotide sequence was obtained from a pUC57-T3.1.1 construct (GenScript, USA) and cloned into the EcoRl/BamHl sites of the construct prepared in Example 2-1-1.

### Example 2-1-8. Preparation of construct including CEE-T3.1.2 nucleotide sequence

The CEE-T3.1.2 nucleotide sequence was obtained from a pUC57-T3.1.2 construct (GenScript, USA) and cloned into the EcoRl/BamHl sites of the construct prepared in Example 2-1-1.

### Example 2-1-9. Preparation of construct including CE nucleotide sequence

A CE fragment was obtained from the construct prepared in Example 2-1-1 by PCR using oligo #019 and #020 and cloned into the EcoRl/BamHl sites of the construct prepared in Example 2-1-1.

### Example 2-2. Preparation of construct including CCE-FL sequence and construct including CC sequence

### Example 2-2-1. Preparation of construct including CCE-FL sequence

A DNA fragment having the CCE-FL sequence (CMV enhancer (SEQ ID NO: 4)-CMV promoter (SEQ ID NO: 6)-30 bp CMV exon 1 (SEQ ID NO: 12)-29 bp EF-1α exon 1 (SEQ ID NO: 13)-EF-1α intron (SEQ ID NO: 1)-9 bp EF-1α exon 2 (SEQ ID NO: 11)) was obtained from a pMK-RQ4_PME construct (GeneArt, Germany). The DNA fragment was cloned into the EcoRl/BamHl sites of the pAAV-eGFP construct prepared in Example 1.

### Example 2-2-2. Preparation of construct including the CC sequence

A CC fragment was obtained from the construct prepared in Example 2-2-1 by PCR using oligo #019 and #021 and cloned into the EcoRl/BamHl sites of the construct prepared in Example 2-2-1.

### Example 2-3. Preparation of construct including CAG-FL sequence, construct including CA-T3.1.1 sequence, construct including CA-T3.1.2 sequence, and construct including CA sequence

### Example 2-3-1. Preparation of construct including CAG-FL sequence

A CAG fragment was obtained from a pCAG-Neo construct (Wako Pure Chemical Industries, Ltd., Japan, Cat. No. 163-25601) and cloned into the SnaBI/BamHI sites of the construct prepared in Example 2-1.

### Example 2-3-2. Preparation of construct including CA sequence

A CA fragment was obtained from a pUC57-CA construct (GenScript, USA) and cloned into the EcoRl/BamHl sites of the construct prepared from Example 2-3-1.

### Example 2-3-3. Preparation of construct including CA-T3.1.1 sequence

A T3.1.1 fragment was obtained from the construct prepared in Example 2-1-7 by PCR using oligo #022 and #023 and cloned into the Afel/BamHI sites of the construct prepared in Example 2-3-1.

### Example 2-3-4. Preparation of construct including CA-T3.1.2 sequence

A T3.1.2 fragment was obtained by annealing oligo #024 and #025 and cloned into the Afel/BamHI sites of the construct prepared from Example 2-3-1.

### Example 2-4. Preparation of construct including CAE-FL sequence, construct including CAE-T3.1.1 sequence and construct including CAE-T3.1.2 sequence

### Example 2-4-1. Preparation of construct including CAE-FL sequence

A CAE (CMV enhancer (SEQ ID NO: 4)-chicken β-actin promoter (SEQ ID NO: 8)-32 bp chicken β-actin exon 1 (SEQ ID NO: 14)-29 bp EF-1α exon 1 (SEQ ID NO: 13)-924 bp EF-1α intron (SEQ ID NO: 1)-9 bp EF-1α exon 2 (SEQ ID NO: 11)) fragment was obtained from a pUC57-CAE construct (GenScript, USA) and cloned into the EcoRl/BamHl sites of the construct prepared from Example 1.

### Example 2-4-2. Preparation of construct including CAE-T3.1.1 sequence

A CAE-T3.1.1 fragment was obtained from the construct prepared in Example 2-1-7 using oligo #026 and #027 and cloned into the Kpnl/BamHI sites of the construct prepared in Example 2-1-7.

### Example 2-4-3. Preparation of construct including CAE-T3.1.2 sequence

A CAE-T3.1.2 fragment was obtained from the construct prepared in Example 2-1-8 using oligo #026 and #027 and cloned into the Kpnl/BamHI sites of the construct prepared in Example 2-1-8.

### Example 2-5. Preparation of pAAV constructs including aflibercept

An aflibercept DNA fragment was obtained from a pcDNA3.1(+)-IgG-aflibercept construct (Genscript, USA) by PCR using oligo #028 and #029 and cloned into the BamHI/Hindlll sites of each of the constructs prepared from Example 2-3-3.

### Example 2-6. Preparation of constructs including TAFA 1, 2, 3 and 4 sequences

### Example 2-6-1. Preparation of construct including mouse TAFA4 sequence

A DNA fragment of mouse TAFA4 (mTAFA4) obtained from a pMA-RQ-mouse TAFA4 construct (Geneart, Thermo Fisher Scientific, USA) using restriction enzymes BamHI/Hindlll was cloned into the BamHI/Hindlll site of the construct (including the CA-T3.1.1 fragment) prepared in Example 2-5. The mouse TAFA4 gene was codon-optimized for mice with SEQ ID NO 305.

### Example 2-6-2. Preparation of construct including human TAFA4 sequence

A DNA fragment of human TAFA4 (hTAFA4) obtained from a pMA-RQ-human TAFA4 construct (Geneart, Thermo Fisher Scientific, USA) using restriction enzymes BamHI/Hindlll was cloned into the BamHI/Hindlll site of the construct (including the CA-T3.1.1 fragment) prepared in Example 2-5. The human TAFA4 gene was codon-optimized for human with SEQ ID NO 306.

### Example 2-6-3. Preparation of construct including gecko TAFA4 sequence

A DNA fragment of gecko TAFA4 (gTAFA4) obtained from a pMK-RQ-gecko TAFA4 construct (Geneart, Thermo Fisher Scientific, USA) using restriction enzymes BamHI/Hindlll was cloned into the BamHI/Hindlll site of the construct (including the CA-T3.1.1 fragment) prepared in Example 2-6-2. The gecko TAFA4 gene was codon-optimized for mice with SEQ ID NO 307.

### Example 2-6-4. Preparation of construct including fish TAFA4 sequence

A DNA fragment of fish TAFA4 obtained from a pMK-RQ-fish TAFA4 construct (Geneart, Thermo Fisher Scientific, USA) using restriction enzymes BamHI/Hindlll was cloned into the BamHI/Hindlll site of the construct (including the CA-T3.1.1 fragment) prepared in Example 2-6-2. The fish TAFA4 gene was codon-optimized for mice with SEQ ID NO 308.

### Example 2-6-5. Preparation of construct including human TAFA1 sequence

A DNA fragment of human TAFA1 obtained from a pMK-RQ-human TAFA1 construct (GeneArt, Germany) using restriction enzymes BamHI/Hindlll was cloned into the BamHI/Hindlll site of the construct (including the CA-T3.1.1 fragment) prepared in Example 2-6-2. The human TAFA1 gene was codon-optimized for human with SEQ ID NO 309.

### Example 2-6-6. Preparation of construct including human TAFA2 sequence

A DNA fragment of human TAFA2 obtained from a pMK-RQ-human TAFA2 construct (GeneArt, Germany) using restriction enzymes BamHI/Hindlll was cloned into the BamHI/Hindlll site of the construct (including the CA-T3.1.1 fragment) prepared in Example 2-6-2. The human TAFA2 gene was codon-optimized for human with SEQ ID NO 310.

### Example 2-6-7. Preparation of construct including human TAFA3 sequence

A DNA fragment of human TAFA3 obtained from a pMK-RQ-human TAFA3 construct (GeneArt, Germany) using BamHI/Hindlll was cloned into the BamHI/Hindlll site of the construct (including the CA-T3.1.1 fragment) prepared in Example 2-6-2. The human TAFA3 gene was codon-optimized for human with SEQ ID NO 311.

### Example 2-7. Sequencing

The nucleotide sequences of all constructs obtained by cloning were verified by DNA sequencing (Macrogen, Korea or Bionics, Korea).

### Example 3. Cell culture

HEK293 and HeLa cell lines were cultured in MEM media (Gibco, USA, Cat. No. 42360-032) under humidified conditions at 5% CO₂ and 37 °C, ARPE-19 cell line was cultured in DMERM/F12 medium (Gibco, USA, Cat. No. 11330-032) under humidified conditions at 5% CO₂ and 37 °C, and RPE-1 and Hep3B cell lines were cultured in DMEM media (Gibco, USA, Cat. No. 10569-010) under humidified conditions at 5% CO₂ and 37 °C. All media had been supplemented with 10% fetal bovine serum (FBS, Gibco, USA, Cat. No. 16000-044) and 1% penicillin-streptomycin (Gioco, USA, Cat. No. 15140-163). Expi293 cell line was cultured in Expi293 medium (Gibco, USA, Cat. No. A14351-01) supplemented with 1% penicillin-streptomycin with shaking (250 rpm) under humidified conditions at 8% CO₂ and 37 °C.

### Example 4. Transduction

### Example 4-1. Transduction of adherent cells

Each of the cell lines was washed twice with DPBS (Gibco, USA, Cat. No. 14190-250), detached from the culture dish by treatment with trypsin-EDTA (Gibco, USA, Cat. No. 25200-114), and seeded in a 12-well plate to a confluency of 80%. After 24-h culture, lipofectamine 3000 (Thermo Fisher Scientific, USA, Cat. No. L300075) was used to transduce plasmid DNA into the cells.

### Example 4-2. Transduction of suspension cells for AAV production

For AAV production, 6Y10⁸ cells were seeded in 220 mL of expi293 medium in a 1 L Erlenmeyer culture flask. After ~3-4 h culture for stabilization, pHelper plasmid DNA, pUC-RC2 plasmid DNA, pUC-RC2 plasmid DNA or pUC-RC8 plasmid DNA, and transgene-containing AAV construct plasmid DNA (each 3.73 pmoles) were dissolved in 10 mL of Opti-MEM (Gibco, USA, Cat. No. 51985-034). Immediately after polyethylenimine (PEI, Polyscience, USA, Cat. No. 23966-1) was diluted with 10 mL of Opti-MEM, the two solutions were mixed to prepare a transduction solution. The PEI was used in an amount corresponding to twice the total amount of DNA. After incubation at room temperature for 30 min, a total of 20 mL of the transduction solution was added to the culture flask.

### Example 5. Purification of AAV

### Example 5-1. Purification of AAV2

72 hours after the transduction (Example 4-2), the cell culture solution was harvested, followed by centrifugation to remove the medium. The precipitated cells were washed with DPBS and resuspended with 16 mL of DPBS. The cells were lysed by three cycles of freezing/thawing and centrifuged to obtain an AAV2-containing supernatant. The supernatant was mixed with AAVanced^{™} concentration reagent (System Bioscience, USA, Cat. No. AAV110A-1) in a ratio of 4:1 at 4 °C for 16 h, followed by centrifugation. The supernatant was discarded and the AAV2-containing precipitate was washed with 500 µL of Opti-MEM. After complete removal of the supernatant, the precipitate was resuspended in 400 µL of ice-cold DPBS to obtain AAV.

### Example 5-2. Purification of AAV8

72 hours after the transduction (Example 4-2), the cell culture solution was harvested, filtered through a 0.45 µm filter to remove cell debris, and subjected to anion exchange and affinity chromatography to obtain AAV.

### Example 6. Titer determination of the AAV

The titer of the AAV2 purified in Example 5 was determined by qPCR (Bio-Rad, USA, CFX96). First, the AAV was treated with DNase I in DNase I reaction buffer (New England Biolab, USA, M0303S) at 37 °C for 1 hour. Thereafter, the DNase I-treated sample was treated with proteinase K (Invitrogen, USA, Cat. No. AM2548) at 55 °C for 30 min and incubated at 95 °C for 15 min to inactivate the proteinase K. The prepared samples were used as templates for qPCR and the AAV constructs were used to plot a standard curve (7.4Y10⁸-7.4Y10⁴, 10-fold dilution). A recombinant adeno-associated virus 2 reference standard stock (rAAV2-RSS, ATCC, USA, Cat. No. VR-1616) or recombinant adeno-associated virus 8 reference standard stock (rAAV8-RSS, ATCC, USA, Cat. No. VR-1816) was used as a positive control. For titer determination, qPCR was performed using 2YSsoAdvanced Universal Probe Supermix (Bio-Rad, USA, Cat. No. 172-5282) and AAV2-ITR specific primers (#030, #031) and probe (#032, FAM-CACTCCCTCTCTGCGCGCTCG-BHQ1). qPCR was repeated 40 times. Each qPCR cycle consisted of denaturation at 95 °C for 10 min, incubation at 95 °C for 30 sec, and subsequent incubation at 60 °C for 1 min. The standard curve and quantification were analyzed using Bio-Rad CFX Maestro 1.1 software (Bio-Rad, USA).

### Example 7. Measurement of expression level of GFP gene by flow cytometry

The eGFP expression was measured by flow cytometry (Beckman Coulter, USA, CytoFlex). The measured value of eGFP was calculated by correcting the efficiency of transduction by measuring red fluorescence by the pCMV-dsRed (Clontech, Japan, Cat. No. 632416) construct that was transduced together. Cells were washed with DPBS and detached with trypsin 72 hours after the transduction. The cells were collected after centrifuging for 5 minutes at 1500 rpm, and then resuspended by adding 500 µL of DPBS supplemented with 2% FBS. In flow cytometry, a single cell region was differentiated using the FSC vs SSC plot, and FL1-A (green) and FL2-A (red) were measured. Single fluorescence vector-transduced samples (pEGFP-C1, Clontech, Japan, Cat. No. PT3286-1 and pCMV-DsRed-Expression2) were used for confirming the measurement result. The results of flow cytometry were analyzed using FlowJo software 10.5.3 (Becton Dickinson & Company, USA).

**[Table 1]**

| Oligo No. | SEQ ID NO | Sequence (5' → 3') |
|---|---|---|
| 001 | 24 | GGAAGATCTCTGTGCCTTCTAGTTGCCAGC |
| 002 | 25 | CACGTGGTTACCCCATAGAGCCCACCGCATC |
| 003 | 26 | AGCTTTCCATAAAGTAGGAAACACTACACGATTCCATAAA GTAGGAAACACTACAACGTTC |
| 004 | 27 | TAGTGTTTCCTACTTTATGGAATCGTGTAGTGTTTCCTAC TTTATGGA |
| 005 | 28 | CATAAAGTAGGAAACACTACATCACTCCATAAAGTAGGAA ACACTACAG |
| 006 | 29 | TCGACTGTAGTGTTTCCTACTTTATGGAGTGATGTAGTGT TTCCTACTTTATGGAACGTTG |
| 007 | 30 | GTGTGTGGTTTGCTGCAGGGAGCTCAAAATG |
| 008 | 31 | CGGTGATGACGGTGAAAACC |
| 009 | 32 | CCCTGCAGCAAACCACACACGGCACTTACC |
| 010 | 33 | GGTTTTCACCGTCATCACCG |
| 011 | 34 | GTGTGTGGTTTCGAGCTTTTGGAGTACGTCG |
| 012 | 35 | AAAAGCTCGAAACCACACACGGCACTTACC |
| 013 | 36 | GTGTGTGGTTGTTAGGCCAGCTTGGCAC |
| 014 | 37 | CTGGCCTAACAACCACACACGGCACTTACC |
| 015 | 38 | GTGTGTGGTTTCATTCTCAAGCCTCAGACAGTG |
| 016 | 39 | TTGAGAATGAAACCACACACGGCACTTACC |
| 017 | 40 | GTGTGTGGTTTGGTTCAAAGTTTTTTTCTTCCATTTCAGG |
| 018 | 41 | CTTTGAACCAAACCACACACGGCACTTACC |
| 019 | 42 | CCGGAATTCTGACATTGATTATTGACTAGTTATTAATAGT AATCAATTACGG |
| 020 | 43 | CGCGGATCCCTGTGTTCTGGCGGCAAAC |
| 021 | 44 | CGCGGATCCCGGTTCACTAAACGAGCTCTGCTTATATAG |
| 022 | 45 | TGTAATTCTCCTTGGAATTTGCCCTTTTTG |
| 023 | 46 | CCCAAGCTTGGATCCTCACGACACCTGAAATG |
| 024 | 47 | CCTTTTTGAGTTTGGATCTTGGTTCATTCTCAAGCCTCAG ACAGTGGTTCAAAGTTTTTTTCTTCCATTTCAGGTGTCGT GAGGATCCA |
| 025 | 48 | AGCTTGGATCCTCACGACACCTGAAATGGAAGAAAAAAA CTTTGAACCACTGTCTGAGGCTTGAGAATGAACCAAGAT CCAAACTCAAAAAGG |
| 026 | 49 | CGGGGTACCTTCGCAACGGGTTTGCCG |
| 027 | 50 | CGCGGATCCTCACGACACCTG |
| 028 | 51 | TGAGGATCCGCCACCATGGAGTTTGG |
| 029 | 52 | CGCAAGCTTCAGTAGCGCTTTAGCCAGGAGACAAGCTCA GAGACTTCTG |
| 030 | 53 | GGAACCCCTAGTGATGGAGTT |
| 031 | 54 | CGGCCTCAGTGAGCGA |
| 032 | 55 | FAM-CACTCCCTCTCTGCGCGCTCG-BHQ1 |

Oligonucleotide sequences used in the present disclosure

### Experiment Results

### 1. Increased eGFP expression by EF-1α intron in combination with EF-1α promoter

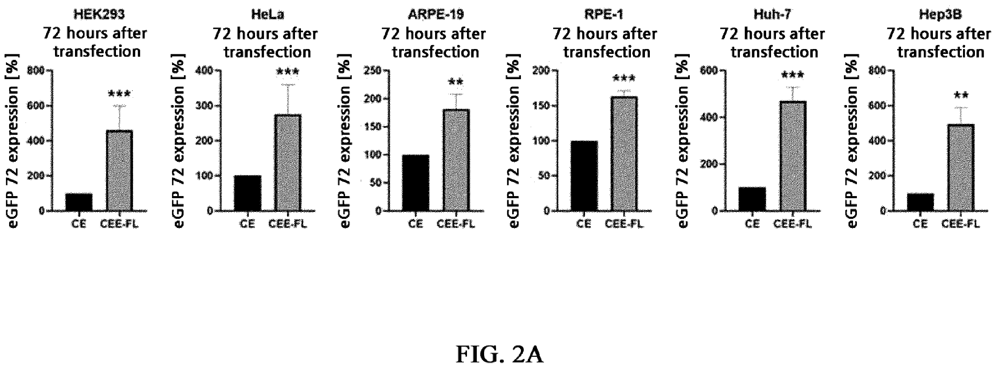

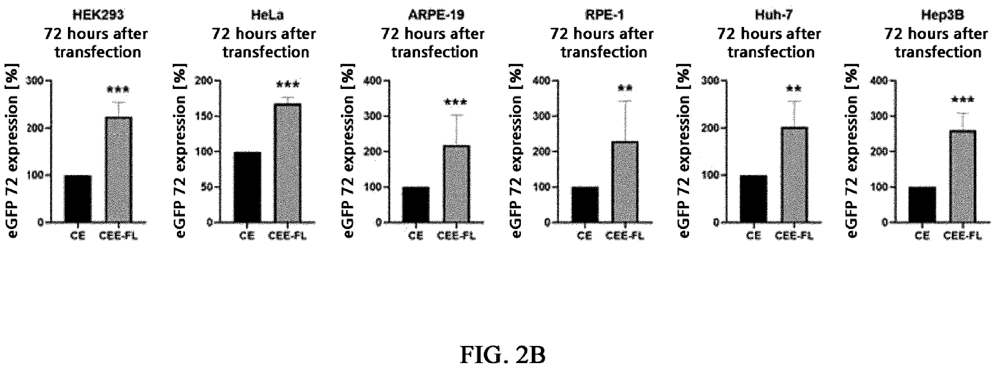

An experiment was conducted to test the influence of human elongation factor-1 alpha (EF-1α) intron on eGFP expression regulated by a combination of a cytomegalovirus (CMV) enhancer and an EF-1α promoter. First, a comparison was performed in the ITR-lacking animal cell expression constructs. As a result, the expression levels of eGFP in all cell lines used (HEK293, HeLa, ARPE-19, RPE-1, Huh-7, and Hep3B) were found to be increased (459.6%, 276.3%, 181.8%, 163.4%, 471.1%, and 494.3%) by EF-1α intron (FIG. 2A). The expression levels of eGFP in the ITR-containing pAAV constructs were found to be increased (224.0%, 167.7%, 218.7%, 229.5%, 202.5%, and 260.5%) by EF-1α intron (FIG. 2B). These results suggest that the combination of EF-1α intron with an EF-1α promoter can increase gene expression in various transgene expression constructs.

### 2. Increased eGFP expression by EF-1α intron in combination with various promoters

A test was conducted to observe whether EF-1α intron increased gene expression when combined with promoters other than EF-1α promoters. Specifically, a test was conducted to determine the influence of EF-1α intron on the expression of eGFP induced by a combination of a cytomegalovirus (CMV) enhancer and a CMV promoter. First, a comparison was performed in the ITR-lacking animal cell expression constructs. As a result, the expression levels of eGFP in all cell lines used (HEK293, HeLa, ARPE-19, RPE-1, Huh-7, and Hep3B) were found to be increased (284.3%, 464.0%, 217.5%, 180.4%, 405.7%, and 370.6%) by EF-1α intron (FIG. 3A).

Similarly, EF-1α intron increased the expression of eGFP regulated by a combination of a cytomegalovirus (CMV) enhancer and an EF-1α promoter in the ITR-containing pAAV constructs (241.4%, 494.8%, 266.8%, 185.5%, 415.5%, and 367.8%) (FIG. 3B).

Next, the influence of EF-1α intron on the expression of eGFP regulated by a combination of a CMV enhancer and a chicken β-actin promoter was determined. A comparison was performed in the animal cell expression constructs. As a result, the expression levels of eGFP in all cell lines used (HEK293, HeLa, ARPE-19, RPE-1, Huh-7, and Hep3B) were found to be increased (415.2%, 396.7%, 233.9%, 217.9%, 353.7%, and 297.7%) by EF-1α intron (FIG. 3C). These results suggest that EF-1α intron increases gene expression even when combined with promoters other than EF-1α promoters.

### 3. Determination of the shortest EF-1α intron fragment showing increasing effect on gene expression

### 3-A. Preparation of series of CEE constructs in which the sequence of EF-1α intron was sequentially truncated

A CEE-FL construct was designed to include CMV enhancer and CMV promoter portions (31 bp at the 5' end), an EF-1α promoter, 29 bp EF-1α exon 1, 924 bp EF-1α intron, and 9 bp EF-1α exon 2. A splicing donor was located at the 5' end of the core sequence associated with the splicing function of the intron and a splicing acceptor including the branch point site (BPS) was located at the 3' end of the core sequence. To find the shortest EF-1α intron sequence having the ability to increase gene expression, the splicing donor sequence at the 5' end of the intron was conserved and the downstream nucleotides were sequentially truncated. That is, CEE constructs were prepared in which up to 19 bp from the 5' end of EF-1α intron, which are assumed to be the splicing donor consensus sequence of EF-1α intron, were present in common and the downstream 659 bp (T2), 720 bp (T3), 807 bp (T3.1), 829 bp (T3.1.1), 851 bp (T3.1.2), 873 bp (T3.2), and 895 bp (T4) were truncated, as in Example 2-1 (FIG. 4A).

### 3-B. Increasing or decreasing effect of sequential truncation of nucleotides in the EF-1α intron sequence on gene expression

The expressions of eGFP in the eight constructs, *i.e.* the construct including the full-length EF-1α intron sequence (CEE-FL) and the constructs including sequentially truncated EF-1α intron sequences, were compared in five animal cell lines. First, the expressions of eGFP in the sequentially truncated constructs in HeLa, Hep3B, and Huh-7 cell lines were compared with those of the construct containing the full-length EF-1α intron A. As a result, the eGFP expressions were maintained in CEE-T2 to CEE-T3.1.2 but were drastically decreased in CEE-T3.2 and CEE-T4. In ARPE-19 and RPE-1 cell lines, the eGFP expressions in CEE-T2 were reduced by ~50% compared to those in CEE-FL, but the eGFP expressions in CEE-T3 to CEE-T3.1.2 were similar to or slightly higher than those in CEE-FL. As in the previous cell lines, the gene expressions in CEE-T3.2 and CEE-T4 were drastically decreased. The above results suggest that fragment T2 to T3.2 of EF-1α intron A are the intron A fragments with the function of increasing gene expression, specifically fragments from T3.2 (51 nucleotide lengths) to T3 (117 nucleotide lengths) (FIG. 4B).

### 4. Increased gene expression by EF-1α intron fragments T3.1.1 and T3.1.2

### 4-A. Construction of EF-1α intron fragments T3.1.1 and T3.1.2 in combination with chicken β-actin promoter

A CAE-FL construct was designed to include a CMV enhancer, a chicken β-actin promoter, 32 bp chicken β-actin exon 1, 29 bp EF-1α exon 1, 924 bp EF-1α intron A, and 9 bp EF-1α exon 2. CA refers to a construct in which all nucleotides of EF-1α intron were truncated. A CAE-T3.1.1 construct was the same as the CAE-FL construct, except that the 19 bp sequence and the T3.1.1 sequence were present at the 5' and 3' ends of EF-1α intron, respectively, and 829 bp between the 5' and 3' ends were truncated in the CAE-T3.1.1 construct. A CAE-T3.1.2 construct was the same as the CAE-FL construct, except that the 19 bp sequence and the T3.1.2 sequence were present at the 5' and 3' ends of EF-1α intron, respectively, and 851 bp between the 5' and 3' ends were truncated in the CAE-T3.1.1 construct (FIG. 5A).

### 4-B. Increased gene expression by EF-1α intron fragment T3.1.1 and T3.1.2 in combination with chicken β-actin promoter

The expressions of eGFP in CA in which all nucleotides of EF-1α intron were truncated and CAE-T3.1.1 and CAE-T3.1.2 in which the nucleotides of EF-1α intron were partially truncated were compared in two animal cell lines (HeLa, ARPE-19). The eGFP expressions in the CAE-T3.1.1 and CAE-T3.1.2 constructs were increased by 330.5% and 243.9% in HeLa and 170.8% and 165.9% in ARPE-19 compared to those in the CA construct (FIG. 5B). These results suggest that the fragments T3.1.1 and T3.1.2 of EF-1α intron can increase gene expression even when combined with a chicken β-actin promoter.

### 4-C. Preparation of constructs including EF-1α intron fragments T3.1.1 and T3.1.2 in combination with splicing donor of chicken β-actin

CA-T3.1.1 and CA-T3.1.2 constructs were designed to have hybrid intron structures including a 95 bp (T3.1.1) or 73 bp fragment (T3.1.2) at the 3' end of EF-1α intron and 9 bp EF-1α exon 2 in a state in which a CMV enhancer, a chicken β-actin promoter, 93 bp chicken β-actin exon 1, and 43 bp chicken β-actin intron were maintained. Portions of the chicken β-actin exon 1 and the chicken β-actin intron were estimated to serve as splicing donors. The EF-1α intron A fragments were estimated to serve as splicing receptors. A CA construct with no intron sequence was prepared and tested as a control (FIG. 5C).

### 4-D. Increased gene expression by intron hybridized with splicing donor fragment of chicken β-actin and fragments T3.1.1 and T3.1.2 at the 3' end of EF-1α intron A

After transduction of the CA, CA-T3.1.1, and CA-T3.1.2 constructs into HeLa and Hep3B cell lines, gene expression levels were compared. As a result, the gene expressions in CA-T3.1.1 and CA-T3.1.2 were increased (HeLa: 215.7%, 211.0%, Hep3B: 155.0%, 167.5%) compared to those in intron-lacking CA. These results suggest that the EF-1α intron fragments T3.1.1 and T3.1.2 can increase gene expression even when combined with splicing donors of other genes (FIG. 5D).

### 5. Delivery of TAFA4 gene using AAV

### 5-A. Evaluation of efficacy of TAFA4 in NaIO₃ (sodium iodate)-induced retinal damage model

To evaluate the efficacy of TAFA4 in alleviating retinal damage, the change in fluorescence angiography (FA) and electoretinogrphy (ERG) following administration of AAV8.mouse TAFA4 was evaluated in a NaIO₃-induced retinal damage mouse model.

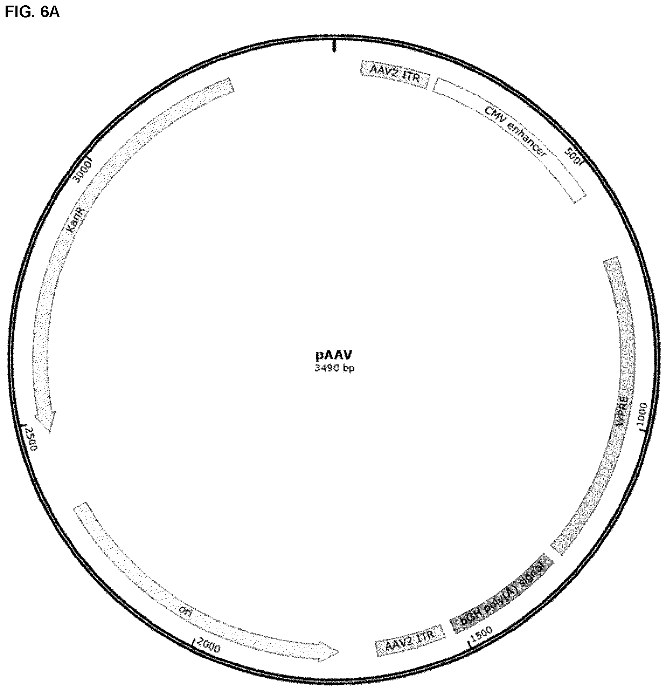

Control AAV8 or AAV8.mouse TAFA4 was administered subretinally (SRI) to C57BL/6 mice (OrientBio). The control AAV8 was prepared using the pAAV construct of FIG. 6A, and the AAV8.mouse TAFA4 was prepared using the pAAV-mTAFA4 construct of FIG. 6B (Example 2-6-1).

After waiting for up to 56 days for sufficient expression of mouse TAFA4 following subretinal injection (SRI) of 5×10⁸ vg of AAV8 into both eyes of mice, 20 mg/kg of NaIO₃ was administered into the tail vein to induce an AMD model. On the 10 days after the model induction (on the 66 days after the SRI administration), a fluorescence contrast agent was injected via the tail vein. Afterwards, the fluorescence angiography (FA) of the fundus was imaged with a Micron-IV imaging camera (Phoenix) On the 11 day after the model induction (on the 67 days after SRI administration), scotopic ERG evaluation was performed. The mouse was placed on ERG stage and theprobes of ERG were contacted to the tail, head and cornea, respectively. Then, A-wave and B-wave amplitudes were measured. ERG analysis was performed using the LabScribeERG (iWorx Data Acquisition software) program.

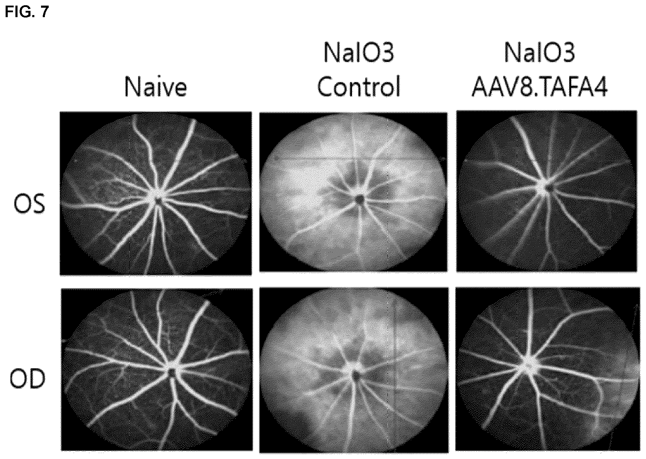

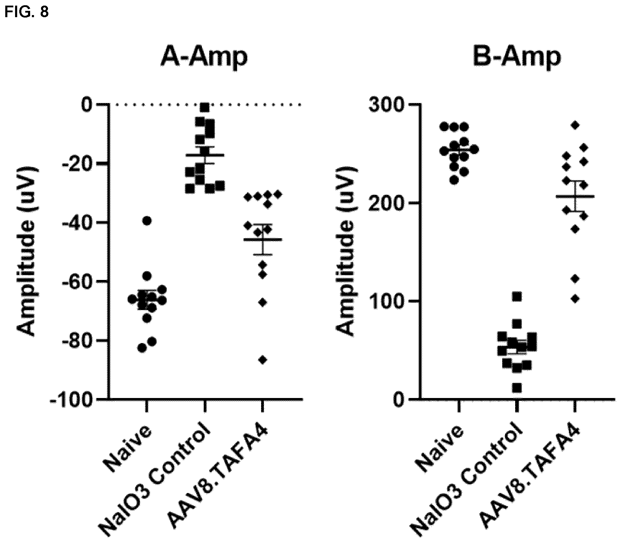

On day 10 after the model induction, the leakage of the fluorescence contrast agent due to damage to the outer retina was observed in the retina of the NaIO₃-administered group (control group, control-AAV8) on the FA image, unlike in the healthy (non-AMD-induced) animals (naive and control-AAV8). On the other hand, the group administered with AAV8.mouse TAFA4 (test group) showed nearly normal retinal status, similarly to the naive group (FIG. 7). The scotopic ERG result assessed on day 11 after the model induction was also similar to FA. Both A-wave and B-wave amplitudes were reduced significantly. On the other hand, the group administered with AAV8.mouse TAFA4 (test group) showed recovery of both A-wave and B-wave amplitudes. In particular, the B-wave amplitude was recovered to a nearly normal level (FIG. 8).

### 5-B. Analysis of TAFA4 mRNA expression in NaIO₃ (sodium iodate)-induced retinal damage model

On day 56 after subretinal administration of control AAV8 or AAV8.mouse TAFA4 to C57BL/6 mice, a retinal damage model was induced by administering 20 mg/kg of NaIO₃ via the tail vein. On day 14 after the model induction (70 days after the SRI administration), the eyes of the mice were extracted. RNA was extracted from the extracted eye using a RNeasy Mini kit (Qiagen). The extracted RNA was synthesized into cDNA using a Primescript 1st strand cDNA synthesis kit (Takara), and then qPCR was performed with the cDNA as a template to evaluate the mRNA level of TAFA4. The TAFA4 mRNA level was expressed as a relative quantity to GAPDH mRNA using ΔΔCq values.

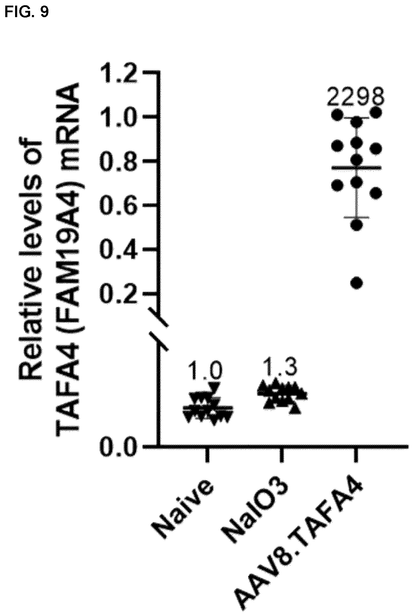

In the NaIO₃-treated group (control group, control-AAV8), the mouse TAFA4 mRNA level was hardly changed regardless of retinal damage as compared to the naive group. However, in the AAV8.mouse TAFA4-administered group (test group), the mouse TAFA4 mRNA level was significantly higher as compared to the unadministered group, and it was found that retinal damage was alleviated by the expressed mouse TAFA4 (FIG. 9).

### 5-C. Comparison of interspecies sequence identity of TAFA4

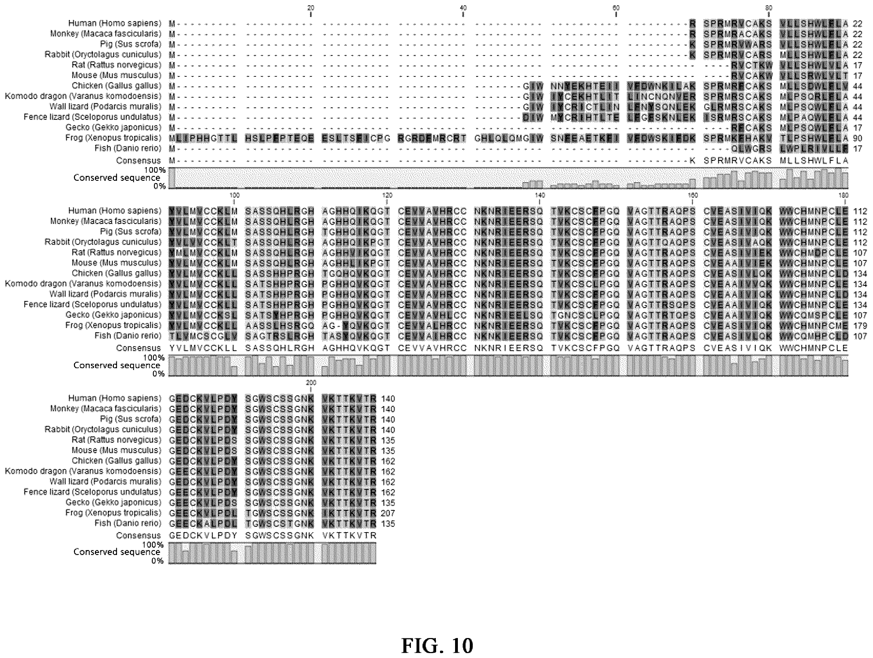

The sequence identities of the full-length TAFA4 protein in mammals (human, monkey, pig, rabbit, rat and mouse), birds (chicken), reptiles (Komodo dragon, wall lizard, Fence lizard and gecko), amphibians (frog) and fish were compared and the consensus sequence between each species was investigated (FIG. 10). The amino acid sequence of the TAFA4 protein in mature human (95 a.a.) showed sequence identity of 90% or higher as compared to mammals and amphibians, 95% or higher as compared to birds, and 85% or higher as compared to reptiles and fish. The sequence identity was very high regardless of species (FIG. 19).

### 5-D. Protective Effect of TAFA4 homology on retina damage in NaIO₃ (sodium iodate)-induced retinal damage model

Since the AAV8.mouse TAFA4 was found to have a protective effect against retinal damage in the NaIO₃-induced retinal damage model, experiment was conducted to investigate whether TAFA4 from other species also has a protective effect against retinal damage. Experiment was conducted to confirm the efficacy of human TAFA4, as well as reptile TAFA4 (gecko TAFA4; sequence identity 87.4%) and fish TAFA4 (sequence identity 86.3%), which show the lowest sequence identity with human TAFA4. The effect of administration of AAV8.human TAFA4, AAV8.gecko TAFA4, and AAV8.fish TAFA4 on the change in fluorescence angiography (FA) and electroretinography (ERG) was evaluated in a NaIO₃-induced AMD mouse model.

Control AAV8 or AAV8.TAFA4 of each species was administered to C57BL/6 mice (OrientBio) by subretinal injection (SRI). The AAV8 and AAV8.TAFA4 of each species used in the experiment were prepared in the same way as described in 5-A.

AAV8.human TAFA4 was prepared using a pAAV-human TAFA4 construct (Example 2-6-2), AAV8.gecko TAFA4 was prepared using pAAV-gecko TAFA4 construct (Example 2-6-3), and AAV8.fish TAFA4 was prepared using a pAAV-fish TAFA4 construct (Example 2-6-4).

After waiting for up to 42 days for sufficient expression of TAFA4 after subretinal injection (SRI) of 1x10⁹ vg of AAV8 into both eyes of mice, 20 mg/kg of NaIO₃ was administered into the tail vein to induce an AMD model. On day 9 after the model induction (51 days after SRI administration), a fluorescence contrast agent was injected via the tail vein. Afterwards, the fundus was imaged with a Micron-IV imaging camera (Phoenix) by fluorescence angiography (FA). On day 7 after the model induction (49 days after SRI administration), scotopic ERG evaluation was performed. The mouse was placed on ERG stage and an ERG probes were contacted with the tail, head and cornea, respectively. Then, A-wave and B-wave amplitudes were measured. ERG analysis was performed using the LabScribeERG (iWorx Data Acquisition software) program.

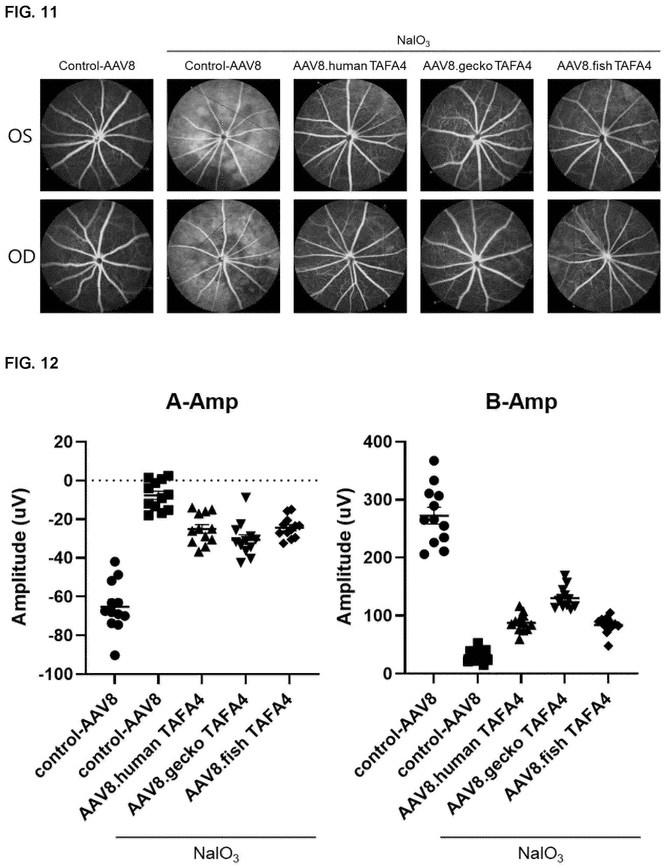

On day 9 after the model induction, the leakage of the fluorescence contrast agent due to damage to the outer retina was observed in the retina of the NaIO₃-administered group (control group, control-AAV8) on the FA image, unlike the healthy animals (naïve and control-AAV8). On the other hand, the groups administered with AAV8.human TAFA4, AAV8.gecko TAFA4 and AAV8.fish TAFA4 (test groups) showed nearly normal retina as the naive group (FIG. 11). As a result of the scotopic ERG result assessed on day 11 after the model induction, the A-wave and B-wave amplitudes were significantly reduced by NaIO₃. On the other hand, the A-wave and B-wave amplitudes were recovered in the groups administered with AAV8.human TAFA4, AAV8.gecko TAFA4 and AAV8.fish TAFA4 (test groups) (FIG. 12).

### 5-F. Comparison of TAFA4 expression depending on difference in signal peptides

In order to confirm the normal expression of 95 amino acid sequences expected to be mature proteins, the expression of human TAFA4 protein substituted of signal sequences was investigated. For this purpose, a full-length TAFA4 construct, a construct in which 45 amino acid sequences at the N-terminal were replaced by the signal sequence of mIgG heavy chain variable region 3 (MGWSCIILFLVATATGVHS, SEQ ID NO 312), and a construct in which 45 amino acid sequences at the terminal were replaced by the signal sequence of human interleukin 2 (IL2) (MYRMQLLSCIALSLALVTNS, SEQ ID NO 313) were cloned into a pcDNA3.1(-) vector. HEK293 cells were seeded in a 12-well plate at 6×10⁵ cells/wells and incubated for 18 hours. The above constructs and pCMV DsRED-Express2 (TAKARA) were transduced together using Lipofectamin 3000 (Invitrogen). After 72 hours of incubation, the cells were harvested and RNA was extracted using an RNeasy Mini kit (Qiagen). The extracted RNA was synthesized into cDNA using a Primescript 1st strand cDNA synthesis kit (Takara), and then qPCR was performed with the cDNA as a template to investigate the mRNA level of TAFA4. The TAFA4 mRNA level was calculated as a relative quantity to GAPDH mRNA using the ΔΔCq value, and then divided by the proportion of cells expressing DsRed. To determine the amount of TAFA4 protein, the culture medium was collected. The supernatant was separated by centrifugation at 12000 rpm and was analyzed using a human TAFA4 ELISA kit (Abbexa) according to the method provided by the manufacturer. The calculated values were compared by dividing by the proportion of DsRed-expressing cells.

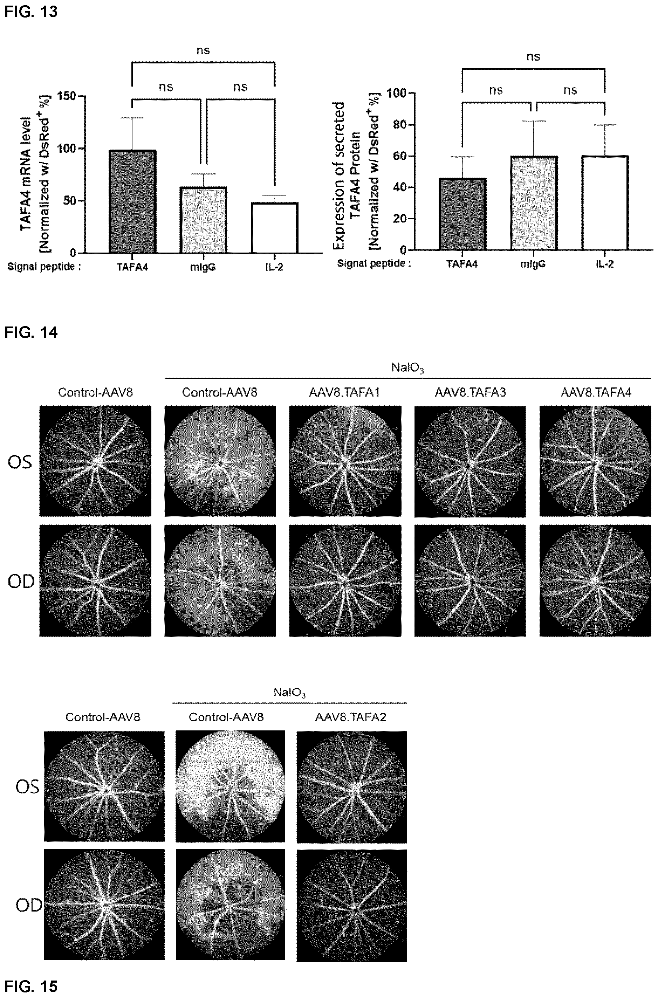

As a result, there was no significant change in mRNA expression and protein expression depending on the signal sequences (p > 0.05) (FIG. 13).

### 6. Delivery of TAFA1-3 gene using AAV

### 6-A. Protective Effect of TAFA4 paralogs based on sequence identity in NaIO₃ (sodium iodate)-induced retinal damage model

It is known that there is a high level of sequence identity between TAFA4 and TAFA protein families. Therefore, it was investigated whether human TAFA1, TAFA2 and TAFA3 also exhibit retina-protecting effect in a NaIO₃-induced retinal injury mouse model. The change in fluorescence angiography (FA) and electroretinography (ERG) was evaluated after administration of AAV8.TAFA1, AAV8.TAFA2, AAV8.TAFA3 and AAV8.TAFA4 to a NaIO₃-induced AMD mouse model.

Control AAV8, AAV8.TAFA1, AAV8.TAFA2, AAV8.TAFA3 or AAV8.TAFA4 was administered to C57BL/6 mice (OrientBio) by subretinal injection (SRI). AAV8, AAV8.TAFA1, AAV8.TAFA2, AAV8.TAFA3 and AAV8.TAFA4 used in the experiment were prepared using the same method as described above in 5-A.

AAV8.human TAFA1 was prepared using a pAAV- human TAFA1 construct (Example 2-6-5), AAV8.human TAFA2 using a pAAV- human TAFA2 construct (Example 2-6-6), AAV8.human TAFA3 using a human TAFA1 construct (Example 2-6-7), and AAV8.human TAFA4 using a human TAFA1 construct (Example 2-6-2).

In order to investigate the effect of protecting the retina of TAFA1, TAFA3 and TAFA4, after waiting for up to 42 days following subretinal injection (SRI) of 1x10⁹ vg of AAV8 into both eyes of mice, 20 mg/kg of NaIO₃ was administered into the tail vein to induce an AMD model. On day 9 after the model induction (51 days after SRI administration), a fluorescence contrast agent was injected via the tail vein. Afterwards, the fundus was imaged with a Micron-IV imaging camera (Phoenix) by fluorescence angiography (FA). On day 7 after the model induction (49 days after SRI administration), scotopic ERG evaluation was performed. The mouse was placed on ERG stage and an ERG probe was brought in contact with the tail, head and cornea, respectively. Then, A-wave and B-wave amplitudes were measured. ERG analysis was performed using the LabScribeERG (iWorx Data Acquisition software) program.

In order to investigate the effect of protecting the retina of TAFA2, after waiting for up to 56 days following subretinal injection (SRI) of 1x10⁹ vg of AAV8 into both eyes of mice, 20 mg/kg of NaIO₃ was administered into the tail vein to induce an AMD model. On day 9 after the model induction (65 days after the SRI administration), a fluorescence contrast agent was injected via the tail vein. Afterwards, the fundus was imaged with a Micron-IV imaging camera (Phoenix) by fluorescence angiography (FA). On day 7 after the model induction (63 days after the SRI administration), scotopic ERG assessment was performed. The mouse was placed on ERG stage and an ERG probe was brought in contact with the tail, head and cornea, respectively. Then, A-wave and B-wave amplitudes were measured. ERG analysis was performed using the LabScribeERG (iWorx Data Acquisition software) program.

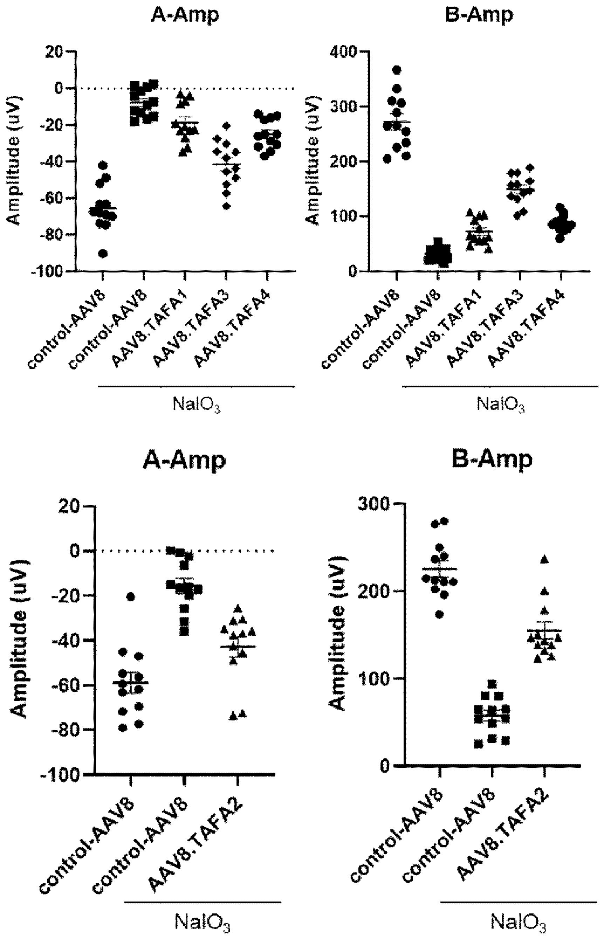

The leakage of the fluorescence contrast agent due to damage to the outer retina was observed in the retina of the NaIO₃-administered group (control group, control-AAV8) on the FA image, unlike the healthy animals (naïve and control-AAV8). On the other hand, the groups administered with AAV8.TAFA1, AAV8.TAFA2, AAV8.TAFA3 or AAV8.TAFA4 (test groups) showed nearly normal retina as the naive group (FIG. 14). The scotopic ERG result also showed that the A-wave and B-wave amplitudes were significantly reduced by NaIO₃. On the contrary, the groups to which AAV8.TAFA1, AAV8.TAFA2, AAV8.TAFA3 or AAV8.TAFA4 was administered (test groups) showed recovery of the change of A-wave and B-wave amplitudes induced by NaIO₃ (FIG. 15).

### 6-B. Comparison of sequence identity between human TAFA4 and TAFA paralogs of different species

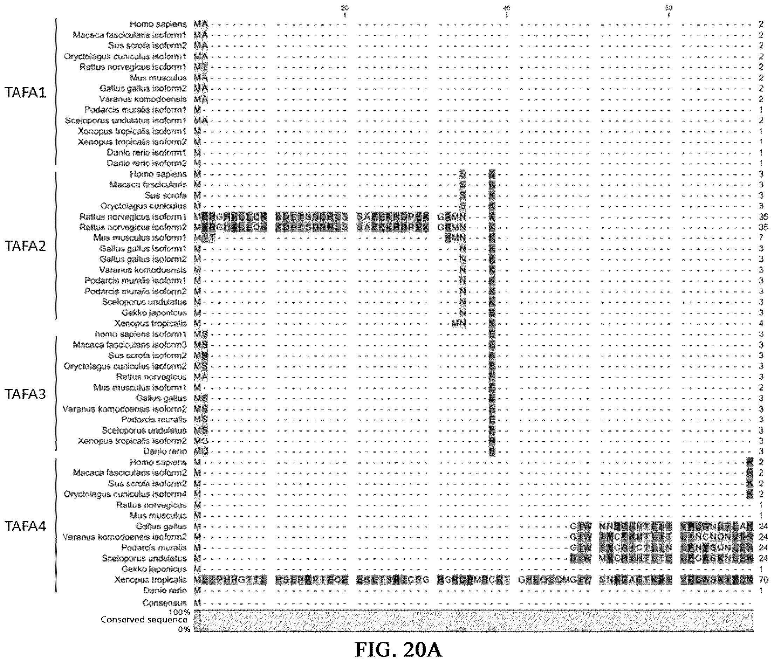

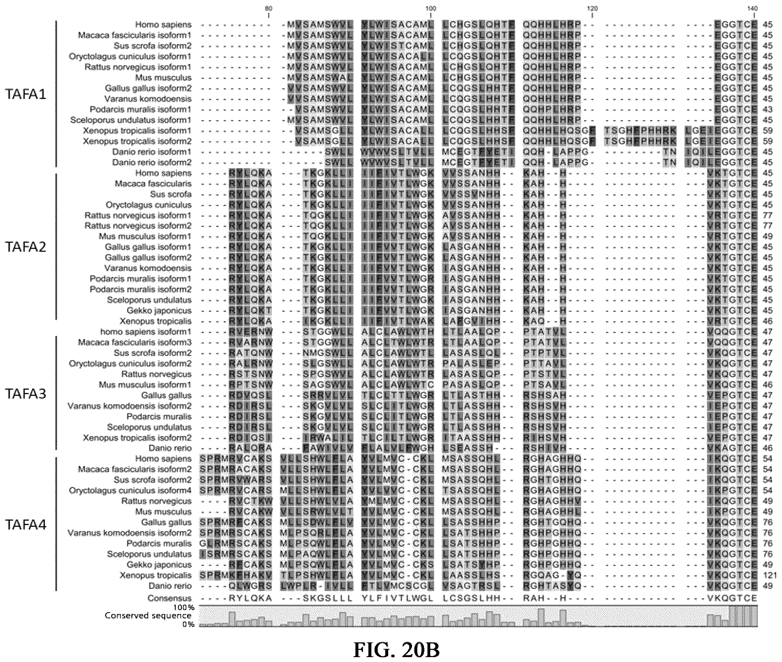

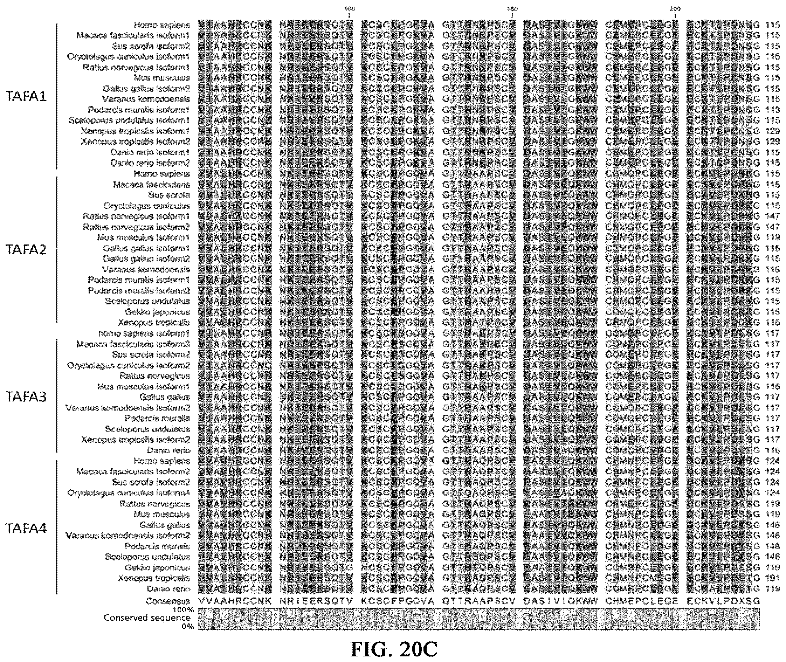

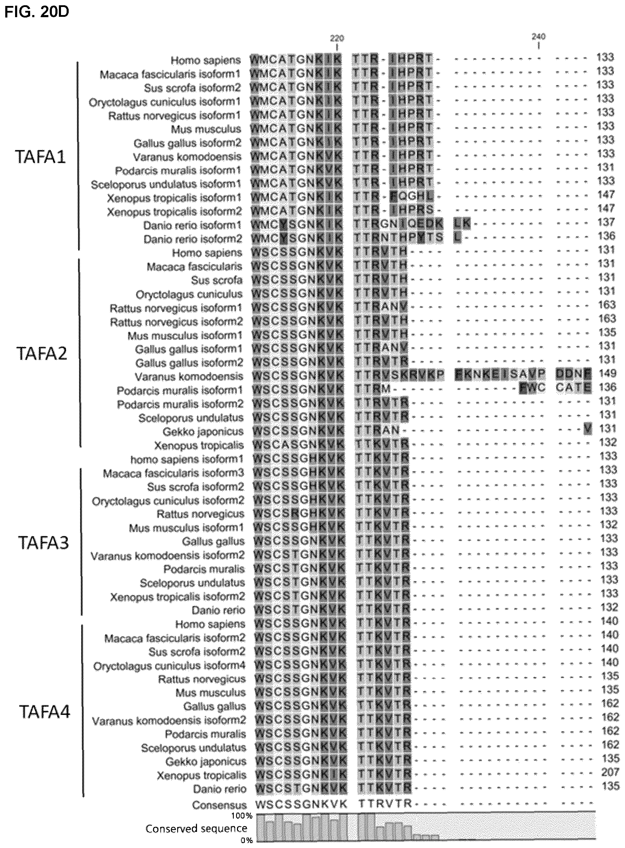

The retina-protecting effect of human TAFA1, human TAFA2, human TAFA3 and human TAFA4, as well as gecko TAFA4 and fish TAFA4, was confirmed in the NaIO₃-induced retinal damage model. Therefore, it was thought that the consensus sequence of the various TAFA proteins would have a retina-protecting effect, and the sequence identity of full-length TAFA1, full-length TAFA2, full-length TAFA3 and full-length TAFA4 proteins of mammals (human, monkey, pig, rabbit, rat and mouse), birds (chicken), reptiles (Komodo dragon, wall lizard, fence lizard and gecko), amphibians (frog) and fish was compared to identify the consensus sequence. The amino acid sequences of full-length TAFA1 to TAFA4 of vertebrates are shown in FIG. 20A to FIG. 20D. FIG. 16 shows a result of comparing the interspecies sequence identity of mature TAFA1 protein, FIG. 17 shows a result of comparing the interspecies sequence identity of mature TAFA2 protein, FIG. 18 shows a result of comparing the interspecies sequence identity of mature TAFA3 protein, and FIG. 19 shows a result of comparing the interspecies sequence identity of mature TAFA4 protein.

The 93 sequences of human TAFA4 (IKQGTCEVVAVHRCCNKNRIEERSQTVKCSCFPGQVAGTTRAQPSCVEASIVIQKWWCHM NPCLEGEDCKVLPDYSGWSCSSGNKVKTTKVTR, SEQ ID NO 75) were found to have very high sequence identity with the TAFA4 sequence of other species and the consensus sequence of TAFA1 to TAFA3 in human and fish. The sequence identity of the consensus sequence is shown in Table 2.

**[Table 2]**

| Sequence identity of consensus sequence of TAFA1, TAFA2, TAFA3 and TAFA4 of different species and human TAFA4 (93 a.a.) | | | | | |
|---|---|---|---|---|---|
| TAFA family | Species | Scientific name | Length | SEQ ID NO | Sequence identity |
| TAFA4 | Human | *Homo sapiens* | 93 | 75 | 100.0% |
| TAFA1 | Human | *Homo sapiens* | 91 | 76 | 75.8% |
| TAFA1 | Monkey | *Macaca* | 91 | 77 | 75.8% |
| | | *fascicularis*_*isoform1* | | | |
| TAFA1 | Pig | *Sus scrofa*_*isoform2* | 91 | 78 | 75.8% |
| TAFA1 | Rabbit | *Oryctolagus cuniculus*_*isoform1* | 91 | 79 | 75.8% |
| TAFA1 | Rat | *Rattus norvegicus*_*isoform1* | 91 | 80 | 75.8% |
| TAFA1 | Mouse | *Mus musculus* | 91 | 81 | 75.8% |
| TAFA1 | Chicken | *Gallus gallus_isoform2* | 91 | 82 | 75.8% |
| TAFA1 | Komodo dragon | *Varanus komodoensis* | 91 | 83 | 76.9% |
| TAFA1 | Wall lizard | *Podarcis muralis*_*isoform1* | 91 | 84 | 76.9% |
| TAFA1 | Fence lizard | *Sceloporus undulatus*_*isoform1* | 91 | 85 | 76.9% |
| TAFA1 | Frog | *Xenopus tropicalis*_*isoform1* | 92 | 86 | 76.1% |
| TAFA1 | Frog | *Xenopus tropicalis_isoform2* | 92 | 87 | 76.1% |
| TAFA1 | Fish | *Danio rerio_isoform1* | 93 | 88 | 74.2% |
| TAFA1 | Fish | *Danio rerio_isoform2* | 93 | 89 | 75.3% |
| TAFA2 | Human | *Homo sapiens* | 93 | 90 | 86.0% |
| TAFA2 | Monkey | *Macaca fascicularis* | 93 | 91 | 86.0% |
| TAFA2 | Pig | *Sus scrofa* | 93 | 92 | 86.0% |
| TAFA2 | Rabbit | *Oryctolagus cuniculus* | 93 | 93 | 86.0% |
| TAFA2 | Rat | *Rattus norvegicus_isoform1* | 93 | 94 | 82.8% |
| TAFA2 | Rat | *Rattus norvegicus_isoform2* | 93 | 95 | 84.9% |
| TAFA2 | Mouse | *Mus musculus_isoform1* | 93 | 96 | 84.9% |
| TAFA2 | Chicken | *Gallus gallus_isoform1* | 93 | 97 | 83.9% |
| TAFA2 | Chicken | *Gallus gallus_isoform2* | 93 | 98 | 87.1% |
| TAFA2 | Komodo dragon | *Varanus komodoensis* | 93 | 99 | 84.9% |
| TAFA2 | Wall lizard | *Podarcis muralis* | 91 | 100 | 85.7% |
| TAFA2 | Wall lizard | *Podarcis muralis 2* | 93 | 101 | 87.1% |
| TAFA2 | Fence lizard | *Sceloporus undulatus* | 93 | 102 | 87.1% |
| TAFA2 | Gecko | *Gekko japonicus* | 92 | 103 | 83.7% |
| TAFA2 | Frog | *Xenopus tropicalis* | 93 | 104 | 84.9% |
| TAFA2 | Fish | *Danio rerio* | 93 | 105 | 82.8% |
| TAFA3 | Human | *Homo sapiens_isoform1* | 93 | 106 | 82.8% |
| TAFA3 | Monkey | *Macaca fascicularis_isoform3* | 93 | 107 | 82.8% |
| TAFA3 | Pig | *Sus scrofa*_*isoform2* | 93 | 108 | 84.9% |
| TAFA3 | Rabbit | *Oryctolagus cuniculus_isoform2* | 93 | 109 | 82.8% |
| TAFA3 | Rat | *Rattus norvegicus* | 93 | 110 | 82.8% |
| TAFA3 | Mouse | *Mus musculus_isoform1* | 93 | 111 | 83.9% |
| TAFA3 | Chicken | *Gallus gallus* | 93 | 112 | 84.9% |
| TAFA3 | Komodo dragon | *Varanus komodoensis_isoform2* | 93 | 113 | 86.0% |
| TAFA3 | Wall lizard | *Podarcis muralis* | 93 | 114 | 83.9% |
| TAFA3 | Fencee lizard | *Sceloporus undulatus* | 93 | 115 | 86.0% |
| TAFA3 | Frog | *Xenopus tropicalis_isoform2* | 93 | 116 | 87.1% |
| TAFA3 | Fish | *Danio rerio* | 93 | 117 | 81.7% |
| TAFA4 | Monkey | *Macaca fascicularis_isoform2* | 93 | 118 | 100.0% |
| TAFA4 | Pig | *Sus scrofa*_*isoform2* | 93 | 119 | 100.0% |
| TAFA4 | Rabbit | *Oryctolagus cuniculus_isoform4* | 93 | 120 | 96.8% |
| TAFA4 | Rat | *Rattus norvegicus* | 93 | 121 | 96.8% |
| TAFA4 | Mouse | *Mus musculus* | 93 | 122 | 95.7% |
| TAFA4 | Chicken | *Gallus gallus* | 93 | 123 | 96.8% |
| TAFA4 | Komodo dragon | *Varanus komodoensis_isoform2* | 93 | 124 | 94.6% |
| TAFA4 | Wall lizard | *Podarcis muralis* | 93 | 125 | 96.8% |
| TAFA4 | Fence lizard | *Sceloporus undulatus* | 93 | 126 | 95.7% |
| TAFA4 | Gecko | *Gekko japonicus* | 93 | 127 | 87.1% |
| TAFA4 | Frog | *Xenopus tropicalis* | 93 | 128 | 92.5% |
| TAFA4 | Fish | *Danio rerio* | 93 | 129 | 87.1% |

Clustal Omega was used to check sequence identity.

All publications, patents, patent applications and other documents cited in this application are hereby incorporated by reference in their entireties for all purposes to the same extent as if each individual publication, patent, patent application or other document were individually indicated to be incorporated by reference for all purposes.

Although the present disclosure has been described herein with reference to the foregoing aspects, those skilled in the art will appreciate that various changes and modifications are possible by addition, modification, deletion or insertion of the elements without departing from the spirit of the present disclosure as disclosed in the accompanying claims. It is to be understood that such changes and modifications are within the scope of the present disclosure.

## Claims

1. A pharmaceutical composition for use in preventing or treating retinal or macular diseases, comprising a polypeptide comprising an amino acid sequence with at least 70% sequence identity with an amino acid sequence represented by SEQ ID NO 75, a nucleic acid encoding the polypeptide, a vector comprising the nucleic acid, a recombinant virus particle comprising the vector and capsid protein, or a cell comprising the nucleic acid or vector.

2. The pharmaceutical composition according to claim 1, wherein the amino acid sequence with at least 70% sequence identity with the amino acid sequence represented by SEQ ID NO 75 comprises an amino acid sequence represented by General Formula 1 (from N-terminal to C-terminal):
<General Formula 1> X1-X2-X3-G-T-C-E-V-X4-A-X5-H-X6-C-C-N-X7-N-X8-I-E-E-X9-S-Q-T-X10-X11-C-S-C-X12-X13-G-X14-V-A-G-T-T-X15-X16-X17-P-S-C-V-X18-A-X19-I-V-X20-X21-X22-W-W-C-X23-M-X24-P-C-X25-X26-G-E-X27-C-K-X28-L-P-D-X29-X30-G-W-X31-C-X32-X33-G-X34-K-X35-K-T-T-X36-X37-X38-X39
wherein
X1 is nonexistent, V, I or L,
X2 is K, E, R or Q,
X3 is G, T, Q, P or A,
X4 is V or I,
X5 is A, L, V or I,
X6 is R or L,
X7 is K, R or Q,
X8 is R or K,
X9 is R or L,
X10 is V or G,
X11 is K or N,
X12 is F or L,
X13 is P or S,
X14 is Q or K,
X15 is R, H or Q,
X16 is A, N, S or T,
X17 is A, Q, R, K or T,
X18 is D or E,
X19 is S or A,
X20 is I, E, L, A or V,
X21 is Q, G or E,
X22 is K or R,
X23 is H, Q or E,
X24 is E, Q, N, D, S or H,
X25 is L, V or M,
X26 is E, D, P, L or A,
X27 is E or D,
X28 is V, T, A or I,
X29 is L, N, R, Y, S or Q,
X30 is S, K or T,
X31 is S or M,
X32 is S, A or Y,
X33 is S, T or R,
X34 is N or H,
X35 is V or I,
X36 is R or K,
X37 is nonexistent, V, A, G, M or N,
X38 is nonexistent, T, I, N, F or S, and
X39 is nonexistent, R, H, V, K, I or Q.

3. The pharmaceutical composition according to claim 1, wherein the polypeptide further comprises an amino acid sequence represented by General Formula 2 (from N-terminal to C-terminal):
<General Formula 2> X1-X2-H-H-K-A-X3-H
wherein
X1 is A or V,
X2 is N or I, and
X3 is H or Q.

4. The pharmaceutical composition according to claim 1, wherein the polypeptide further comprises an amino acid sequence represented by General Formula 3 (from N-terminal to C-terminal):
<General Formula 3> X1-X2-X3-X4-X5-X6-X7-X8-X9-X10
wherein
X1 is A or S,
X2 is L, T or S,
X3 is Q, E or H,
X4 is nonexistent, P, L or H,
X5 is P or R,
X6 is T, S or I,
X7 is A, P, T, S or H,
X8 is T, A, S or I,
X9 is V or A, and
X10 is L or H.

5. The pharmaceutical composition according to claim 1, wherein the polypeptide further comprises amino acid sequence represented by General Formula 4 (from N-terminal to C-terminal):
<General Formula 4> X1-X2
wherein
X1 is H or Y, and
X2 is Q, V or L.

6. The pharmaceutical composition according to claim 1, wherein the polypeptide further comprises the following amino acid sequence (from N-terminal to C-terminal):
LHRP (SEQ ID NO 293);
LHQSGFTSGHFPHHRKLGE (SEQ ID NO 294); or
LAPPGTNIQI (SEQ ID NO 295)

7. The pharmaceutical composition according to claim 1, wherein the polypeptide further comprises an amino acid sequence represented by General Formula 5 (from N-terminal to C-terminal):
<General Formula 5> X1-X2-X3
wherein
X1 is P or G,
X2 is R or H, and
X3 is T, S or L.

8. The pharmaceutical composition according to claim 1, wherein the polypeptide further comprises the following amino acid sequence (from N-terminal to C-terminal):
PYTSL (SEQ ID NO 296); or
QEDKLK (SEQ ID NO 297).

9. The pharmaceutical composition according to claim 1, wherein the polypeptide comprises one or more amino acid sequence selected from a group consisting of amino acid sequences represented by SEQ ID NOS 75 to 129.

10. The pharmaceutical composition according to claim 1, wherein the nucleic acid encoding the polypeptide is one or more nucleotide sequence selected from a group consisting of nucleotide sequences represented by SEQ ID NOS 184 to 238.

11. The pharmaceutical composition according to claim 1, wherein the polypeptide comprises one or more amino acid sequence selected from a group consisting of amino acid sequences represented by SEQ ID NOS 130 to 183.

12. The pharmaceutical composition according to claim 1, wherein the nucleic acid encoding the polypeptide is one or more nucleotide sequence selected from a group consisting of nucleotide sequences represented by SEQ ID NOS 239 to 292.

13. The pharmaceutical composition according to claim 1, wherein the vector is a viral vector.

14. The pharmaceutical composition according to claim 1 or 13, wherein the virus is an adeno-associated virus (AAV).

15. The pharmaceutical composition according to claim 14, wherein the serotype of the AAV is AAV1, AAV2, AAV3, AAV4, AAV5, AAV6, AAV7, AAV8, AAV9 or AAVrh10.

16. The pharmaceutical composition according to claim 1, wherein the retinal or macular disease comprises diabetic retinopathy, choroidal neovascularization, maculopathy, macular degeneration, retinal degeneration, macular edema, retinal edema, macular swelling, retinal cell degeneration, retinal vascular occlusion, retinal detachment, inherited retinal disease, or a combination thereof.

17. The pharmaceutical composition according to claim 16, wherein the macular degeneration is age-related macular degeneration.

18. The pharmaceutical composition according to claim 17, wherein the age-related macular degeneration is wet form or dry form.

19. An adeno-associated virus (AAV) vector comprising a nucleic acid encoding a polypeptide comprising an amino acid sequence with at least 70% sequence identity with an amino acid sequence represented by SEQ ID NO 75.

20. The AAV vector according to claim 19, wherein the vector further comprises one or more sequence selected from a group consisting of a promoter sequence, an enhancer sequence, an exon sequence, an intron sequence, a signal sequence-coding sequence, a splicing donor sequence, and one or more adeno-associated viral inverted terminal repeat (ITR) sequence.

21. The AAV vector according to claim 20, wherein the intron consists of 51 to 117 nucleotides and comprises a nucleotide sequence represented by SEQ ID NO 57.

22. The AAV vector according to claim 20, wherein the intron has at least 70% sequence identity with (i) the 871st to 924th nucleotides of SEQ ID NO 1 (SEQ ID NO 58), (ii) the 861st to 924th nucleotides of SEQ ID NO 1 (SEQ ID NO 59), (iii) the 852nd to 924th nucleotides of SEQ ID NO 1 (SEQ ID NO 3), (iv) the 851st to 924th nucleotides of SEQ ID NO 1 (SEQ ID NO 61), (v) the 830th to 924th nucleotides of SEQ ID NO 1 (SEQ ID NO 2), (vi) the 821st to 924th nucleotides of SEQ ID NO 1 (SEQ ID NO 63), (vii) the 811th to 924th nucleotides of SEQ ID NO 1 (SEQ ID NO 64), or (viii) the 808th to 924th nucleotides of SEQ ID NO 1 (SEQ ID NO 65).

23. The AAV vector according to claim 22, wherein the intron comprises (i) the 871st to 924th nucleotides of SEQ ID NO 1 (SEQ ID NO 58), (ii) the 861st to 924th nucleotides of SEQ ID NO 1 (SEQ ID NO 59), (iii) the 852nd to 924th nucleotides of SEQ ID NO 1 (SEQ ID NO 3), (iv) the 851st to 924th nucleotides of SEQ ID NO 1 (SEQ ID NO 61), (v) the 830th to 924th nucleotides of SEQ ID NO 1 (SEQ ID NO 2), (vi) the 821st to 924th nucleotides of SEQ ID NO 1 (SEQ ID NO 63), (vii) the 811th to 924th nucleotides of SEQ ID NO 1 (SEQ ID NO 64), or (viii) the 808th to 924th nucleotides of SEQ ID NO 1 (SEQ ID NO 65).

24. The AAV vector according to claim 20, wherein the vector comprises:
(1) a CMV enhancer sequence represented by SEQ ID NO 4;
(2) a promoter sequence selected from a CMV promoter sequence represented by SEQ ID NO 5 or 6, an EF-1α promoter sequence represented by SEQ ID NO 7, or a chicken β actin promoter represented by SEQ ID NO 8;
(3) an exon 1 (E1) sequence selected from a CMV E1 sequence represented by SEQ ID NO 12, an EF-1α E1 sequence represented by SEQ ID NO 13, or a chicken β actin E1 sequence represented by SEQ ID NO 14 or 15;
(4) a splicing donor sequence represented by SEQ ID NO 9 or 10; and/or
(5) an EF-1α E2 sequence represented by SEQ ID NO 11.

25. The AAV vector according to claim 19, wherein the amino acid sequence with at least 70% sequence identity to the amino acid sequence represented by SEQ ID NO 75 comprises an amino acid sequence of General Formula 1 (from N-terminal to C-terminal):
<General Formula 1> X1-X2-X3-G-T-C-E-V-X4-A-X5-H-X6-C-C-N-X7-N-X8-I-E-E-X9-S-Q-T-X10-X11-C-S-C-X12-X13-G-X14-V-A-G-T-T-X15-X16-X17-P-S-C-V-X18-A-X19-I-V-X20-X21-X22-W-W-C-X23-M-X24-P-C-X25-X26-G-E-X27-C-K-X28-L-P-D-X29-X30-G-W-X31-C-X32-X33-G-X34-K-X35-K-T-T-X36-X37-X38-X39
wherein
X1 is nonexistent, V, I or L,
X2 is K, E, R or Q,
X3 is G, T, Q, P or A,
X4 is V or I,
X5 is A, L, V or I,
X6 is R or L,
X7 is K, R or Q,
X8 is R or K,
X9 is R or L,
X10 is V or G,
X11 is K or N,
X12 is F or L,
X13 is P or S,
X14 is Q or K,
X15 is R, H or Q,
X16 is A, N, S or T,
X17 is A, Q, R, K or T,
X18 is D or E,
X19 is S or A,
X20 is I, E, L, A or V,
X21 is Q, G or E,
X22 is K or R,
X23 is H, Q or E,
X24 is E, Q, N, D, S or H,
X25 is L, V or M,
X26 is E, D, P, L or A,
X27 is E or D,
X28 is V, T, A or I,
X29 is L, N, R, Y, S or Q,
X30 is S, K or T,
X31 is S or M,
X32 is S, A or Y,
X33 is S, T or R,
X34 is N or H,
X35 is V or I,
X36 is R or K,
X37 is nonexistent, V, A, G, M or N,
X38 is nonexistent, T, I, N, F or S, and
X39 is nonexistent, R, H, V, K, I or Q.

26. The AAV vector according to claim 19, wherein the polypeptide further comprises an amino acid sequence represented by General Formula 2 (from N-terminal to C-terminal):
<General Formula 2> X1-X2-H-H-K-A-X3-H
wherein
X1 is A or V,
X2 is N or I, and
X3 is H or Q.

27. The AAV vector according to claim 19, wherein the polypeptide further comprises an amino acid sequence represented by General Formula 3 (from N-terminal to C-terminal):
<General Formula 3> X1-X2-X3-X4-X5-X6-X7-X8-X9-X10
wherein
X1 is A or S,
X2 is L, T or S,
X3 is Q, E or H,
X4 is nonexistent, P, L or H,
X5 is P or R,
X6 is T, S or H,
X7 is A, P, T, S or I,
X8 is T, A, S or I,
X9 is V or A, and
X10 is L or H.

28. The AAV vector according to claim 19, wherein the polypeptide further comprises an amino acid sequence represented by General Formula 4 (from N-terminal to C-terminal):
<General Formula 4> X1-X2
wherein
X1 is H or Y, and
X2 is Q, V or L.

29. The AAV vector according to claim 19, wherein the polypeptide further comprises the following amino acid sequence (from N-terminal to C-terminal):
LHRP (SEQ ID NO 293);
LHQSGFTSGHFPHHRKLGE (SEQ ID NO 294); or
LAPPGTNIQI (SEQ ID NO 295).

30. The AAV vector according to claim 19, wherein the polypeptide further comprises an amino acid sequence represented by General Formula 5 (from N-terminal to C-terminal):
<General Formula 5> X1-X2-X3
wherein
X1 is P or G,
X2 is R or H, and
X3 is T, S or L.

31. The AAV vector according to claim 19, wherein the polypeptide further comprises the following amino acid sequence (from N-terminal to C-terminal):
PYTSL (SEQ ID NO 296); or
QEDKLK (SEQ ID NO 297).

32. The AAV vector according to claim 19, wherein the amino acid sequence with at least 70% sequence identity with the amino acid sequence represented by SEQ ID NO 75 consists of 91 to 119 amino acid sequences.

33. The AAV vector according to claim 19, wherein the polypeptide comprises one or more amino acid sequence selected from a group consisting of amino acid sequences represented by SEQ ID NOS 75 to 129.

34. The AAV vector according to claim 19, wherein the nucleic acid encoding the polypeptide is one or more nucleotide sequence selected from a group consisting of nucleotide sequences represented by SEQ ID NOS 184 to 238.

35. The AAV vector according to claim 19, wherein the polypeptide comprises one or more amino acid sequence selected from a group consisting of amino acid sequences represented by SEQ ID NOS 130 to 183.

36. The AAV vector according to claim 19, wherein the nucleic acid encoding the polypeptide is one or more nucleotide sequence selected from a group consisting of nucleotide sequences represented by SEQ ID NOS 239 to 292.

37. The AAV vector according to claim 19, wherein the AAV vector is for use in gene therapy.

38. The AAV vector according to claim 19, wherein the AAV vector is for use in the expression of a polypeptide comprising an amino acid sequence with at least 70% sequence identity with the amino acid sequence represented by the SEQ ID NO 75.

39. An isolated host cell comprising the AAV vector according to claim 19.

40. An isolated host cell transformed with the AAV vector according to claim 19.

41. A composition comprising the AAV vector according to claim 19 or a host cell comprising the AAV vector or transformed with the AAV vector.

42. The composition according to claim 41, wherein the composition further comprises a pharmaceutically acceptable carrier.

43. The composition according to claim 42, wherein the composition is for use in preventing or treating retinal or macular disease.

44. A kit comprising the AAV vector according to claim 19 or a host cell comprising the AAV vector or transformed with the AAV vector and an instruction manual.

45. A method for producing a recombinant viral particle, comprising a step of transfecting a cell with a construct comprising the AAV vector according to claim 19 and rep and cap genes.

46. The method according to claim 45, wherein the method further comprises a step of isolating the produced recombinant viral particle.

47. A recombinant virus particle produced by the method according to claim 45.

48. A recombinant virus particle comprising (a) capsid protein and (b) the AAV vector according to claim 19.

49. A method for preventing or treating a disease or disorder in a subject in need thereof, comprising administering the AAV vector according to claim 19 or the recombinant viral particle according to claim 48 to the subject.

50. The method according to claim 49, wherein the disease or disorder is a retinal or macular disease.

51. The method according to claim 50, wherein the retinal or macular disease comprises retinopathy, choroidal neovascularization, maculopathy, macular degeneration, retinal degeneration, macular edema, retinal edema, macular swelling, retinal cell degeneration, retinal vascular occlusion, retinal detachment, inherited retinal disease, or a combination thereof.

52. The method according to claim 51, wherein the macular degeneration comprises age-related macular degeneration (AMD).

53. The method according to claim 52, wherein the age-related macular degeneration is wet form or dry form.

54. The method according to claim 51, wherein the retinopathy is diabetic retinopathy.

55. The method according to claim 51, which further comprises administering an additional therapeutic agent to the subject.
